# EUROPEAN PATENT SPECIFICATION

(11) **EP 1 913 028 B1**
(45) Date of publication and mention of the grant of the patent: **04.03.2015**
(21) Application number: 06800398.7
(22) Date of filing: 27.07.2006
(51) Int. Cl.: C07K 16/24, A61K 39/395, A61P 19/08

(54) **USE OF ANTIBODY TO M-CSF**
ANWENDUNG VON ANTIKÖRPERN GEGEN M-CSF
UTILISATION D'ANTICORPS ANTI-M-CSF

(30) Priority: 28.07.2005 US 703191 P
(43) Date of publication of application: 23.04.2008
(60) Divisional application: 10177804.1
(73) Proprietor: Novartis AG, 4056 Basel (CH); XOMA Technology Ltd., Berkeley, CA 94710 (US)
(72) Inventor: KAVANAUGH, William, Michael, Orinda, CA 94563 (US)
(74) Representative: Marshall, Cameron John
(86) International application number: PCT/US2006/029186
(87) International publication number: WO 2007/016240

(56) References cited:
- WO-A2-2004/045532
- WO-A2-2005/068503
- GB-A- 2 405 873
- US-A- 5 766 886
- US-A1- 2002 141 994
- STUDNICKA G M ET AL: "Human-engineered monoclonal antibodies retaine full specific binding activity by preserving non-CDR complementarity-modulating residues" PROTEIN ENGINEERING, OXFORD UNIVERSITY PRESS, SURREY, GB, vol. 7, no. 6, 1 June 1994 (1994-06-01), pages 805-814, XP000447301 ISSN: 0269-2139 cited in the application
- ANONYMOUS: "Mouse anti-Human M-CSF Monoclonal Antibody [116] Datasheet, Catalogue No. MO-C40048A, Clone No. 116" ANOGEN, [Online] XP002414614 Retrieved from the Internet: URL:http://www.yesbiotech.com/datasheets/M onoclonal%20antibodies%20(MAbs)/Cytokines/ MO-C40048A_M-CSF_ANTIBODY_%5b116%5d_DATASH EET.pdf>
- ANONYMOUS: "Mouse anti-Human M-CSF Monoclonal Antibody [692] Datasheet, Catalogue No. MO-C40048B, Clone No. 692" ANOGEN, [Online] XP002414615 Retrieved from the Internet: URL:http://www.yesbiotech.com/datasheets/M onoclonal%20antibodies%20(MAbs)/Cytokines/ MO-C40048B_M-CSF_ANTIBODY_%5b692%5d_DATASH EET.pdf>
- ANONYMOUS: "Mouse anti-Human M-CSF Monoclonal Antibody [21] Datasheet, Catalogue No. MO-C40048D, Clone No. 21" ANOGEN, [Online] XP002414616 Retrieved from the Internet: URL:http://www.yesbiotech.com/datasheets/M onoclonal%20antibodies%20(MAbs)/Cytokines/ MO-C40048D_M-CSF_ANTIBODY_%5b21%5d_DATASHE ET.pdf>
- ANONYMOUS: "Monoclonal Anti-human M-CSF Antibody, Catalogue No. MAB216, Clone No. 26730" R&D SYSTEMS, [Online] XP002414617 Retrieved from the Internet: URL:http://www.rndsystems.com/pdf/mab216.p df>

### SEQUENCE LISTING

<110> Novartis, et al.
<120> USES OF ANTIBODY TO M-CSF
<130> 28094.0003
<140>
   <141>
<150>
   <151>
<150>
   <151>
<160> 137
<170> PatentIn version 3.3
<210> 1
   <211> 1401
   <212> DNA
   <213> Mus musculus
<400> 1
   atgggttggt cctgtatcat cctattcctg gtggccactg ccacaggtgt gcactccgac     60
   gtgcagcttc aggagtcagg acctggcctc gtgaaacctt ctcagagtct gtccctcacc    120
   tgtactgtca ctgactactc catcaccagt gattacgcct ggaactggat acggcaattc    180
   ccagggaata aacttgagtg gatggggtac ataagctaca gtggtagcac ttcctacaat    240
   ccatctctca aaagtcggat ctccatcact cgagacacat ccaagaacca gttcttcctg    300
   cagctgaact ctgtgactac tgaggacaca gccacatatt actgtgcatc cttcgactat    360
   gcccacgcca tggattactg gggccaaggg acttcggtca ctgtctcttc cgccaaaaca    420
   acagccccat cggtctatcc actggcccct gtgtgtggag atacaactgg ctcctcggtg    480
   actctaggat gcctggtcaa gggttatttc cctgagccag tgaccttgac ctggaactct    540
   ggatccctgt ccagtggtgt gcacaccttc ccagctgtcc tgcagtctga cctctacacc    600
   ctcagcagct cagtgactgt aacctcgagc acctggccca gccagtccat cacctgcaat    660
   gtggcccacc cggcaagcag caccaaggtg gacaagaaaa ttgagcccag agggcccaca    720
   atcaagccct gtcctccatg caaatgccca gcacctaacc tcttgggtgg accatccgtc    780
   ttcatcttcc ctccaaagat caaggatgta ctcatgatct ccctgagccc catagtcaca    840
   tgtgtggtgg tggatgtgag cgaggatgac ccagatgtcc agatcagctg gtttgtgaac    900
   aacgtggaag tacacacagc tcagacacaa acccatagag aggattacaa cagtactctc    960
   cgggtggtca gtgccctccc catccagcac caggactgga tgagtggcaa ggagttcaaa   1020
   tgcaaggtca acaacaaaga cctcccagcg cccatcgaga gaaccatctc aaaacccaaa   1080
   gggtcagtaa gagctccaca ggtatatgtc ttgcctccac cagaagaaga gatgactaag   1140
   aaacaggtca ctctgacctg catggtcaca gacttcatgc ctgaagacat ttacgtggag   1200
   tggaccaaca acgggaaaac agagctaaac tacaagaaca ctgaaccagt cctggactct   1260
   gatggttctt acttcatgta cagcaagctg agagtggaaa agaagaactg ggtggaaaga   1320
   aatagctact cctgttcagt ggtccacgag ggtctgcaca atcaccacac gactaagagc   1380
   ttctcccgga ctccgggtaa a                                             1401
<210> 2
   <211> 447
   <212> PRT
   <213> Mus musculus
<400> 2
   Asp Val Gln Leu Gln Glu Ser Gly Pro Gly Leu Val Lys Pro Ser Gln
   1               5                   10                  15
   Ser Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Ser Ile Thr Arg Asp Thr Ser Lys Asn Gln Phe Phe
   65                  70                  75                  80
   Leu Gln Leu Asn Ser Val Thr Thr Glu Asp Thr Ala Thr Tyr Tyr Cys
                   85                  90                  95
   Ala Ser Phe Asp Tyr Ala His Ala Met Asp Tyr Trp Gly Gln Gly Thr
               100                 105                 110
   Ser Val Thr Val Ser Ser Ala Lys Thr Thr Ala Pro Ser Val Tyr Pro
           115                 120                 125
   Leu Ala Pro Val Cys Gly Asp Thr Thr Gly Ser Ser Val Thr Leu Gly
       130                 135                 140
   Cys Leu Val Lys Gly Tyr Phe Pro Glu Pro Val Thr Leu Thr Trp Asn
   145                 150                 155                 160
   Ser Gly Ser Leu Ser Ser Gly Val His Thr Phe Pro Ala Val Leu Gln
                   165                 170                 175
   Ser Asp Leu Tyr Thr Leu Ser Ser Ser Val Thr Val Thr Ser Ser Thr
               180                 185                 190
   Trp Pro Ser Gln Ser Ile Thr Cys Asn Val Ala His Pro Ala Ser Ser
           195                 200                 205
   Thr Lys Val Asp Lys Lys Ile Glu Pro Arg Gly Pro Thr Ile Lys Pro
       210                 215                 220
   Cys Pro Pro Cys Lys Cys Pro Ala Pro Asn Leu Leu Gly Gly Pro Ser
   225                 230                 235                 240
   Val Phe Ile Phe Pro Pro Lys Ile Lys Asp Val Leu Met Ile Ser Leu
                   245                 250                 255
   Ser Pro Ile Val Thr Cys Val Val Val Asp Val Ser Glu Asp Asp Pro
               260                 265                 270
   Asp Val Gln Ile Ser Trp Phe Val Asn Asn Val Glu Val His Thr Ala
           275                 280                 285
   Gln Thr Gln Thr His Arg Glu Asp Tyr Asn Ser Thr Leu Arg Val Val
       290                 295                 300
   Ser Ala Leu Pro Ile Gln His Gln Asp Trp Met Ser Gly Lys Glu Phe
   305                 310                 315                 320
   Lys Cys Lys Val Asn Asn Lys Asp Leu Pro Ala Pro Ile Glu Arg Thr
                   325                 330                 335
   Ile Ser Lys Pro Lys Gly Ser Val Arg Ala Pro Gln Val Tyr Val Leu
               340                 345                 350
   Pro Pro Pro Glu Glu Glu Met Thr Lys Lys Gln Val Thr Leu Thr Cys
           355                 360                 365
   Met Val Thr Asp Phe Met Pro Glu Asp Ile Tyr Val Glu Trp Thr Asn
       370                 375                 380
   Asn Gly Lys Thr Glu Leu Asn Tyr Lys Asn Thr Glu Pro Val Leu Asp
   385                 390                 395                 400
   Ser Asp Gly Ser Tyr Phe Met Tyr Ser Lys Leu Arg Val Glu Lys Lys
                   405                 410                 415
   Asn Trp Val Glu Arg Asn Ser Tyr Ser Cys Ser Val Val His Glu Gly
               420                 425                 430
   Leu His Asn His His Thr Thr Lys Ser Phe Ser Arg Thr Pro Gly
           435                 440                 445
<210> 3
   <211> 702
   <212> DNA
   <213> Mus musculus
<400> 3
   atggtatcca cacctcagtt ccttgtattt ttgcttttct ggattccagc ctccagaggt      60
   gacatcttgc tgactcagtc tccagccatc ctgtctgtga gtccaggaga aagagtcagt     120
   ttctcctgca gggccagtca gagcattggc acaagcatac actggtatca gcaaagaaca     180
   aatggttctc caaggcttct cataaagtat gcttctgagt ctatctctgg gatcccttcc     240
   aggtttagtg gcagtggatc agggacagat tttactctta gcatcaacag tgtggagtct     300
   gaagatattg cagattatta ctgtcaacaa attaatagct ggccaaccac gttcggcggg     360
   gggacaaagt tggaaataaa acgggctgat gctgcaccaa ctgtatccat cttcccacca     420
   tccagtgagc agttaacatc tggaggtgcc tcagtcgtgt gcttcttgaa caacttctac     480
   cccaaagaca tcaatgtcaa gtggaagatt gatggcagtg aacgacaaaa tggcgtcctg     540
   aacagttgga ctgatcagga cagcaaagac agcacctaca gcatgagcag caccctcacg     600
   ttgaccaagg acgagtatga acgacataac agctatacct gtgaggccac tcacaagaca     660
   tcaacttcac ccattgtcaa gagcttcaac aggaatgagt gt                        702
<210> 4
   <211> 214
   <212> PRT
   <213> Mus musculus
<400> 4
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Ser Ile Gly Thr Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Ile Asn Ser Trp Pro Thr
                   85                  90                  95
   Thr Phe Gly Gly Gly Thr Lys Leu Glu Ile Lys Arg Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 5
   <211> 109
   <212> PRT
   <213> Mus musculus
<400> 5
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Ser Ile Gly Thr Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Ile Asn Ser Trp Pro Thr
                       85                  90                  95
   Thr Phe Gly Gly Gly Thr Lys Leu Glu Ile Lys Arg Ala
               100                 105
<210> 6
   <211> 118
   <212> PRT
   <213> Mus musculus
<400> 6
   Asp Val Gln Leu Gln Glu Ser Gly Pro Gly Leu Val Lys Pro Ser Gln
   1               5                   10                  15
   Ser Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Ser Ile Thr Arg Asp Thr Ser Lys Asn Gln Phe Phe
   65                  70                  75                  80
   Leu Gln Leu Asn Ser Val Thr Thr Glu Asp Thr Ala Thr Tyr Tyr Cys
                   85                  90                  95
   Ala Ser Phe Asp Tyr Ala His Ala Met Asp Tyr Trp Gly Gln Gly Thr
               100                 105                 110
   Ser Val Thr Val Ser Ser
           115
<210> 7
   <211> 256
   <212> PRT
   <213> Mus musculus
<400> 7
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Gly His Glu Arg Gln Ser Glu Gly Ser Ser Ser
               180                 185                 190
   Pro Gln Leu Gln Glu Ser Val Phe His Leu Leu Val Pro Ser Val Ile
           195                 200                 205
   Leu Val Leu Leu Ala Val Gly Gly Leu Leu Phe Tyr Arg Trp Arg Arg
       210                 215                 220
   Arg Ser His Gln Glu Pro Gln Arg Ala Asp Ser Pro Leu Glu Gln Pro
   225                 230                 235                 240
   Glu Gly Ser Pro Leu Thr Gln Asp Asp Arg Gln Val Glu Leu Pro Val
                   245                 250                 255
<210> 8
   <211> 554
   <212> PRT
   <213> Mus musculus
<400> 8
   Met Thr Ala Pro Gly Ala Ala Gly Arg Cys Pro Pro Thr Thr Trp Leu
   1               5                   10                  15
   Gly Ser Leu Leu Leu Leu Val Cys Leu Leu Ala Ser Arg Ser Ile Thr
               20                  25                  30
   Glu Glu Val Ser Glu Tyr Cys Ser His Met Ile Gly Ser Gly His Leu
           35                  40                  45
   Gln Ser Leu Gln Arg Leu Ile Asp Ser Gln Met Glu Thr Ser Cys Gln
       50                  55                  60
   Ile Thr Phe Glu Phe Val Asp Gln Glu Gln Leu Lys Asp Pro Val Cys
   65                  70                  75                  80
   Tyr Leu Lys Lys Ala Phe Leu Leu Val Gln Asp Ile Met Glu Asp Thr
                   85                  90                  95
   Met Arg Phe Arg Asp Asn Thr Pro Asn Ala Ile Ala Ile Val Gln Leu
               100                 105                 110
   Gln Glu Leu Ser Leu Arg Leu Lys Ser Cys Phe Thr Lys Asp Tyr Glu
           115                 120                 125
   Glu His Asp Lys Ala Cys Val Arg Thr Phe Tyr Glu Thr Pro Leu Gln
       130                 135                 140
   Leu Leu Glu Lys Val Lys Asn Val Phe Asn Glu Thr Lys Asn Leu Leu
   145                 150                 155                 160
   Asp Lys Asp Trp Asn Ile Phe Ser Lys Asn Cys Asn Asn Ser Phe Ala
                   165                 170                 175
   Glu Cys Ser Ser Gln Asp Val Val Thr Lys Pro Asp Cys Asn Cys Leu
               180                 185                 190
   Tyr Pro Lys Ala Ile Pro Ser Ser Asp Pro Ala Ser Val Ser Pro His
           195                 200                 205
   Gln Pro Leu Ala Pro Ser Met Ala Pro Val Ala Gly Leu Thr Trp Glu
       210                 215                 220
   Asp Ser Glu Gly Thr Glu Gly Ser Ser Leu Leu Pro Gly Glu Gln Pro
   225                 230                 235                 240
   Leu His Thr Val Asp Pro Gly Ser Ala Lys Gln Arg Pro Pro Arg Ser
                   245                 250                 255
   Thr Cys Gln Ser Phe Glu Pro Pro Glu Thr Pro Val Val Lys Asp Ser
               260                 265                 270
   Thr Ile Gly Gly Ser Pro Gln Pro Arg Pro Ser Val Gly Ala Phe Asn
           275                 280                 285
   Pro Gly Met Glu Asp Ile Leu Asp Ser Ala Met Gly Thr Asn Trp Val
       290                 295                 300
   Pro Glu Glu Ala Ser Gly Glu Ala Ser Glu Ile Pro Val Pro Gln Gly
   305                 310                 315                 320
   Thr Glu Leu Ser Pro Ser Arg Pro Gly Gly Gly Ser Met Gln Thr Glu
                   325                 330                 335
   Pro Ala Arg Pro Ser Asn Phe Leu Ser Ala Ser Ser Pro Leu Pro Ala
               340                 345                 350
   Ser Ala Lys Gly Gln Gln Pro Ala Asp Val Thr Gly Thr Ala Leu Pro
           355                 360                 365
   Arg Val Gly Pro Val Arg Pro Thr Gly Gln Asp Trp Asn His Thr Pro
       370                 375                 380
   Gln Lys Thr Asp His Pro Ser Ala Leu Leu Arg Asp Pro Pro Glu Pro
   385                 390                 395                 400
   Gly Ser Pro Arg Ile Ser Ser Leu Arg Pro Gln Gly Leu Ser Asn Pro
                   405                 410                 415
   Ser Thr Leu Ser Ala Gln Pro Gln Leu Ser Arg Ser His Ser Ser Gly
               420                 425                 430
   Ser Val Leu Pro Leu Gly Glu Leu Glu Gly Arg Arg Ser Thr Arg Asp
           435                 440                 445
   Arg Arg Ser Pro Ala Glu Pro Glu Gly Gly Pro Ala Ser Glu Gly Ala
       450                 455                 460
   Ala Arg Pro Leu Pro Arg Phe Asn Ser Val Pro Leu Thr Asp Thr Gly
   465                 470                 475                 480
   His Glu Arg Gln Ser Glu Gly Ser Ser Ser Pro Gln Leu Gln Glu Ser
                   485                 490                 495
   Val Phe His Leu Leu Val Pro Ser Val Ile Leu Val Leu Leu Ala Val
               500                 505                 510
   Gly Gly Leu Leu Phe Tyr Arg Trp Arg Arg Arg Ser His Gln Glu Pro
           515                 520                 525
   Gln Arg Ala Asp Ser Pro Leu Glu Gln Pro Glu Gly Ser Pro Leu Thr
   530 535 540
   Gln Asp Asp Arg Gln Val Glu Leu Pro Val
   545 550
<210> 9
   <211> 438
   <212> PRT
   <213> Mus musculus
<400> 9
   Met Thr Ala Pro Gly Ala Ala Gly Arg Cys Pro Pro Thr Thr Trp Leu
   1 5 10 15
   Gly Ser Leu Leu Leu Leu Val Cys Leu Leu Ala Ser Arg Ser Ile Thr
   20 25 30
   Glu Glu Val Ser Glu Tyr Cys Ser His Met Ile Gly Ser Gly His Leu
   35 40 45
   Gln Ser Leu Gln Arg Leu Ile Asp Ser Gln Met Glu Thr Ser Cys Gln
   50 55 60
   Ile Thr Phe Glu Phe Val Asp Gln Glu Gln Leu Lys Asp Pro Val Cys
   65 70 75 80
   Tyr Leu Lys Lys Ala Phe Leu Leu Val Gln Asp Ile Met Glu Asp Thr
   85 90 95
   Met Arg Phe Arg Asp Asn Thr Pro Asn Ala Ile Ala Ile Val Gln Leu
   100 105 110
   Gln Glu Leu Ser Leu Arg Leu Lys Ser Cys Phe Thr Lys Asp Tyr Glu
   115 120 125
   Glu His Asp Lys Ala Cys Val Arg Thr Phe Tyr Glu Thr Pro Leu Gln
   130 135 140
   Leu Leu Glu Lys Val Lys Asn Val Phe Asn Glu Thr Lys Asn Leu Leu
   145 150 155 160
   Asp Lys Asp Trp Asn Ile Phe Ser Lys Asn Cys Asn Asn Ser Phe Ala
   165 170 175
   Glu Cys Ser Ser Gln Asp Val Val Thr Lys Pro Asp Cys Asn Cys Leu
   180 185 190
   Tyr Pro Lys Ala Ile Pro Ser Ser Asp Pro Ala Ser Val Ser Pro His
   195 200 205
   Gln Pro Leu Ala Pro Ser Met Ala Pro Val Ala Gly Leu Thr Trp Glu
   210 215 220
   Asp Ser Glu Gly Thr Glu Gly Ser Ser Leu Leu Pro Gly Glu Gln Pro
   225 230 235 240
   Leu His Thr Val Asp Pro Gly Ser Ala Lys Gln Arg Pro Pro Arg Ser
   245 250 255
   Thr Cys Gln Ser Phe Glu Pro Pro Glu Thr Pro Val Val Lys Asp Ser
   260 265 270
   Thr Ile Gly Gly Ser Pro Gln Pro Arg Pro Ser Val Gly Ala Phe Asn
   275 280 285
   Pro Gly Met Glu Asp Ile Leu Asp Ser Ala Met Gly Thr Asn Trp Val
   290 295 300
   Pro Glu Glu Ala Ser Gly Glu Ala Ser Glu Ile Pro Val Pro Gln Gly
   305 310 315 320
   Thr Glu Leu Ser Pro Ser Arg Pro Gly Gly Gly Ser Met Gln Thr Glu
   325 330 335
   Pro Ala Arg Pro Ser Asn Phe Leu Ser Ala Ser Ser Pro Leu Pro Ala
   340 345 350
   Ser Ala Lys Gly Gln Gln Pro Ala Asp Val Thr Gly His Glu Arg Gln
   355 360 365
   Ser Glu Gly Ser Ser Ser Pro Gln Leu Gln Glu Ser Val Phe His Leu
   370 375 380
   Leu Val Pro Ser Val Ile Leu Val Leu Leu Ala Val Gly Gly Leu Leu
   385 390 395 400
   Phe Tyr Arg Trp Arg Arg Arg Ser His Gln Glu Pro Gln Arg Ala Asp
   405 410 415
   Ser Pro Leu Glu Gln Pro Glu Gly Ser Pro Leu Thr Gln Asp Asp Arg
   420 425 430
   Gln Val Glu Leu Pro Val
   435
<210> 10
   <211> 441
   <212> PRT
   <213> Mus musculus
<400> 10
   Glu Ile Gln Leu Gln Gln Ser Gly Pro Glu Leu Val Lys Thr Gly Thr
   1               5                   10                  15
   Ser Val Lys Ile Ser Cys Lys Ala Ser Gly Tyr Ser Phe Thr Gly Tyr
               20                  25                  30
   Phe Met His Trp Val Lys Gln Ser His Gly Lys Ser Leu Glu Trp Ile
           35                  40                  45
   Gly Tyr Ile Ser Cys Tyr Asn Gly Asp Thr Asn Tyr Asn Gln Asn Phe
       50                  55                  60
   Lys Gly Lys Ala Thr Phe Thr Val Asp Thr Ser Ser Ser Thr Ala Tyr
   65                  70                  75                  80
   Met Gln Phe Asn Ser Leu Thr Ser Glu Asp Ser Ala Val Tyr Tyr Cys
                   85                  90                  95
   Ala Arg Glu Gly Gly Asn Tyr Pro Ala Tyr Trp Gly Gln Gly Thr Leu
               100                 105                 110
   Val Thr Val Ser Ala Ala Lys Thr Thr Pro Pro Ser Val Tyr Pro Leu
           115                 120                 125
   Ala Pro Gly Ser Ala Ala Gln Thr Asn Ser Met Val Thr Leu Gly Cys
       130                 135                 140
   Leu Val Lys Gly Tyr Phe Pro Glu Pro Val Thr Val Thr Trp Asn Ser
   145                 150                 155                 160
   Gly Ser Leu Ser Ser Gly Val His Thr Phe Pro Ala Val Leu Gln Ser
                   165                 170                 175
   Asp Leu Tyr Thr Leu Ser Ser Ser Val Thr Val Pro Ser Ser Thr Trp
               180                 185                 190
   Pro Ser Glu Thr Val Thr Cys Asn Val Ala His Pro Ala Ser Ser Thr
           195                 200                 205
   Lys Val Asp Lys Lys Ile Val Pro Arg Asp Cys Gly Cys Lys Pro Cys
       210                 215                 220
   Ile Cys Thr Val Pro Glu Val Ser Ser Val Phe Ile Phe Pro Pro Lys
   225 230 235 240
   Pro Lys Asp Val Leu Thr Ile Thr Leu Thr Pro Lys Val Thr Cys Val
   245 250 255
   Val Val Asp Ile Ser Lys Asp Asp Pro Glu Val Gln Phe Ser Trp Phe
   260 265 270
   Val Asp Asp Val Glu Val His Thr Ala Gln Thr Gln Pro Arg Glu Glu
   275 280 285
   Gln Phe Asn Ser Thr Phe Arg Ser Val Ser Glu Leu Pro Ile Met His
   290 295 300
   Gln Asp Trp Leu Asn Gly Lys Glu Phe Lys Cys Arg Val Asn Ser Ala
   305 310 315 320
   Ala Phe Pro Ala Pro Ile Glu Lys Thr Ile Ser Lys Thr Lys Gly Arg
   325 330 335
   Pro Lys Ala Pro Gln Val Tyr Thr Ile Pro Pro Pro Lys Glu Gln Met
   340 345 350
   Ala Lys Asp Lys Val Ser Leu Thr Cys Met Ile Thr Asp Phe Phe Pro
   355 360 365
   Glu Asp Ile Thr Val Glu Trp Gln Trp Asn Gly Gln Pro Ala Glu Asn
   370 375 380
   Tyr Lys Asn Thr Gln Pro Ile Met Asp Thr Asp Gly Ser Tyr Phe Val
   385 390 395 400
   Tyr Ser Lys Leu Asn Val Gln Lys Ser Asn Trp Glu Ala Gly Asn Thr
   405 410 415
   Phe Thr Cys Ser Val Leu His Glu Gly Leu His Asn His His Thr Glu
   420 425 430
   Lys Ser Leu Ser His Ser Pro Gly Lys
   435 440
<210> 11
   <211> 214
   <212> PRT
   <213> Mus musculus
<400> 11
   Asp Ile Val Met Thr Gln Ser His Lys Phe Met Ser Thr Ser Val Gly
   1 5 10 15
   Asp Arg Val Thr Ile Thr Cys Lys Ala Ser Gln Asn Val Gly Thr Ala
   20 25 30
   Val Thr Trp Tyr Gln Gln Lys Pro Gly Gln Ser Pro Lys Leu Leu Ile
   35 40 45
   Tyr Trp Thr Ser Thr Arg His Ala Gly Val Pro Asp Arg Phe Thr Gly
   50 55 60
   Ser Gly Ser Gly Thr Asp Phe Thr Leu Thr Ile Ser Asp Val Gln Ser
   65 70 75 80
   Glu Asp Leu Ala Asp Tyr Phe Cys Gln Gln Tyr Ser Ser Tyr Pro Leu
   85 90 95
   Thr Phe Gly Ala Gly Thr Lys Leu Glu Leu Lys Arg Ala Asp Ala Ala
   100 105 110
   Pro Thr Val Ser Ile Phe Pro Pro Ser Ser Glu Gln Leu Thr Ser Gly
   115 120 125
   Gly Ala Ser Val Val Cys Phe Leu Asn Asn Phe Tyr Pro Lys Asp Ile
   130 135 140
   Asn Val Lys Trp Lys Ile Asp Gly Ser Glu Arg Gln Asn Gly Val Leu
   145 150 155 160
   Asn Ser Trp Thr Asp Gln Asp Ser Lys Asp Ser Thr Tyr Ser Met Ser
   165 170 175
   Ser Thr Leu Thr Leu Thr Lys Asp Glu Tyr Glu Arg His Asn Ser Tyr
   180 185 190
   Thr Cys Glu Ala Thr His Lys Thr Ser Thr Ser Pro Ile Val Lys Ser
   195 200 205
   Phe Asn Arg Asn Glu Cys
   210
<210> 12
   <211> 449
   <212> PRT
   <213> Mus musculus
<400> 12
   Glu Val Lys Leu Val Glu Ser Gly Gly Gly Leu Val Gln Pro Gly Gly
   1 5 10 15
   Ser Leu Lys Leu Ser Cys Ala Thr Ser Gly Phe Thr Phe Ser Asp Tyr
   20 25 30
   Tyr Met Tyr Trp Val Arg Gln Thr Pro Glu Lys Arg Leu Glu Trp Val
   35 40 45
   Ala Tyr Ile Ser Asn Gly Gly Gly Ser Thr Tyr Tyr Pro Asp Thr Val
   50 55 60
   Lys Gly Arg Phe Thr Ile Ser Arg Asp Asn Ala Lys Asn Thr Leu Tyr
   65 70 75 80
   Leu Gln Met Ser Arg Leu Lys Ser Glu Asp Thr Ala Met Tyr Tyr Cys
   85 90 95
   Ala Arg Gln Gly Ser Tyr Gly Tyr Pro Phe Ala Tyr Trp Gly Gln Gly
   100 105 110
   Thr Leu Val Thr Val Ser Ala Ala Lys Thr Thr Ala Pro Ser Val Tyr
   115 120 125
   Pro Leu Ala Pro Val Cys Gly Asp Thr Thr Gly Ser Ser Val Thr Leu
   130 135 140
   Gly Cys Leu Val Lys Gly Tyr Phe Pro Glu Pro Val Thr Leu Thr Trp
   145 150 155 160
   Asn Ser Gly Ser Leu Ser Ser Gly Val His Thr Phe Pro Ala Val Leu
   165 170 175
   Gln Ser Asp Leu Tyr Thr Leu Ser Ser Ser Val Thr Val Thr Ser Ser
   180 185 190
   Thr Trp Pro Ser Gln Ser Ile Thr Cys Asn Val Ala His Pro Ala Ser
   195 200 205
   Ser Thr Lys Val Asp Lys Lys Ile Glu Pro Arg Gly Pro Thr Ile Lys
   210 215 220
   Pro Cys Pro Pro Cys Lys Cys Pro Ala Pro Asn Leu Leu Gly Gly Pro
   225 230 235 240
   Ser Val Phe Ile Phe Pro Pro Lys Ile Lys Asp Val Leu Met Ile Ser
   245 250 255
   Leu Ser Pro Ile Val Thr Cys Val Val Val Asp Val Ser Glu Asp Asp
               260                 265                 270
   Pro Asp Val Gln Ile Ser Trp Phe Val Asn Asn Val Glu Val His Thr
           275                 280                 285
   Ala Gln Thr Gln Thr His Arg Glu Asp Tyr Asn Ser Thr Leu Arg Val
       290                 295                 300
   Val Ser Ala Leu Pro Ile Gln His Gln Asp Trp Met Ser Gly Lys Glu
   305                 310                 315                 320
   Phe Lys Cys Lys Val Asn Asn Lys Asp Leu Pro Ala Pro Ile Glu Arg
                   325                 330                 335
   Thr Ile Ser Lys Pro Lys Gly Ser Val Arg Ala Pro Gln Val Tyr Val
               340                 345                 350
   Leu Pro Pro Pro Glu Glu Glu Met Thr Lys Lys Gln Val Thr Leu Thr
           355                 360                 365
   Cys Met Val Thr Asp Phe Met Pro Glu Asp Ile Tyr Val Glu Trp Thr
       370                 375                 380
   Asn Asn Gly Lys Thr Glu Leu Asn Tyr Lys Asn Thr Glu Pro Val Leu
   385                 390                 395                 400
   Asp Ser Asp Gly Ser Tyr Phe Met Tyr Ser Lys Leu Arg Val Glu Lys
                   405                 410                 415
   Lys Asn Trp Val Glu Arg Asn Ser Tyr Ser Cys Ser Val Val His Glu
               420                 425                 430
   Gly Leu His Asn His His Thr Thr Lys Ser Phe Ser Arg Thr Pro Gly
           435                 440                 445
   Lys
<210> 13
   <211> 214
   <212> PRT
   <213> Mus musculus
<400> 13
   Ala Ile Gln Met Thr Gln Thr Thr Ser Ser Leu Ser Ala Ser Leu Gly
   1               5                   10                  15
   Asp Arg Val Thr Ile Ser Cys Ser Ala Ser Gln Gly Ile Ser Asn Tyr
   20 25 30
   Leu Asn Trp Tyr Gln Gln Lys Pro Asp Gly Thr Val Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Trp
   85 90 95
   Thr Phe Gly Gly Gly Thr Lys Leu Glu Ile Lys Arg Ala Asp Ala Ala
   100 105 110
   Pro Thr Val Ser Ile Phe Pro Pro Ser Ser Glu Gln Leu Thr Ser Gly
   115 120 125
   Gly Ala Ser Val Val Cys Phe Leu Asn Asn Phe Tyr Pro Lys Asp Ile
   130 135 140
   Asn Val Lys Trp Lys Ile Asp Gly Ser Glu Arg Gln Asn Gly Val Leu
   145 150 155 160
   Asn Ser Trp Thr Asp Gln Asp Ser Lys Asp Ser Thr Tyr Ser Met Ser
   165 170 175
   Ser Thr Leu Thr Leu Thr Lys Asp Glu Tyr Glu Arg His Asn Ser Tyr
   180 185 190
   Thr Cys Glu Ala Thr His Lys Thr Ser Thr Ser Pro Ile Val Lys Ser
   195 200 205
   Phe Asn Arg Asn Glu Cys
   210
<210> 14
   <211> 522
   <212> PRT
   <213> Mus musculus
<400> 14
   Asp Val Gln Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Asp
   20 25 30
   Tyr Ala Trp Asn Trp Ile Arg Gln Phe Pro Gly Asn Lys Leu Glu Trp
   35 40 45
   Met Gly Tyr Ile Ser Tyr Ser Gly Ser Thr Ser Tyr Asn Pro Ser Leu
   50 55 60
   Lys Ser Arg Ile Ser Ile Thr Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Arg Leu Glu Thr Trp Leu Phe Asp Tyr Trp Gly Gln Gly Thr Thr
   100 105 110
   Leu Thr Val Ser Ser Ala Lys Thr Thr Pro Pro Ser Val Tyr Pro Leu
   115 120 125
   Ala Pro Gly Cys Gly Asp Thr Thr Gly Ser Ser Val Thr Leu Gly Cys
   130 135 140
   Leu Val Lys Gly Tyr Phe Pro Glu Ser Val Thr Val Thr Trp Asn Ser
   145 150 155 160
   Gly Ser Leu Ser Ser Ser Val His Thr Phe Pro Ala Leu Leu Gln Ser
   165 170 175
   Gly Leu Tyr Thr Met Ser Ser Ser Val Thr Val Pro Ser Ser Thr Trp
   180 185 190
   Pro Ser Gln Thr Val Thr Cys Ser Val Ala His Pro Ala Ser Ser Thr
   195 200 205
   Thr Val Asp Lys Lys Leu Glu Pro Ser Gly Pro Ile Ser Thr Ile Asn
   210 215 220
   Pro Cys Pro Pro Cys Lys Glu Cys His Lys Cys Pro Ala Pro Asn Leu
   225 230 235 240
   Glu Gly Gly Pro Ser Val Phe Ile Phe Pro Pro Asn Ile Lys Asp Val
   245 250 255
   Leu Met Ile Ser Leu Thr Pro Lys Val Thr Cys Val Val Val Asp Val
   260 265 270
   Ser Glu Asp Asp Pro Asp Val Gln Ile Ser Trp Phe Val Asn Asn Val
           275                     280                     285
   Glu Val His Thr Ala Gln Thr Gln Thr His Arg Glu Asp Tyr Asn Ser
       290                     295                     300
   Thr Ile Arg Val Val Ser Thr Leu Pro Ile Gln His Gln Asp Trp Met
   305                 310                     315                 320
   Ser Gly Lys Glu Phe Lys Cys Lys Val Asn Asn Lys Asp Leu Pro Ser
                   325                     330                     335
   Pro Ile Glu Arg Thr Ile Ser Lys Ile Lys Gly Leu Val Arg Ala Pro
               340                     345                     350
   Gln Val Tyr Ile Leu Pro Pro Pro Ala Glu Gln Leu Ser Arg Lys Asp
           355                     360                     365
   Val Ser Leu Thr Cys Leu Val Val Gly Phe Asn Pro Gly Asp Ile Ser
       370                     375                     380
   Val Glu Trp Thr Ser Asn Gly His Thr Glu Glu Asn Tyr Lys Asp Thr
   385                 390                     395                 400
   Ala Pro Val Leu Asp Ser Asp Gly Ser Tyr Phe Ile Tyr Ser Lys Leu
                   405                     410                     415
   Asn Met Lys Thr Ser Lys Trp Glu Lys Thr Asp Ser Phe Ser Cys Asn
               420                     425                     430
   Val Arg His Glu Gly Leu Lys Asn Tyr Tyr Leu Lys Lys Thr Ile Ser
           435                     440                     445
   Arg Ser Pro Gly Leu Asp Leu Asp Asp Ile Cys Ala Glu Ala Lys Asp
       450                     455                     460
   Gly Glu Leu Asp Gly Leu Trp Thr Thr Ile Thr Ile Phe Ile Ser Leu
   465                 470                     475                 480
   Phe Leu Leu Ser Val Cys Tyr Ser Ala Ser Val Thr Leu Phe Lys Val
                   485                     490                     495
   Lys Trp Ile Phe Ser Ser Val Val Glu Leu Lys Gln Lys Ile Ser Pro
               500                     505                     510
   Asp Tyr Arg Asn Met Ile Gly Gln Gly Ala
           515                     520
<210> 15
   <211> 214
   <212> PRT
   <213> Mus musculus
<400> 15
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Ser Ile Gly Thr Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Ser Asn Ser Trp Pro Thr
                   85                  90                  95
   Thr Phe Gly Gly Gly Thr Lys Leu Glu Ile Lys Trp Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 16
   <211> 5
   <212> PRT
   <213> Homo sapiens
<400> 16
   Gly Tyr Phe Met His
   1               5
<210> 17
   <211> 5
   <212> PRT
   <213> Homo sapiens
<400> 17
   Asp Tyr Tyr Met Tyr
   1               5
<210> 18
   <211> 6
   <212> PRT
   <213> Homo sapiens
<400> 18
   Ser Asp Tyr Ala Trp Asn
   1               5
<210> 19
   <211> 17
   <212> PRT
   <213> Homo sapiens
<400> 19
   Tyr Ile Ser Cys Tyr Asn Gly Asp Thr Asn Tyr Asn Gln Asn Phe Lys
   1               5                   10                  15
   Gly
<210> 20
   <211> 17
   <212> PRT
   <213> Homo sapiens
<400> 20
   Tyr Ile Ser Asn Gly Gly Gly Ser Thr Tyr Tyr Pro Asp Thr Val Lys
   1 5 10 15
   Gly
<210> 21
   <211> 16
   <212> PRT
   <213> Homo sapiens
<400> 21
   Tyr Ile Ser Tyr Ser Gly Ser Thr Ser Tyr Asn Pro Ser Leu Lys Ser
   1 5 10 15
<210> 22
   <211> 8
   <212> PRT
   <213> Homo sapiens
<400> 22
   Glu Gly Gly Asn Tyr Pro Ala Tyr
   1 5
<210> 23
   <211> 10
   <212> PRT
   <213> Homo sapiens
<400> 23
   Gln Gly Ser Tyr Gly Tyr Pro Phe Ala Tyr
   1 5 10
<210> 24
   <211> 9
   <212> PRT
   <213> Homo sapiens
<400> 24
   Phe Asp Tyr Ala His Ala Met Asp Tyr
   1 5
<210> 25
   <211> 8
   <212> PRT
   <213> Homo sapiens
<400> 25
   Leu Glu Thr Trp Leu Phe Asp Tyr
   1 5
<210> 26
   <211> 7
   <212> PRT
   <213> Homo sapiens
<400> 26
   Asp Tyr Gly Trp Phe Asp Tyr
   1 5
<210> 27
   <211> 11
   <212> PRT
   <213> Homo sapiens
<400> 27
   Lys Ala Ser Gln Asn Val Gly Thr Ala Val Thr
   1 5 10
<210> 28
   <211> 11
   <212> PRT
   <213> Homo sapiens
<400> 28
   Ser Ala Ser Gln Gly Ile Ser Asn Tyr Leu Asn
   1 5 10
<210> 29
   <211> 11
   <212> PRT
   <213> Homo sapiens
<400> 29
   Arg Ala Ser Gln Ser Ile Gly Thr Ser Ile His
   1 5 10
<210> 30
   <211> 7
   <212> PRT
   <213> Homo sapiens
<400> 30
   Trp Thr Ser Thr Arg His Ala
   1 5
<210> 31
   <211> 7
   <212> PRT
   <213> Homo sapiens
<400> 31
   Tyr Thr Ser Ser Leu His Ser
   1 5
<210> 32
   <211> 7
   <212> PRT
   <213> Homo sapiens
<400> 32
   Tyr Ala Ser Glu Ser Ile Ser
   1 5
<210> 33
   <211> 7
   <212> PRT
   <213> Homo sapiens
<400> 33
   Tyr Thr Ser Glu Ser Ile Ser
   1 5
<210> 34
   <211> 9
   <212> PRT
   <213> Homo sapiens
<400> 34
   Gln Gln Tyr Ser Ser Tyr Pro Leu Thr
   1 5
<210> 35
   <211> 9
   <212> PRT
   <213> Homo sapiens
<400> 35
   Gln Gln Tyr Ser Lys Leu Pro Trp Thr
   1 5
<210> 36
   <211> 9
   <212> PRT
   <213> Homo sapiens
<400> 36
   Gln Gln Ile Asn Ser Trp Pro Thr Thr
   1 5
<210> 37
   <211> 9
   <212> PRT
   <213> Homo sapiens
<400> 37
   Gln Gln Ser Asn Ser Trp Pro Thr Thr
   1 5
<210> 38
   <211> 9
   <212> PRT
   <213> Homo sapiens
<400> 38
   Gln Gln Tyr Ser Ser Trp Pro Thr Thr
   1 5
<210> 39
   <211> 130
   <212> PRT
   <213> Homo sapiens
<220>
   <221> misc_feature
   <222> (23)..(23)
   <223> Xaa= any amino acid
<220>
   <221> misc_feature
   <222> (27)..(27)
   <223> Xaa= any amino acid
<220>
   <221> misc_feature
   <222> (29).. (29)
   <223> Xaa= any amino acid
<220>
   <221> misc_feature
   <222> (31)..(36)
   <223> Xaa= any amino acid
<220>
   <221> misc_feature
   <222> (51)..(51)
   <223> Xaa= any amino acid
<220>
   <221> misc_feature
   <222> (56)..(57)
   <223> Xaa= any amino acid
<220>
   <221> misc_feature
   <222> (59)..(59)
   <223> Xaa= any amino acid
<220>
   <221> misc_feature
   <222> (61)..(61)
   <223> Xaa= any amino acid
<220>
   <221> misc_feature
   <222> (84)..(84)
   <223> Xaa= any amino acid
<220>
   <221> misc_feature
   <222> (86)..(86)
   <223> Xaa= any amino acid
<220>
   <221> misc_feature
   <222> (101)..(116)
   <223> Xaa= any amino acid
<220>
   <221> misc_feature
   <222> (119)..(119)
   <223> Xaa= any amino acid
<220>
   <221> misc_feature
   <222> (125)..(125)
   <223> Xaa= any amino acid
<400> 39
   Asp Val Gln Leu Gln Glu Ser Gly Pro Gly Leu Val Lys Pro Ser Gln
   1               5                   10                  15
   Thr Leu Ser Leu Thr Cys Xaa Val Ser Gly Xaa Ser Xaa Ser Xaa Xaa
               20                  25                  30
   Xaa Xaa Xaa Xaa Trp Ile Arg Gln Pro Pro Gly Lys Gly Leu Glu Trp
           35                  40                  45
   Ile Gly Xaa Tyr Tyr Arg Ala Xaa Xaa Gly Xaa Thr Xaa Tyr Asn Pro
       50                  55                  60
   Ser Leu Lys Ser Arg Val Thr Ile Ser Val Asp Thr Ser Lys Asn Gln
   65                  70                  75                  80
   Phe Ser Leu Xaa Leu Xaa Ser Val Thr Ala Ala Asp Thr Ala Val Tyr
                   85                  90                  95
   Tyr Cys Ala Arg Xaa Xaa Xaa Xaa Xaa Xaa Xaa Xaa Xaa Xaa Xaa Xaa
               100                 105                 110
   Xaa Xaa Xaa Xaa Phe Asp Xaa Trp Gly Gln Gly Thr Xaa Val Thr Val
           115                 120                 125
   Ser Ser
       130
<210> 40
   <211> 354
   <212> DNA
   <213> Homo sapiens
<400> 40
   gacgtacaac ttcaagaatc tggcccaggt ctcgtcaaac cttctcaaac tctctcactc      60
   acctgcactg ttactgacta ctctattaca tccgactacg cttggaactg gatccgacaa     120
   tttcctggta aaaaactcga atggatgggt tatatttctt actctggctc cacctcctac     180
   aatccttctc tgaaatcacg catcacaatt tcccgcgata cctctaaaaa tcaattttca     240
   ctccaactca attctgttac cgccgccgat actgccacct actactgtgc ctcttttgac     300
   tacgctcacg ccatggatta ttggggacag ggtactaccg ttaccgtaag ctca           354
<210> 41
   <211> 118
   <212> PRT
   <213> Homo sapiens
<400> 41
   Asp Val Gln Leu Gln Glu Ser Gly Pro Gly Leu Val Lys Pro Ser Gln
   1               5                   10                  15
   Thr Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Lys Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Thr Ile Ser Arg Asp Thr Ser Lys Asn Gln Phe Ser
   65                  70                  75                  80
   Leu Gln Leu Asn Ser Val Thr Ala Ala Asp Thr Ala Thr Tyr Tyr Cys
                   85                  90                  95
   Ala Ser Phe Asp Tyr Ala His Ala Met Asp Tyr Trp Gly Gln Gly Thr
               100                 105                 110
   Thr Val Thr Val Ser Ser
           115
<210> 42
   <211> 354
   <212> DNA
   <213> Homo sapiens
<400> 42
   caagttcaac ttcaagaatc aggccccgga ctcgttaaac cctctcaaac tctctctctt    60
   acttgcactg tatccgatta ctctattact tcagactacg cttggaactg gatcagacaa   120
   tttcccggaa aaggactcga atggatggga tatatctctt actctggctc aacctcttac   180
   aacccctctc tcaaatctcg aataacaatc tcacgcgata cttctaaaaa tcaattctca   240
   cttcaactta actccgttac tgccgccgac actgccgttt actactgtgc ttccttcgat   300
   tacgcccacg ctatggatta ttggggacaa ggaactaccg tcactgtcag ctca         354
<210> 43
   <211> 118
   <212> PRT
   <213> Homo sapiens
<400> 43
   Gln Val Gln Leu Gln Glu Ser Gly Pro Gly Leu Val Lys Pro Ser Gln
   1 5 10 15
   Thr Leu Ser Leu Thr Cys Thr Val Ser Asp Tyr Ser Ile Thr Ser Asp
   20 25 30
   Tyr Ala Trp Asn Trp Ile Arg Gln Phe Pro Gly Lys Gly Leu Glu Trp
   35 40 45
   Met Gly Tyr Ile Ser Tyr Ser Gly Ser Thr Ser Tyr Asn Pro Ser Leu
   50 55 60
   Lys Ser Arg Ile Thr Ile Ser Arg Asp Thr Ser Lys Asn Gln Phe Ser
   65 70 75 80
   Leu Gln Leu Asn Ser Val Thr Ala Ala Asp Thr Ala Val Tyr Tyr Cys
   85 90 95
   Ala Ser Phe Asp Tyr Ala His Ala Met Asp Tyr Trp Gly Gln Gly Thr
   100 105 110
   Thr Val Thr Val Ser Ser
   115
<210> 44
   <211> 327
   <212> DNA
   <213> Homo sapiens
<400> 44
   gaaatagttc ttactcaatc ccccggtaca ctctcagttt ccccaggcga acgcgtcact 60
   ttttcttgca gagcatcaca atcaatcggc acttcaattc attggtatca acaaaaaaca 120
   ggacaggccc cacgacttct tattaaatat gcatcagaac gagccacagg catcccagac 180
   agattttcag gttcaggatc aggcaccgat ttcacactta caatatccag agtcgaatca 240
   gaagattttg cagattacta ttgtcaacaa ataaacagct ggcccactac attcggacaa 300
   ggcacaaaac tcgaaattaa acgtacg 327
<210> 45
   <211> 109
   <212> PRT
   <213> Homo sapiens
<400> 45
   Glu Ile Val Leu Thr Gln Ser Pro Gly Thr Leu Ser Val Ser Pro Gly
   1               5                   10                  15
   Glu Arg Val Thr Phe Ser Cys Arg Ala Ser Gln Ser Ile Gly Thr Ser
               20                  25                  30
   Ile His Trp Tyr Gln Gln Lys Thr Gly Gln Ser Pro Arg Leu Leu Ile
           35                  40                  45
   Lys Tyr Ala Ser Glu Arg Ile Ser Gly Ile Pro Asp Arg Phe Ser Gly
       50                  55                  60
   Ser Gly Ser Gly Thr Asp Phe Thr Leu Thr Ile Ser Arg Val Glu Ser
   65                  70                  75                  80
   Glu Asp Phe Ala Asp Tyr Tyr Cys Gln Gln Ile Asn Ser Trp Pro Thr
                   85                  90                  95
   Thr Phe Gly Gln Gly Thr Lys Leu Glu Ile Lys Arg Thr
               100                 105
<210> 46
   <211> 327
   <212> DNA
   <213> Homo sapiens
<400> 46
   gaaatagttc ttactcaatc ccccggtaca ctctcagttt ccccaggcga acgcgtcact      60
   ttttcttgca gagcatcaca atcaatcggc acttcaattc attggtatca acaaaaaaca     120
   ggacaggccc cacgacttct tattaaatat gcatcagaac gagccacagg catcccagac     180
   agattttcag gttcaggatc aggcaccgat ttcacactta caatatccag agtcgaatca     240
   gaagattttg cagattacta ttgtcaacaa ataaacagct ggcccactac attcggacaa     300
   ggcacaaaac tcgaaattaa acgtacg                                         327
<210> 47
   <211> 109
   <212> PRT
   <213> Homo sapiens
<400> 47
   Glu Ile Val Leu Thr Gln Ser Pro Gly Thr Leu Ser Val Ser Pro Gly
   1               5                   10                  15
   Glu Arg Val Thr Phe Ser Cys Arg Ala Ser Gln Ser Ile Gly Thr Ser
   20 25 30
   Ile His Trp Tyr Gln Gln Lys Thr Gly Gln Ala Pro Arg Leu Leu Ile
   35 40 45
   Lys Tyr Ala Ser Glu Arg Ala Thr Gly Ile Pro Asp Arg Phe Ser Gly
   50 55 60
   Ser Gly Ser Gly Thr Asp Phe Thr Leu Thr Ile Ser Arg Val Glu Ser
   65 70 75 80
   Glu Asp Phe Ala Asp Tyr Tyr Cys Gln Gln Ile Asn Ser Trp Pro Thr
   85 90 95
   Thr Phe Gly Gln Gly Thr Lys Leu Glu Ile Lys Arg Thr
   100 105
<210> 48
   <211> 109
   <212> PRT
   <213> Homo sapiens
<400> 48
   Asp Ile Leu Leu Thr Gln Ser Pro Ala Ile Leu Ser Val Ser Pro Gly
   1 5 10 15
   Glu Arg Val Ser Phe Ser Cys Arg Ala Ser Gln Ser Ile Gly Thr Ser
   20 25 30
   Ile His Trp Tyr Gln Gln Arg Thr Asn Gly Ser Pro Arg Leu Leu Ile
   35 40 45
   Lys Tyr Ala Ser Glu Ser Ile Ser Gly Ile Pro Asp Arg Phe Ser Gly
   50 55 60
   Ser Gly Ser Gly Thr Asp Phe Thr Leu Thr Ile Ser Arg Val Glu Ser
   65 70 75 80
   Glu Asp Phe Ala Asp Tyr Tyr Cys Gln Gln Ile Asn Ser Trp Pro Thr
   85 90 95
   Thr Phe Gly Gln Gly Thr Lys Leu Glu Ile Lys Arg Thr
   100 105
<210> 49
   <211> 111
   <212> PRT
   <213> Homo sapiens
<220>
   <221> misc_feature
   <222> (98)..(98)
   <223> Xaa= any amino acid
<400> 49
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Pro Xaa Thr Phe Gly Gln Gly Thr Lys Val Glu Ile Lys Arg Thr
               100                 105                 110
<210> 50
   <211> 108
   <212> PRT
   <213> Homo sapiens
<400> 50
   Asp Val Val Met Thr Gln Ser Pro Ala Phe Leu Ser Val Thr Pro Gly
   1               5                   10                  15
   Glu Lys Val Thr Ile Thr Cys Gln Ala Ser Glu Gly Ile Gly Asn Tyr
               20                  25                  30
   Leu Tyr Trp Tyr Gln Gln Lys Pro Asp Gln Ala Lys Leu Leu Ile Lys
           35                  40                  45
   Tyr Ala Ser Gln Ser Ile Ser Gly Val Pro Ser Arg Phe Ser Gly Ser
       50                  55                  60
   Gly Ser Gly Thr Asp Phe Thr Phe Thr Ile Ser Ser Leu Glu Ala Glu
   65                  70                  75                  80
   Asp Ala Ala Thr Tyr Tyr Cys Gln Gln Gly Asn Lys His Pro Leu Thr
   85 90 95
   Phe Gly Gly Gly Thr Lys Val Glu Ile Lys Arg Thr
   100 105
<210> 51
   <211> 109
   <212> PRT
   <213> Artificial sequence
<220>
   <223> Low Risk Light Chain vs. VK6 Subgroup 2-1-(1) A14:
<400> 51
   Asp Ile Val Leu Thr Gln Ser Pro Ala Phe Leu Ser Val Thr Pro Gly
   1 5 10 15
   Glu Lys Val Thr Phe Thr Cys Gln Ala Ser Gln Ser Ile Gly Thr Ser
   20 25 30
   Ile His Trp Tyr Gln Gln Lys Thr Asp Gln Ser Pro Arg Leu Leu Ile
   35 40 45
   Lys Tyr Ala Ser Glu Ser Ile Ser Gly Ile Pro Ser Arg Phe Ser Gly
   50 55 60
   Ser Gly Ser Gly Thr Asp Phe Thr Leu Thr Ile Ser Ser Val Glu Ala
   65 70 75 80
   Glu Asp Ala Ala Asp Tyr Tyr Cys Gln Gln Ile Asn Ser Trp Pro Thr
   85 90 95
   Thr Phe Gly Gly Gly Thr Lys Leu Glu Ile Lys Arg Thr
   100 105
<210> 52
   <211> 327
   <212> DNA
   <213> Homo sapiens
<400> 52
   gacatagttc tcacacaatc accagcattc ctctcagtta cacccggcga aaaagtaacc 60
   tttacctgtc aggcttctca atctatcggc acttctattc actggtatca acaaaaaacc 120
   gatcaagctc ctaaactcct cataaaatac gcatccgaat ccatctccgg tatcccctcc 180
   agattttcag gctccggctc cggcacagat ttcaccctta ccattagctc agttgaagcc 240
   gaagacgcag ctgattacta ctgtcaacaa ataaactcat ggcccactac tttcggcggc 300
   ggcactaaac tcgaaataaa acgtacg 327
<210> 53
   <211> 109
   <212> PRT
   <213> Homo sapiens
<400> 53
   Asp Ile Val Leu Thr Gln Ser Pro Ala Phe Leu Ser Val Thr Pro Gly
   1               5                   10                  15
   Glu Lys Val Thr Phe Thr Cys Gln Ala Ser Gln Ser Ile Gly Thr Ser
               20                  25                  30
   Ile His Trp Tyr Gln Gln Lys Thr Asp Gln Ala Pro Lys Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Thr Ile Ser Ser Val Glu Ala
   65                  70                  75                  80
   Glu Asp Ala Ala Asp Tyr Tyr Cys Gln Gln Ile Asn Ser Trp Pro Thr
                   85                  90                  95
   Thr Phe Gly Gly Gly Thr Lys Leu Glu Ile Lys Arg Thr
               100                 105
<210> 54
   <211> 99
   <212> PRT
   <213> Homo sapiens
<400> 54
   Asp Ile Leu Leu Thr Gln Ser Pro Ala Ile Leu Ser Val Ser Pro Gly
   1 5 10 15
   Glu Arg Val Ser Phe Ser Cys Arg Ala Ser Gln Ser Ile Gly Thr Ser
   20 25 30
   Ile His Trp Tyr Gln Gln Arg Thr Asn Gly Ser Pro Arg Leu Leu Ile
   35 40 45
   Lys Tyr Ala Ser Glu Ser Ile Ser Gly Ile Pro Ser Arg Phe Ser Gly
   50 55 60
   Ser Gly Ser Gly Thr Asp Phe Thr Leu Ser Ile Asn Ser Val Glu Ser
   65 70 75 80
   Glu Asp Ile Ala Asp Tyr Tyr Cys Gln Gln Ile Asn Ser Trp Pro Thr
   85 90 95
   Thr Phe Gly
<210> 55
   <211> 95
   <212> PRT
   <213> Homo sapiens
<400> 55
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro
   85 90 95
<210> 56
   <211> 101
   <212> PRT
   <213> Homo sapiens
<400> 56
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Gln Ser Leu Leu Asp Ser
               20                  25                  30
   Asp Asp Gly Asn Thr Tyr Leu Asp Trp Tyr Leu Gln Lys Pro Gly Gln
           35                  40                  45
   Ser Pro Gln Leu Leu Ile Tyr Thr Leu Ser Tyr Arg Ala Ser Gly Val
       50                  55                  60
   Pro Asp Arg Phe Ser Gly Ser Gly Ser Gly Thr Asp Phe Thr Leu Lys
   65                  70                  75                  80
   Ile Ser Arg Val Glu Ala Glu Asp Val Gly Val Tyr Tyr Cys Met Gln
                   85                  90                  95
   Arg Ile Glu Phe Pro
               100
<210> 57
   <211> 96
   <212> PRT
   <213> Homo sapiens
<400> 57
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
<210> 58
   <211> 101
   <212> PRT
   <213> Homo sapiens
<400> 58
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro
               100
<210> 59
   <211> 95
   <212> PRT
   <213> Homo sapiens
<400> 59
   Glu Thr Thr Leu Thr Gln Ser Pro Ala Phe Met Ser Ala Thr Pro Gly
   1               5                   10                  15
   Asp Lys Val Asn Ile Ser Cys Lys Ala Ser Gln Asp Ile Asp Asp Asp
               20                  25                  30
   Met Asn Trp Tyr Gln Gln Lys Pro Gly Glu Ala Ala Ile Phe Ile Ile
           35                  40                  45
   Gln Glu Ala Thr Thr Leu Val Pro Gly Ile Pro Pro Arg Phe Ser Gly
       50                  55                  60
   Ser Gly Tyr Gly Thr Asp Phe Thr Leu Thr Ile Asn Asn Ile Glu Ser
   65                  70                  75                  80
   Glu Asp Ala Ala Tyr Tyr Phe Cys Leu Gln His Asp Asn Phe Pro
                   85                  90                  95
<210> 60
   <211> 95
   <212> PRT
   <213> Homo sapiens
<400> 60
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys Tyr Ala Ser Gln Ser Phe Ser Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Thr Tyr Tyr Cys His Gln Ser Ser Ser Leu Pro
                   85                  90                  95
<210> 61
   <211> 52
   <212> PRT
   <213> Mus musculus
<400> 61
   Asp Ile Leu Leu Thr Gln Ser Pro Ala Ile Leu Ser Val Ser Pro Gly
   1 5 10 15
   Glu Arg Val Ser Phe Ser Cys Arg Ala Ser Gln Ser Ile Gly Thr Ser
   20 25 30
   Ile His Trp Tyr Gln Gln Arg Thr Asn Gly Ser Pro Arg Leu Leu Ile
   35 40 45
   Lys Tyr Ala Ser
   50
<210> 62
   <211> 57
   <212> PRT
   <213> Homo sapiens
<220>
   <221> misc_feature
   <222> (31)..(33)
   <223> Xaa= any amino acid
<220>
   <221> misc_feature
   <222> (36)..(37)
   <223> Xaa= any amino acid
<220>
   <221> misc_feature
   <222> (39)..(39)
   <223> Xaa= any amino acid
<220>
   <221> misc_feature
   <222> (55)..(55)
   <223> Xaa= any amino acid
<400> 62
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Leu Val Xaa Xaa
   20 25 30
   Xaa Ile Ser Xaa Xaa Leu Xaa Trp Tyr Gln Gln Lys Pro Gly Lys Ala
   35 40 45
   Pro Lys Leu Leu Ile Tyr Xaa Ala Ser
   50 55
<210> 63
   <211> 58
   <212> PRT
   <213> Homo sapiens
<220>
   <221> misc_feature
   <222> (33)..(33)
   <223> Xaa= any amino acid
<220>
   <221> misc_feature
   <222> (36)..(37)
   <223> Xaa= any amino acid
<220> .
   <221> misc_feature
   <222> (56)..(57)
   <223> Xaa= any amino acid
<400> 63
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Xaa Asp Gly Xaa Xaa Tyr Leu Asn Trp Tyr Leu Gln Lys Pro Gly Gln
           35                  40                  45
   Ser Pro Gln Leu Leu Ile Tyr Xaa Xaa Ser
       50                  55
<210> 64
   <211> 53
   <212> PRT
   <213> Homo sapiens
<400> 64
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser
       50
<210> 65
   <211> 58
   <212> PRT
   <213> Homo sapiens
<400> 65
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser
       50                  55
<210> 66
   <211> 52
   <212> PRT
   <213> Homo sapiens
<400> 66
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser
       50
<210> 67
   <211> 58
   <212> PRT
   <213> Homo sapiens
<400> 67
   Asp Ile Val Met Thr Gln Thr Pro Leu Ser Leu Pro Val Thr Pro Gly
   1 5 10 15
   Glu Pro Ala Ser Ile Ser Cys Arg Ser Ser Gln Ser Leu Leu Asp Ser
   20 25 30
   Asp Asp Gly Asn Thr Tyr Leu Asp Trp Tyr Leu Gln Lys Pro Gly Gln
   35 40 45
   Ser Pro Gln Leu Leu Ile Tyr Thr Leu Ser
   50 55
<210> 68
   <211> 53
   <212> PRT
   <213> Homo sapiens
<400> 68
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser
   50
<210> 69
   <211> 58
   <212> PRT
   <213> Homo sapiens
<400> 69
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser
   50 55
<210> 70
   <211> 52
   <212> PRT
   <213> Homo sapiens
<400> 70
   Glu Thr Thr Leu Thr Gln Ser Pro Ala Phe Met Ser Ala Thr Pro Gly
   1               5                   10                  15
   Asp Lys Val Asn Ile Ser Cys Lys Ala Ser Gln Asp Ile Asp Asp Asp
               20                  25                  30
   Met Asn Trp Tyr Gln Gln Lys Pro Gly Glu Ala Ala Ile Phe Ile Ile
           35                  40                  45
   Gln Glu Ala Thr
       50
<210> 71
   <211> 52
   <212> PRT
   <213> Homo sapiens
<400> 71
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys Tyr Ala Ser
       50
<210> 72
   <211> 57
   <212> PRT
   <213> Mus musculus
<400> 72
   Glu Ser Ile Ser Gly Ile Pro Ser Arg Phe Ser Gly Ser Gly Ser Gly
   1               5                   10                  15
   Thr Asp Phe Thr Leu Ser Ile Asn Ser Val Glu Ser Glu Asp Ile Ala
               20                  25                  30
   Asp Tyr Tyr Cys Gln Gln Ile Asn Ser Trp Pro Thr Thr Phe Gly Gly
   35 40 45
   Gly Thr Lys Leu Glu Ile Lys Arg Ala
   50 55
<210> 73
   <211> 58
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (39)..(42)
   <223> Xaa= any amino acid
<220>
   <221> misc_feature
   <222> (45)..(45)
   <223> Xaa= any amino acid
<400> 73
   Xaa Leu Xaa Ser Gly Val Pro Ser Arg Phe Ser Gly Ser Gly Ser Gly
   1 5 10 15
   Thr Asp Phe Thr Leu Thr Ile Ser Ser Leu Gln Pro Glu Asp Phe Ala
   20 25 30
   Thr Tyr Tyr Cys Gln Gln Xaa Xaa Xaa Xaa Pro Glu Xaa Thr Phe Gly
   35 40 45
   Gln Gly Thr Lys Val Glu Ile Lys Arg Thr
   50 55
<210> 74
   <211> 58
   <212> PRT
   <213> Homo sapiens
<220>
   <221> misc_feature
   <222> (3)..(3)
   <223> Xaa= any amino acid
<220>
   <221> misc_feature
   <222> (40)..(40)
   <223> Xaa= any amino acid
<220>
   <221> misc_feature
   <222> (42)..(42)
   <223> Xaa= any amino acid
<220>
   <221> misc_feature
   <222> (45)..(45)
   <223> Xaa= any amino acid
<400> 74
   Asn Arg Xaa Ser Gly Val Pro Asp Arg Phe Ser Gly Ser Gly Ser Gly
   1               5                   10                  15
   Thr Asp Phe Thr Leu Lys Ile Ser Arg Val Glu Ala Glu Asp Val Gly
               20                  25                  30
   Val Tyr Tyr Cys Met Gln Ala Xaa Gln Xaa Pro Arg Xaa Thr Phe Gly
           35                  40                  45
   Gln Gly Thr Lys Val Glu Ile Lys Arg Thr
       50                  55
<210> 75
   <211> 58
   <212> PRT
   <213> Homo sapiens
<220>
   <221> misc_feature
   <222> (45)..(45)
   <223> Xaa= any amino acid
<400> 75
   Ser Arg Ala Thr Gly Ile Pro Asp Arg Phe Ser Gly Ser Gly Ser Gly
   1               5                   10                  15
   Thr Asp Phe Thr Leu Thr Ile Ser Arg Leu Glu Pro Glu Asp Phe Ala
               20                  25                  30
   Val Tyr Tyr Cys Gln Gln Tyr Gly Ser Ser Pro Pro Xaa Thr Phe Gly
           35                  40                  45
   Gln Gly Thr Lys Val Glu Ile Lys Arg Thr
       50                  55
<210> 76
   <211> 57
   <212> PRT
   <213> Homo sapiens
<220>
   <221> misc_feature
   <222> (44)..(44)
   <223> Xaa= any amino acid
<400> 76
   Thr Arg Glu Ser Gly Val Pro Asp Arg Phe Ser Gly Ser Gly Ser Gly
   1               5                   10                  15
   Thr Asp Phe Thr Leu Thr Ile Ser Ser Leu Gln Ala Glu Asp Val Ala
               20                  25                  30
   Val Tyr Tyr Cys Gln Gln Tyr Tyr Ser Thr Pro Xaa Thr Phe Gly Gln
           35                  40                  45
   Gly Thr Lys Val Glu Ile Lys Arg Thr
       50                  55
<210> 77
   <211> 57
   <212> PRT
   <213> Homo sapiens
<400> 77
   Ser Leu Gln Ser Gly Val Pro Ser Arg Phe Ser Gly Ser Gly Ser Gly
   1               5                   10                  15
   Thr Asp Phe Thr Leu Thr Ile Ser Ser Leu Gln Pro Glu Asp Phe Ala
               20                  25                  30
   Thr Tyr Tyr Cys Gln Gln Ser Tyr Ser Thr Pro Leu Thr Phe Gly Gly
           35                  40                  45
   Gly Thr Lys Val Glu Ile Lys Arg Thr
       50                  55
<210> 78
   <211> 57
   <212> PRT
   <213> Homo sapiens
<400> 78
   Tyr Arg Ala Ser Gly Val Pro Asp Arg Phe Ser Gly Ser Gly Ser Gly
   1               5                   10                  15
   Thr Asp Phe Thr Leu Lys Ile Ser Arg Val Glu Ala Glu Asp Val Gly
   20 25 30
   Val Tyr Tyr Cys Met Gln Arg Ile Glu Phe Pro Leu Thr Phe Gly Gly
   35 40 45
   Gly Thr Lys Val Glu Ile Lys Arg Thr
   50 55
<210> 79
   <211> 57
   <212> PRT
   <213> Homo sapiens
<400> 79
   Ser Arg Ala Thr Gly Ile Pro Asp Arg Phe Ser Gly Ser Gly Ser Gly
   1 5 10 15
   Thr Asp Phe Thr Leu Thr Ile Ser Arg Leu Glu Pro Glu Asp Phe Ala
   20 25 30
   Val Tyr Tyr Cys Gln Gln Tyr Gly Ser Ser Pro Leu Thr Phe Gly Gly
   35 40 45
   Gly Thr Lys Val Glu Ile Lys Arg Thr
   50 55
<210> 80
   <211> 57
   <212> PRT
   <213> Homo sapiens
<400> 80
   Thr Arg Glu Ser Gly Val Pro Asp Arg Phe Ser Gly Ser Gly Ser Gly
   1 5 10 15
   Thr Asp Phe Thr Leu Thr Ile Ser Ser Leu Gln Ala Glu Asp Val Ala
   20 25 30
   Val Tyr Tyr Cys Gln Gln Tyr Tyr Ser Thr Pro Leu Thr Phe Gly Gly
   35 40 45
   Gly Thr Lys Val Glu Ile Lys Arg Thr
   50 55
<210> 81
   <211> 57
   <212> PRT
   <213> Homo sapiens
<400> 81
   Thr Leu Val Pro Gly Ile Pro Pro Arg Phe Ser Gly Ser Gly Tyr Gly
   1               5                   10                  15
   Thr Asp Phe Thr Leu Thr Ile Asn Asn Ile Glu Ser Glu Asp Ala Ala
               20                  25                  30
   Tyr Tyr Phe Cys Leu Gln His Asp Asn Phe Pro Leu Thr Phe Gly Gly
           35                  40                  45
   Gly Thr Lys Val Glu Ile Lys Arg Thr
       50                  55
<210> 82
   <211> 57
   <212> PRT
   <213> Homo sapiens
<400> 82
   Gln Ser Phe Ser Gly Val Pro Ser Arg Phe Ser Gly Ser Gly Ser Gly
   1               5                   10                  15
   Thr Asp Phe Thr Leu Thr Ile Asn Ser Leu Glu Ala Glu Asp Ala Ala
               20                  25                  30
   Thr Tyr Tyr Cys His Gln Ser Ser Ser Leu Pro Leu Thr Phe Gly Gly
           35                  40                  45
   Gly Thr Lys Val Glu Ile Lys Arg Thr
       50                  55
<210> 83
   <211> 101
   <212> PRT
   <213> Homo sapiens
<400> 83
   Asp Val Gln Leu Gln Glu Ser Gly Pro Gly Leu Val Lys Pro Ser Gln
   1               5                   10                  15
   Ser Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Ser Ile Thr Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Ser Phe Asp Tyr
   100
<210> 84
   <211> 98
   <212> PRT
   <213> Homo sapiens
<400> 84
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg
<210> 85
   <211> 100
   <212> PRT
   <213> Homo sapiens
<400> 85
   Gln Ile Thr Leu Lys Glu Ser Gly Pro Thr Leu Val Lys Pro Thr Gln
   1 5 10 15
   Thr Leu Thr Leu Thr Cys Thr Phe Ser Gly Phe Ser Leu Ser Thr Ser
   20 25 30
   Gly Val Gly Val Gly Trp Ile Arg Gln Pro Pro Gly Lys Ala Leu Glu
   35 40 45
   Trp Leu Ala Leu Ile Tyr Trp Asn Asp Asp Lys Arg Tyr Ser Pro Ser
   50 55 60
   Leu Lys Ser Arg Leu Thr Ile Thr Lys Asp Thr Ser Lys Asn Gln Val
   65 70 75 80
   Val Leu Thr Met Thr Asn Met Asp Pro Val Asp Thr Ala Thr Tyr Tyr
   85 90 95
   Cys Ala His Arg
   100
<210> 86
   <211> 98
   <212> PRT
   <213> Homo sapiens
<400> 86
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Trp Met Ser Trp Val Arg Gln Ala Pro Gly Lys Gly Leu Glu Trp Val
   35 40 45
   Ala Asn Ile Lys Gln Asp Gly Ser Glu Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg
<210> 87
   <211> 98
   <212> PRT
   <213> Homo sapiens
<400> 87
   Gln Val Gln Leu Gln Glu Ser Gly Pro Gly Leu Val Lys Pro Ser Gly
   1 5 10 15
   Thr Leu Ser Leu Thr Cys Ala Val Ser Gly Gly Ser Ile Ser Ser Ser
   20 25 30
   Asn Trp Trp Ser Trp Val Arg Gln Pro Pro Gly Lys Gly Leu Glu Trp
   35 40 45
   Ile Gly Glu Ile Tyr His Ser Gly Ser Thr Asn Tyr Asn Pro Ser Leu
   50 55 60
   Lys Ser Arg Val Thr Ile Ser Val Asp Lys Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg
<210> 88
   <211> 98
   <212> PRT
   <213> Homo sapiens
<400> 88
   Glu Val Gln Leu Val Gln Ser Gly Ala Glu Val Lys Lys Pro Gly Glu
   1 5 10 15
   Ser Leu Lys Ile Ser Cys Lys Gly Ser Gly Tyr Ser Phe Thr Ser Tyr
   20 25 30
   Trp Ile Gly Trp Val Arg Gln Met Pro Gly Lys Gly Leu Glu Trp Met
   35 40 45
   Gly Ile Ile Tyr Pro Gly Asp Ser Asp Thr Arg Tyr Ser Pro Ser Phe
   50 55 60
   Gln Gly Gln Val Thr Ile Ser Ala Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg
<210> 89
   <211> 101
   <212> PRT
   <213> Homo sapiens
<400> 89
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Asn
   20 25 30
   Ser Ala Ala Trp Asn Trp Ile Arg Gln Ser Pro Ser Arg Gly Leu Glu
   35 40 45
   Trp Leu Gly Arg Thr Tyr Tyr Arg Ser Lys Trp Tyr Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg
   100
<210> 90
   <211> 98
   <212> PRT
   <213> Homo sapiens
<400> 90
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Ser Tyr
   20 25 30
   Ala Met Asn Trp Val Arg Gln Ala Pro Gly Gln Gly Leu Glu Trp Met
   35 40 45
   Gly Trp Ile Asn Thr Asn Thr Gly Asn Pro Thr Tyr Ala Gln Gly Phe
   50 55 60
   Thr Gly Arg Phe Val Phe Ser Leu Asp Thr Ser Val Ser Thr Ala Tyr
   65 70 75 80
   Leu Gln Ile Cys Ser Leu Lys Ala Glu Asp Thr Ala Val Tyr Tyr Cys
   85 90 95
   Ala Arg
<210> 91
   <211> 58
   <212> PRT
   <213> Mus musculus
<400> 91
   Asp Val Gln Leu Gln Glu Ser Gly Pro Gly Leu Val Lys Pro Ser Gln
   1               5                   10                  15
   Ser Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Asn Lys Leu Glu Trp
           35                  40                  45
   Met Gly Tyr Ile Ser Tyr Ser Gly Ser Thr
       50                  55
<210> 92
   <211> 59
   <212> PRT
   <213> Homo sapiens
<220>
   <221> misc_feature
   <222> (1)..(1)
   <223> Xaa= any amino acid
<220>
   <221> misc_feature
   <222> (16)..(16)
   <223> Xaa= any amino acid
<220>
   <221> misc_feature
   <222> (30)..(30)
   <223> Xaa= any amino acid
<220>
   <221> misc_feature
   <222> (33)..(33)
   <223> Xaa= any amino acid
<220>
   <221> misc_feature
   <222> (35)..(35)
   <223> Xaa= any amino acid
<220>
   <221> misc_feature
   <222> (50)..(50)
   <223> Xaa= any amino acid
<220>
   <221> misc_feature
   <222> (52)..(52)
   <223> Xaa= any amino acid
<220>
   <221> misc_feature
   <222> (55)..(56)
   <223> Xaa= any amino acid
<220>
   <221> misc_feature
   <222> (58)..(58)
   <223> Xaa= any amino acid
<400> 92
   Xaa Val Gln Leu Val Gln Ser Gly Ala Glu Val Lys Lys Pro Gly Xaa
   1               5                   10                  15
   Ser Val Lys Val Ser Cys Lys Ala Ser Gly Tyr Thr Phe Xaa Ser Tyr
               20                  25                  30
   Xaa Ile Xaa Trp Val Arg Gln Ala Pro Gly Gln Gly Leu Glu Trp Met
           35                  40                  45
   Gly Xaa Ile Xaa Pro Tyr Xaa Xaa Gly Xaa Thr
       50                  55
<210> 93
   <211> 62
   <212> PRT
   <213> Homo sapiens
<220>
   <221> misc_feature
   <222> (23)..(23)
   <223> Xaa= any amino acid
<220>
   <221> misc_feature
   <222> (27)..(27)
   <223> Xaa= any amino acid
<220>
   <221> misc_feature
   <222> (29)..(29)
   <223> Xaa= any amino acid
<220>
   <221> misc_feature
   <222> (32)..(37)
   <223> Xaa= any amino acid
<220>
   <221> misc_feature
   <222> (52)..(52)
   <223> Xaa= any amino acid
<220>
   <221> misc_feature
   <222> (58)..(59)
   <223> Xaa= any amino acid
<220>
   <221> misc_feature
   <222> (61)..(61)
   <223> Xaa= any amino acid
<400> 93
   Gln Val Gln Leu Gln Glu Ser Gly Pro Gly Leu Val Lys Pro Ser Gln
   1               5                   10                  15
   Thr Leu Ser Leu Thr Cys Xaa Val Ser Gly Xaa Ser Xaa Ser Ser Xaa
               20                  25                  30
   Xaa Xaa Xaa Xaa Xaa Trp Ile Arg Gln Pro Pro Gly Lys Gly Leu Glu
           35                  40                  45
   Trp Ile Gly Xaa Ile Tyr Tyr Arg Ala Xaa Xaa Gly Xaa Thr
       50                  55                  60
<210> 94
   <211> 60
   <212> PRT
   <213> Homo sapiens
<220>
   <221> misc_feature
   <222> (31)..(31)
   <223> Xaa= any amino acid
<220>
   <221> misc_feature
   <222> (33)..(33)
   <223> Xaa= any amino acid
<220>
   <221> misc_feature
   <222> (35)..(35)
   <223> Xaa= any amino acid
<220>
   <221> misc_feature
   <222> (49)..(50)
   <223> Xaa= any amino acid
<220>.
   <221> misc_feature
   <222> (52)..(53)
   <223> Xaa= any amino acid
<220>
   <221> misc_feature
   <222> ,(55).. (56)
   <223> Xaa= any amino acid
<220>
   <221> misc_feature
   <222> (58)..(59)
   <223> Xaa= any amino acid
<400> 94
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Xaa Tyr
               20                  25                  30
   Xaa Met Xaa Trp Val Arg Gln Ala Pro Gly Lys Gly Leu Glu Trp Val
           35                  40                  45
   Xaa Xaa Ile Xaa Xaa Lys Xaa Xaa Gly Xaa Xaa Thr
       50                  55                  60
<210> 95
   <211> 58
   <212> PRT
   <213> Homo sapiens
<400> 95
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr
       50                  55
<210> 96
   <211> 59
   <212> PRT
   <213> Homo sapiens
<400> 96
   Gln Ile Thr Leu Lys Glu Ser Gly Pro Thr Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Asp Asp Lys
       50                  55
<210> 97
   <211> 58
   <212> PRT
   <213> Homo sapiens
<400> 97
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Glu Lys
       50                  55
<210> 98
   <211> 58
   <212> PRT
   <213> Homo sapiens
<400> 98
   Gln Val Gln Leu Gln Glu Ser Gly Pro Gly Leu Val Lys Pro Ser Gly
   1               5                   10                  15
   Thr Leu Ser Leu Thr Cys Ala Val Ser Gly Gly Ser Ile Ser Ser Ser
               20                  25                  30
   Asn Trp Trp Ser Trp Val Arg Gln Pro Pro Gly Lys Gly Leu Glu Trp
           35                  40                  45
   Ile Gly Glu Ile Tyr His Ser Gly Ser Thr
       50                  55
<210> 99
   <211> 58
   <212> PRT
   <213> Homo sapiens
<400> 99
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr
       50                  55
<210> 100
   <211> 61
   <212> PRT
   <213> Homo sapiens
<400> 100
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn
       50                  55                  60
<210> 101
   <211> 58
   <212> PRT
   <213> Homo sapiens
<400> 101
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Ser Tyr
               20                  25                  30
   Ala Met Asn Trp Val Arg Gln Ala Pro Gly Gln Gly Leu Glu Trp Met
           35                  40                  45
   Gly Trp Ile Asn Thr Asn Thr Gly Asn Pro
       50                  55
<210> 102
   <211> 60
   <212> PRT
   <213> Mus musculus
<400> 102
   Ser Tyr Asn Pro Ser Leu Lys Ser Arg Ile Ser Ile Thr Arg Asp Thr
   1               5                   10                  15
   Ser Lys Asn Gln Phe Phe Leu Gln Leu Asn Ser Val Thr Thr Glu Asp
               20                  25                  30
   Thr Ala Thr Tyr Tyr Cys Ala Ser Phe Asp Tyr Ala His Ala Met Asp
           35                  40                  45
   Tyr Trp Gly Gln Gly Thr Ser Val Thr Val Ser Ser
       50                  55                  60
<210> 103
   <211> 70
   <212> PRT
   <213> Homo sapiens
<220>
   <221> misc_feature
   <222> (14)..(14)
   <223> Xaa= any amino acid
<220>
   <221> misc_feature
   <222> (16)..(16)
   <223> Xaa= any amino acid
<220>
   <221> misc_feature
   <222> (31)..(31)
   <223> Xaa= any amino acid
<220>
   <221> misc_feature
   <222> (41)..(53)
   <223> Xaa= any amino acid
<220>
   <221> misc_feature
   <222> (55)..(56)
   <223> Xaa= any amino acid
<220>
   <221> misc_feature
   <222> (59)..(59)
   <223> Xaa= any amino acid
<400> 103
   Asn Tyr Ala Gln Lys Phe Gln Gly Arg Val Thr Ile Thr Xaa Asp Xaa
   1               5                   10                  15
   Ser Thr Ser Thr Ala Tyr Met Glu Leu Ser Ser Leu Arg Ser Xaa Asp
               20                  25                  30
   Thr Ala Val Tyr Tyr Cys Ala Arg Xaa Xaa Xaa Xaa Xaa Xaa Xaa Xaa
           35                  40                  45
   Xaa Xaa Xaa Xaa Xaa Asp Xaa Xaa Phe Asp Xaa Trp Gly Gln Gly Thr
       50                  55                  60
   Leu Val Thr Val Ser Ser
   65                  70
<210> 104
   <211> 70
   <212> PRT
   <213> Homo sapiens
<220>
   <221> misc_feature
   <222> (1)..(1)
   <223> Xaa= any amino acid
<220>
   <221> misc_feature
   <222> (24)..(24)
   <223> Xaa= any amino acid
<220>
   <221> misc_feature
   <222> (26)..(26)
   <223> Xaa= any amino acid
<220>
   <221> misc_feature
   <222> (41)..(56)
   <223> Xaa= any amino acid
<220>
   <221> misc_feature
   <222> (59)..(59)
   <223> Xaa= any amino acid
<220>
   <221> misc_feature
   <222> (65)..(65)
   <223> Xaa= any amino acid
<400> 104
   Xaa Tyr Asn Pro Ser Leu Lys Ser Arg Val Thr Ile Ser Val Asp Thr
   1               5                   10                  15
   Ser Lys Asn Gln Phe Ser Leu Xaa Leu Xaa Ser Val Thr Ala Ala Asp
               20                  25                  30
   Thr Ala Val Tyr Tyr Cys Ala Arg Xaa Xaa Xaa Xaa Xaa Xaa Xaa Xaa
           35                  40                  45
   Xaa Xaa Xaa Xaa Xaa Xaa Xaa Xaa Phe Asp Xaa Trp Gly Gln Gly Thr
       50                  55                  60
   Xaa Val Thr Val Ser Ser
   65                  70
<210> 105
   <211> 70
   <212> PRT
   <213> Homo sapiens
<220>
   <221> misc_feature
   <222> (40)..(52)
   <223> Xaa= any amino acid
<220>
   <221> misc_feature
   <222> (55)..(56)
   <223> Xaa= any amino acid
<220>
   <221> misc_feature
   <222> (59)..(59)
   <223> Xaa= any amino acid
<400> 105
   Tyr Tyr Ala Asp Ser Val Lys Gly Arg Phe Thr Ile Ser Arg Asp Asn
   1 5 10 15
   Ser Lys Asn Thr Leu Tyr Leu Gln Met Asn Ser Leu Arg Ala Glu Asp
   20 25 30
   Thr Ala Val Tyr Tyr Cys Ala Xaa Xaa Xaa Xaa Xaa Xaa Xaa Xaa Xaa
   35 40 45
   Xaa Xaa Xaa Xaa Tyr Tyr Xaa Xaa Phe Asp Xaa Trp Gly Gln Gly Thr
   50 55 60
   Leu Val Thr Val Ser Ser
   65 70
<210> 106
   <211> 70
   <212> PRT
   <213> Homo sapiens
<220>
   <221> misc_feature
   <222> (41)..(55)
   <223> Xaa= any amino acid
<400> 106
   Asn Tyr Ala Gln Lys Phe Gln Gly Arg Val Thr Met Thr Arg Asp Thr
   1 5 10 15
   Ser Ile Ser Thr Ala Tyr Met Glu Leu Ser Arg Leu Arg Ser Asp Asp
   20 25 30
   Thr Ala Val Tyr Tyr Cys Ala Arg Xaa Xaa Xaa Xaa Xaa Xaa Xaa Xaa
   35 40 45
   Xaa Xaa Xaa Xaa Xaa Xaa Xaa Tyr Phe Asp Tyr Trp Gly Gln Gly Thr
   50 55 60
   Leu Val Thr Val Ser Ser
   65 70
<210> 107
   <211> 70
   <212> PRT
   <213> Homo sapiens
<220>
   <221> misc_feature
   <222> (42)..(55)
   <223> Xaa= any amino acid
<400> 107
   Arg Tyr Ser Pro Ser Leu Lys Ser Arg Leu Thr Ile Thr Lys Asp Thr
   1 5 10 15
   Ser Lys Asn Gln Val Val Leu Thr Met Thr Asn Met Asp Pro Val Asp
   20 25 30
   Thr Ala Thr Tyr Tyr Cys Ala His Arg Xaa Xaa Xaa Xaa Xaa Xaa Xaa
   35 40 45
   Xaa Xaa Xaa Xaa Xaa Xaa Xaa Tyr Phe Asp Tyr Trp Gly Gln Gly Thr
   50 55 60
   Leu Val Thr Val Ser Ser
   65 70
<210> 108
   <211> 70
   <212> PRT
   <213> Homo sapiens
<220>
   <221> misc_feature
   <222> (41)..(55)
   <223> Xaa= any amino acid
<400> 108
   Tyr Tyr Val Asp Ser Val Lys Gly Arg Phe Thr Ile Ser Arg Asp Asn
   1 5 10 15
   Ala Lys Asn Ser Leu Tyr Leu Gln Met Asn Ser Leu Arg Ala Glu Asp
   20 25 30
   Thr Ala Val Tyr Tyr Cys Ala Arg Xaa Xaa Xaa Xaa Xaa Xaa Xaa Xaa
   35 40 45
   Xaa Xaa Xaa Xaa Xaa Xaa Xaa Tyr Phe Asp Tyr Trp Gly Gln Gly Thr
   50 55 60
   Leu Val Thr Val Ser Ser
   65 70
<210> 109
   <211> 70
   <212> PRT
   <213> Homo sapiens
<220>
   <221> misc_feature
   <222> (41)..(55)
   <223> Xaa= any amino acid
<400> 109
   Asn Tyr Asn Pro Ser Leu Lys Ser Arg Val Thr Ile Ser Val Asp Lys
   1               5                   10                  15
   Ser Lys Asn Gln Phe Ser Leu Lys Leu Ser Ser Val Thr Ala Ala Asp
               20                  25                  30
   Thr Ala Val Tyr Tyr Cys Ala Arg Xaa Xaa Xaa Xaa Xaa Xaa Xaa Xaa
           35                  40                  45
   Xaa Xaa Xaa Xaa Xaa Xaa Xaa Tyr Phe Asp Tyr Trp Gly Gln Gly Thr
       50                  55                  60
   Leu Val Thr Val Ser Ser
   65                  70
<210> 110
   <211> 70
   <212> PRT
   <213> Homo sapiens
<220>
   <221> misc_feature
   <222> (41)..(55)
   <223> Xaa= any amino acid
<400> 110
   Arg Tyr Ser Pro Ser Phe Gln Gly Gln Val Thr Ile Ser Ala Asp Lys
   1               5                   10                  15
   Ser Ile Ser Thr Ala Tyr Leu Gln Trp Ser Ser Leu Lys Ala Ser Asp
               20                  25                  30
   Thr Ala Met Tyr Tyr Cys Ala Arg Xaa Xaa Xaa Xaa Xaa Xaa Xaa Xaa
           35                  40                  45
   Xaa Xaa Xaa Xaa Xaa Xaa Xaa Tyr Phe Asp Tyr Trp Gly Gln Gly Thr
       50                  55                  60
   Leu Val Thr Val Ser Ser
   65                  70
<210> 111
   <211> 70
   <212> PRT
   <213> Homo sapiens
<220>
   <221> misc_feature
   <222> (41)..(55)
   <223> Xaa= any amino acid
<400> 111
   Asp Tyr Ala Val Ser Val Lys Ser Arg Ile Thr Ile Asn Pro Asp Thr
   1               5                   10                  15
   Ser Lys Asn Gln Phe Ser Leu Gln Leu Asn Ser Val Thr Pro Glu Asp
               20                  25                  30
   Thr Ala Val Tyr Tyr Cys Ala Arg Xaa Xaa Xaa Xaa Xaa Xaa Xaa Xaa
           35                  40                  45
   Xaa Xaa Xaa Xaa Xaa Xaa Xaa Tyr Phe Asp Tyr Trp Gly Gln Gly Thr
       50                  55                  60
   Leu Val Thr Val Ser Ser
   65                  70
<210> 112
   <211> 70
   <212> PRT
   <213> Homo sapiens
<220>
   <221> misc_feature
   <222> (41)..(55)
   <223> Xaa= any amino acid
<400> 112
   Thr Tyr Ala Gln Gly Phe Thr Gly Arg Phe Val Phe Ser Leu Asp Thr
   1               5                   10                  15
   Ser Val Ser Thr Ala Tyr Leu Gln Ile Cys Ser Leu Lys Ala Glu Asp
               20                  25                  30
   Thr Ala Val Tyr Tyr Cys Ala Arg Xaa Xaa Xaa Xaa Xaa Xaa Xaa Xaa
           35                  40                  45
   Xaa Xaa Xaa Xaa Xaa Xaa Xaa Tyr Phe Asp Tyr Trp Gly Gln Gly Thr
       50                  55                  60
   Leu Val Thr Val Ser Ser
   65                  70
<210> 113
   <211> 1404
   <212> DNA
   <213> Homo sapiens
<400> 113
   atgggatgga gttgcattat acttttcctc gttgccaccg ccactggagt tcactctgac     60
   gtacaacttc aagaatctgg cccaggtctc gtcaaacctt ctcaaactct ctcactcacc    120
   tgcactgtta ctgactactc tattacatcc gactacgctt ggaactggat ccgacaattt    180
   cctggtaaaa aactcgaatg gatgggttat atttcttact ctggctccac ctcctacaat    240
   ccttctctga aatcacgcat cacaatttcc cgcgatacct ctaaaaatca attttcactc    300
   caactcaatt ctgttaccgc cgccgatact gccacctact actgtgcctc ttttgactac    360
   gctcacgcca tggattattg gggacagggt actaccgtta ccgtaagctc agccagcaca    420
   aagggcccat cggtcttccc cctggcaccc tcctccaaga gcacctctgg gggcacagcg    480
   gccctgggct gcctggtcaa ggactacttc cccgaaccgg tgacggtgtc gtggaactca    540
   ggcgccctga ccagcggcgt gcacaccttc ccggctgtcc tacagtcctc aggactctac    600
   tccctcagca gcgtggtgac cgtgccctcc agcagcttgg gcacccagac ctacatctgc    660
   aacgtgaatc acaagcccag caacaccaag gtggacaaga gagttgagcc caaatcttgt    720
   gacaaaactc acacatgtcc accgtgccca gcacctgaac tcctgggggg accgtcagtc    780
   ttcctcttcc ccccaaaacc caaggacacc ctcatgatct cccggacccc tgaggtcaca    840
   tgcgtggtgg tggacgtgag ccacgaagac cctgaggtca agttcaactg gtacgtggac    900
   ggcgtggagg tgcataatgc caagacaaag ccgcgggagg agcagtacaa cagcacgtac    960
   cgtgtggtca gcgtcctcac cgtcctgcac caggactggc tgaatggcaa ggagtacaag   1020
   tgcaaggtct ccaacaaagc cctcccagcc cccatcgaga aaaccatctc caaagccaaa   1080
   gggcagcccc gagaaccaca ggtgtacacc ctgcccccat cccgggagga gatgaccaag   1140
   aaccaggtca gcctgacctg cctggtcaaa ggcttctatc ccagcgacat cgccgtggag   1200
   tgggagagca atgggcagcc ggagaacaac tacaagacca cgcctcccgt gctggactcc   1260
   gacggctcct tcttcctcta tagcaagctc accgtggaca agagcaggtg gcagcagggg   1320
   aacgtcttct catgctccgt gatgcatgag gctctgcaca accactacac gcagaagagc   1380
   ctctccctgt ccccgggtaa atga                                          1404
<210> 114
   <211> 467
   <212> PRT
   <213> Homo sapiens
<400> 114
   Met Gly Trp Ser Cys Ile Ile Leu Phe Leu Val Ala Thr Ala Thr Gly
   1               5                   10                  15
   Val His Ser Asp Val Gln Leu Gln Glu Ser Gly Pro Gly Leu Val Lys
               20                  25                  30
   Pro Ser Gln Thr Leu Ser Leu Thr Cys Thr Val Thr Asp Tyr Ser Ile
           35                  40                  45
   Thr Ser Asp Tyr Ala Trp Asn Trp Ile Arg Gln Phe Pro Gly Lys Lys
       50                  55                  60
   Leu Glu Trp Met Gly Tyr Ile Ser Tyr Ser Gly Ser Thr Ser Tyr Asn
   65                  70                  75                  80
   Pro Ser Leu Lys Ser Arg Ile Thr Ile Ser Arg Asp Thr Ser Lys Asn
                   85                  90                  95
   Gln Phe Ser Leu Gln Leu Asn Ser Val Thr Ala Ala Asp Thr Ala Thr
               100                 105                 110
   Tyr Tyr Cys Ala Ser Phe Asp Tyr Ala His Ala Met Asp Tyr Trp Gly
           115                 120                 125
   Gln Gly Thr Thr Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Arg Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Asn Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Glu Glu Met Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465
<210> 115
   <211> 1404
   <212> DNA
   <213> Homo sapiens
<400> 115
   atgggttggt cttgcatcat tctctttctc gtcgctaccg caactggtgt acactcccaa 60
   gttcaacttc aagaatcagg ccccggactc gttaaaccct ctcaaactct ctctcttact 120
   tgcactgtat ccgattactc tattacttca gactacgctt ggaactggat cagacaattt 180
   cccggaaaag gactcgaatg gatgggatat atctcttact ctggctcaac ctcttacaac 240
   ccctctctca aatctcgaat aacaatctca cgcgatactt ctaaaaatca attctcactt 300
   caacttaact ccgttactgc cgccgacact gccgtttact actgtgcttc cttcgattac 360
   gcccacgcta tggattattg gggacaagga actaccgtca ctgtcagctc agccagcaca 420
   aagggcccat cggtcttccc cctggcaccc tcctccaaga gcacctctgg gggcacagcg 480
   gccctgggct gcctggtcaa ggactacttc cccgaaccgg tgacggtgtc gtggaactca 540
   ggcgccctga ccagcggcgt gcacaccttc ccggctgtcc tacagtcctc aggactctac 600
   tccctcagca gcgtggtgac cgtgccctcc agcagcttgg gcacccagac ctacatctgc 660
   aacgtgaatc acaagcccag caacaccaag gtggacaaga gagttgagcc caaatcttgt 720
   gacaaaactc acacatgtcc accgtgccca gcacctgaac tcctgggggg accgtcagtc 780
   ttcctcttcc ccccaaaacc caaggacacc ctcatgatct cccggacccc tgaggtcaca 840
   tgcgtggtgg tggacgtgag ccacgaagac cctgaggtca agttcaactg gtacgtggac 900
   ggcgtggagg tgcataatgc caagacaaag ccgcgggagg agcagtacaa cagcacgtac 960
   cgtgtggtca gcgtcctcac cgtcctgcac caggactggc tgaatggcaa ggagtacaag 1020
   tgcaaggtct ccaacaaagc cctcccagcc cccatcgaga aaaccatctc caaagccaaa 1080
   gggcagcccc gagaaccaca ggtgtacacc ctgcccccat cccgggagga gatgaccaag 1140
   aaccaggtca gcctgacctg cctggtcaaa ggcttctatc ccagcgacat cgccgtggag 1200
   tgggagagca atgggcagcc ggagaacaac tacaagacca cgcctcccgt gctggactcc 1260
   gacggctcct tcttcctcta tagcaagctc accgtggaca agagcaggtg gcagcagggg 1320
   aacgtcttct catgctccgt gatgcatgag gctctgcaca accactacac gcagaagagc 1380
   ctctccctgt ccccgggtaa atga 1404
<210> 116
   <211> 467
   <212> PRT
   <213> Homo sapiens
<400> 116
<210> 117
   <211> 2002
   <212> DNA
   <213> Homo sapiens
<400> 117
   atgggatgga gttgcattat acttttcctc gttgccaccg ccactggagt tcactctgac     60
   gtacaacttc aagaatctgg cccaggtctc gtcaaacctt ctcaaactct ctcactcacc    120
   tgcactgtta ctgactactc tattacatcc gactacgctt ggaactggat ccgacaattt    180
   cctggtaaaa aactcgaatg gatgggttat atttcttact ctggctccac ctcctacaat    240
   ccttctctga aatcacgcat cacaatttcc cgcgatacct ctaaaaatca attttcactc    300
   caactcaatt ctgttaccgc cgccgatact gccacctact actgtgcctc ttttgactac    360
   gctcacgcca tggattattg gggacagggt actaccgtta ccgtaagctc agccagcaca    420
   aagggcccat ccgtcttccc cctggcgccc tgctccagga gcacctccga gagcacagcc    480
   gccctgggct gcctggtcaa ggactacttc cccgaaccgg tgacggtgtc gtggaactca    540
   ggcgccctga ccagcggcgt gcacaccttc ccggctgtcc tacagtcctc aggactctac    600
   tccctcagca gcgtggtgac cgtgccctcc agcagcttgg gcacgaagac ctacacctgc    660
   aacgtagatc acaagcccag caacaccaag gtggacaaga gagttggtga gaggccagca    720
   cagggaggga gggtgtctgc tggaagccag gctcagccct cctgcctgga cgcaccccgg    780
   ctgtgcagcc ccagcccagg gcagcaaggc atgccccatc tgtctcctca cccggaggcc    840
   tctgaccacc ccactcatgc tcagggagag ggtcttctgg atttttccac caggctccgg    900
   gcagccacag gctggatgcc cctaccccag gccctgcgca tacaggggca ggtgctgcgc    960
   tcagacctgc caagagccat atccgggagg accctgcccc tgacctaagc ccaccccaaa   1020
   ggccaaactc tccactccct cagctcagac accttctctc ctcccagatc tgagtaactc   1080
   ccaatcttct ctctgcagag tccaaatatg gtcccccatg cccatcatgc ccaggtaagc   1140
   caacccaggc ctcgccctcc agctcaaggc gggacaggtg ccctagagta gcctgcatcc   1200
   agggacaggc cccagccggg tgctgacgca tccacctcca tctcttcctc agcacctgag   1260
   ttcctggggg gaccatcagt cttcctgttc cccccaaaac ccaaggacac tctcatgatc   1320
   tcccggaccc ctgaggtcac gtgcgtggtg gtggacgtga gccaggaaga ccccgaggtc   1380
   cagttcaact ggtacgtgga tggcgtggag gtgcataatg ccaagacaaa gccgcgggag   1440
   gagcagttca acagcacgta ccgtgtggtc agcgtcctca ccgtcctgca ccaggactgg   1500
   ctgaacggca aggagtacaa gtgcaaggtc tccaacaaag gcctcccgtc ctccatcgag 1560
   aaaaccatct ccaaagccaa aggtgggacc cacggggtgc gagggccaca tggacagagg 1620
   tcagctcggc ccaccctctg ccctgggagt gaccgctgtg ccaacctctg tccctacagg 1680
   gcagccccga gagccacagg tgtacaccct gcccccatcc caggaggaga tgaccaagaa 1740
   ccaggtcagc ctgacctgcc tggtcaaagg cttctacccc agcgacatcg ccgtggagtg 1800
   ggagagcaat gggcagccgg agaacaacta caagaccacg cctcccgtgc tggactccga 1860
   cggctccttc ttcctctaca gcaggctaac cgtggacaag agcaggtggc aggaggggaa 1920
   tgtcttctca tgctccgtga tgcatgaggc tctgcacaac cactacacac agaagagcct 1980
   ctccctgtct ctgggtaaat ga 2002
<210> 118
   <211> 1395
   <212> DNA
   <213> Homo sapiens
<400> 118
   atgggatgga gttgcattat acttttcctc gttgccaccg ccactggagt tcactctgac 60
   gtacaacttc aagaatctgg cccaggtctc gtcaaacctt ctcaaactct ctcactcacc 120
   tgcactgtta ctgactactc tattacatcc gactacgctt ggaactggat ccgacaattt 180
   cctggtaaaa aactcgaatg gatgggttat atttcttact ctggctccac ctcctacaat 240
   ccttctctga aatcacgcat cacaatttcc cgcgatacct ctaaaaatca attttcactc 300
   caactcaatt ctgttaccgc cgccgatact gccacctact actgtgcctc ttttgactac 360
   gctcacgcca tggattattg gggacagggt actaccgtta ccgtaagctc agccagcaca 420
   aagggcccat ccgtcttccc cctggcgccc tgctccagga gcacctccga gagcacagcc 480
   gccctgggct gcctggtcaa ggactacttc cccgaaccgg tgacggtgtc gtggaactca 540
   ggcgccctga ccagcggcgt gcacaccttc ccggctgtcc tacagtcctc aggactctac 600
   tccctcagca gcgtggtgac cgtgccctcc agcagcttgg gcacgaagac ctacacctgc 660
   aacgtagatc acaagcccag caacaccaag gtggacaaga gagttgagtc caaatatggt 720
   cccccatgcc catcatgccc agcacctgag ttcctggggg gaccatcagt cttcctgttc 780
   cccccaaaac ccaaggacac tctcatgatc tcccggaccc ctgaggtcac gtgcgtggtg 840
   gtggacgtga gccaggaaga ccccgaggtc cagttcaact ggtacgtgga tggcgtggag 900
   gtgcataatg ccaagacaaa gccgcgggag gagcagttca acagcacgta ccgtgtggtc 960
   agcgtcctca ccgtcctgca ccaggactgg ctgaacggca aggagtacaa gtgcaaggtc 1020
   tccaacaaag gcctcccgtc ctccatcgag aaaaccatct ccaaagccaa agggcagccc 1080
   cgagagccac aggtgtacac cctgccccca tcccaggagg agatgaccaa gaaccaggtc 1140
   agcctgacct gcctggtcaa aggcttctac cccagcgaca tcgccgtgga gtgggagagc 1200
   aatgggcagc cggagaacaa ctacaagacc acgcctcccg tgctggactc cgacggctcc 1260
   ttcttcctct acagcaggct aaccgtggac aagagcaggt ggcaggaggg gaatgtcttc 1320
   tcatgctccg tgatgcatga ggctctgcac aaccactaca cacagaagag cctctccctg 1380
   tctctgggta aatga 1395
<210> 119
   <211> 464
   <212> PRT
   <213> Homo sapiens
<400> 119
   Met Gly Trp Ser Cys Ile Ile Leu Phe Leu Val Ala Thr Ala Thr Gly
   1 5 10 15
   Val His Ser Asp Val Gln Leu Gln Glu Ser Gly Pro Gly Leu Val Lys
   20 25 30
   Pro Ser Gln Thr Leu Ser Leu Thr Cys Thr Val Thr Asp Tyr Ser Ile
   35 40 45
   Thr Ser Asp Tyr Ala Trp Asn Trp Ile Arg Gln Phe Pro Gly Lys Lys
   50 55 60
   Leu Glu Trp Met Gly Tyr Ile Ser Tyr Ser Gly Ser Thr Ser Tyr Asn
   65 70 75 80
   Pro Ser Leu Lys Ser Arg Ile Thr Ile Ser Arg Asp Thr Ser Lys Asn
   85 90 95
   Gln Phe Ser Leu Gln Leu Asn Ser Val Thr Ala Ala Asp Thr Ala Thr
   100 105 110
   Tyr Tyr Cys Ala Ser Phe Asp Tyr Ala His Ala Met Asp Tyr Trp Gly
   115 120 125
   Gln Gly Thr Thr Val Thr Val Ser Ser Ala Ser Thr Lys Gly Pro Ser
   130 135 140
   Val Phe Pro Leu Ala Pro Cys Ser Arg Ser Thr Ser Glu Ser Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Lys Thr Tyr Thr Cys Asn Val Asp His
   210 215 220
   Lys Pro Ser Asn Thr Lys Val Asp Lys Arg Val Glu Ser Lys Tyr Gly
   225 230 235 240
   Pro Pro Cys Pro Ser Cys Pro Ala Pro Glu Phe Leu Gly Gly Pro Ser
   245 250 255
   Val Phe Leu Phe Pro Pro Lys Pro Lys Asp Thr Leu Met Ile Ser Arg
   260 265 270
   Thr Pro Glu Val Thr Cys Val Val Val Asp Val Ser Gln Glu Asp Pro
   275 280 285
   Glu Val Gln Phe Asn Trp Tyr Val Asp Gly Val Glu Val His Asn Ala
   290 295 300
   Lys Thr Lys Pro Arg Glu Glu Gln Phe Asn Ser Thr Tyr Arg Val Val
   305 310 315 320
   Ser Val Leu Thr Val Leu His Gln Asp Trp Leu Asn Gly Lys Glu Tyr
   325 330 335
   Lys Cys Lys Val Ser Asn Lys Gly Leu Pro Ser Ser Ile Glu Lys Thr
   340 345 350
   Ile Ser Lys Ala Lys Gly Gln Pro Arg Glu Pro Gln Val Tyr Thr Leu
   355 360 365
   Pro Pro Ser Gln Glu Glu Met Thr Lys Asn Gln Val Ser Leu Thr Cys
   370 375 380
   Leu Val Lys Gly Phe Tyr Pro Ser Asp Ile Ala Val Glu Trp Glu Ser
   385 390 395 400
   Asn Gly Gln Pro Glu Asn Asn Tyr Lys Thr Thr Pro Pro Val Leu Asp
   405 410 415
   Ser Asp Gly Ser Phe Phe Leu Tyr Ser Arg Leu Thr Val Asp Lys Ser
   420 425 430
   Arg Trp Gln Glu Gly Asn Val Phe Ser Cys Ser Val Met His Glu Ala
   435 440 445
   Leu His Asn His Tyr Thr Gln Lys Ser Leu Ser Leu Ser Leu Gly Lys
   450 455 460
<210> 120
   <211> 8
   <212> PRT
   <213> Homo sapiens
<400> 120
   Arg Phe Arg Asp Asn Thr Pro Asn
   1 5
<210> 121
   <211> 8
   <212> PRT
   <213> Homo sapiens
<400> 121
   Arg Phe Arg Asp Asn Thr Ala Asn
   1 5
<210> 122
   <211> 9
   <212> PRT
   <213> Homo sapiens
<400> 122
   Ile Thr Phe Glu Phe Val Asp Gln Glu
   1 5
<210> 123
   <211> 7
   <212> PRT
   <213> Homo sapiens
<400> 123
   Phe Tyr Glu Thr Pro Leu Gln
   1 5
<210> 124
   <211> 118
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(7)
   <223> Xaa= any amino acid
<220>
   <221> misc_feature
   <222> (9)..(17)
   <223> Xaa= any amino acid
<220>
   <221> misc_feature
   <222> (19)..(19)
   <223> Xaa= any amino acid
<220>
   <221> misc_feature
   <222> (21).. (21)
   <223> Xaa= any amino acid
<220>
   <221> misc_feature
   <222> (23)..(23)
   <223> Xaa= any amino acid
<220>
   <221> misc_feature
   <222> (25)..(25)
   <223> Xaa= any amino acid
<220>
   <221> misc_feature
   <222> (39)..(39)
   <223> Xaa= any amino acid
<220>
   <221> misc_feature
   <222> (41)..(45)
   <223> Xaa= any amino acid
<220>
   <221> misc_feature
   <222> (47)..(47)
   <223> Xaa= any amino acid
<220>
   <221> misc_feature
   <222> (60)..(60)
   <223> Xaa= any amino acid
<220>
   <221> misc_feature
   <222> (62)..(63)
   <223> Xaa= any amino acid
<220>
   <221> misc_feature
   <222> (65)..(67)
   <223> Xaa= any amino acid
<220>
   <221> misc_feature
   <222> (69)..(69)
   <223> Xaa= any amino acid
<220>
   <221> misc_feature
   <222> (71)..(71)
   <223> Xaa= any amino acid
<220>
   <221> misc_feature
   <222> (73)..(78)
   <223> Xaa= any amino acid
<220>
   <221> misc_feature
   <222> (80)..(80)
   <223> Xaa= any amino acid
<220>
   <221> misc_feature
   <222> (82)..(82)
   <223> Xaa= any amino acid
<220>
   <221> misc_feature
   <222> (84)..(85)
   <223> Xaa= any amino acid
<220>
   <221> misc_feature
   <222> (87)..(89)
   <223> Xaa= any amino acid
<220>
   <221> misc_feature
   <222> (91)..(91)
   <223> Xaa= any amino acid
<220>
   <221> misc_feature
   <222> (93)..(93)
   <223> Xaa= any amino acid
<220>
   <221> misc_feature
   <222> (110)..(110)
   <223> Xaa= any amino acid
<220>
   <221> misc_feature
   <222> (113)..(113)
   <223> Xaa= any amino acid
<220>
   <221> misc_feature
   <222> (115)..(115)
   <223> Xaa= any amino acid
<220>
   <221> misc_feature
   <222> (117)..(118)
   <223> Xaa= any amino acid
<400> 124
   Xaa Val Xaa Leu Xaa Glu Xaa Gly Xaa Xaa Xaa Xaa Xaa Xaa Xaa Xaa
   1               5                   10                  15
   Xaa Leu Xaa Leu Xaa Cys Xaa Val Xaa Asp Tyr Ser Ile Thr Ser Asp
               20                  25                  30
   Tyr Ala Trp Asn Trp Ile Xaa Gln Xaa Xaa Xaa Xaa Xaa Leu Xaa Trp
           35                  40                  45
   Met Gly Tyr Ile Ser Tyr Ser Gly Ser Thr Ser Xaa Asn Xaa Xaa Leu
       50                  55                  60
   Xaa Xaa Xaa Ile Xaa Ile Xaa Arg Xaa Xaa Xaa Xaa Xaa Xaa Phe Xaa
   65                  70                  75                  80
   Leu Xaa Leu Xaa Xaa Val Xaa Xaa Xaa Asp Xaa Ala Xaa Tyr Tyr Cys
                   85                  90                  95
   Ala Ser Phe Asp Tyr Ala His Ala Met Asp Tyr Trp Gly Xaa Gly Thr
               100                 105                 110
   Xaa Val Xaa Val Xaa Xaa
           115
<210> 125
   <211> 118
   <212> PRT
   <213> Homo sapiens
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(7)
   <223> Xaa= any amino acid
<220>
   <221> misc_feature
   <222> (10)..(11)
   <223> Xaa= any amino acid
<220>
   <221> misc_feature
   <222> (13)..(13)
   <223> Xaa= any amino acid
<220>
   <221> misc_feature
   <222> (15)..(17)
   <223> Xaa= any amino acid
<220>
   <221> misc_feature
   <222> (19)..(19)
   <223> Xaa= any amino acid
<220>
   <221> misc_feature
   <222> (21)..(21)
   <223> Xaa= any amino acid
<220>
   <221> misc_feature
   <222> (23)..(23)
   <223> Xaa= any amino acid
<220>
   <221> misc_feature
   <222> (41)..(41)
   <223> Xaa= any amino acid
<220>
   <221> misc_feature
   <222> (43)..(44)
   <223> Xaa= any amino acid
<220>
   <221> misc_feature
   <222> (66)..(66)
   <223> Xaa= any amino acid
<220>
   <221> misc_feature
   <222> (69)..(69)
   <223> Xaa= any amino acid
<220>
   <221> misc_feature
   <222> (71)..(71)
   <223> Xaa= any amino acid
<220>
   <221> misc_feature
   <222> (73)..(73)
   <223> Xaa= any amino acid
<220>
   <221> misc_feature
   <222> (75)..(76)
   <223> Xaa= any amino acid
<220>
   <221> misc_feature
   <222> (78)..(78)
   <223> Xaa= any amino acid
<220>
   <221> misc_feature
   <222> (80)..(80)
   <223> Xaa= any amino acid
<220>
   <221> misc_feature
   <222> (82)..(82)
   <223> Xaa= any amino acid
<220>
   <221> misc_feature
   <222> (84)..(85)
   <223> Xaa= any amino acid
<220>
   <221> misc_feature
   <222> (87)..(89)
   <223> Xaa= any amino acid
<220>
   <221> misc_feature
   <222> (91)..(91)
   <223> Xaa= any amino acid
<220>
   <221> misc_feature
   <222> (110)..(110)
   <223> Xaa= any amino acid
<220>
   <221> misc_feature
   <222> (113)..(113)
   <223> Xaa= any amino acid
<220>
   <221> misc_feature
   <222> (115)..(115)
   <223> Xaa= any amino acid
<220>
   <221> misc_feature
   <222> (117)..(118)
   <223> Xaa= any amino acid
<400> 125
   Asp Val Xaa Leu Xaa Glu Xaa Gly Pro Xaa Xaa Val Xaa Pro Xaa Xaa
   1               5                   10                  15
   Xaa Leu Xaa Leu Xaa Cys Xaa Val Thr Asp Tyr Ser Ile Thr Ser Asp
               20                  25                  30
   Tyr Ala Trp Asn Trp Ile Arg Gln Xaa Pro Xaa Xaa Lys Leu Glu Trp
           35                  40                  45
   Met Gly Tyr Ile Ser Tyr Ser Gly Ser Thr Ser Tyr Asn Pro Ser Leu
       50                  55                  60
   Lys Xaa Arg Ile Xaa Ile Xaa Arg Xaa Thr Xaa Xaa Asn Xaa Phe Xaa
   65                  70                  75                  80
   Leu Xaa Leu Xaa Xaa Val Xaa Xaa Xaa Asp Xaa Ala Thr Tyr Tyr Cys
                   85                  90                  95
   Ala Ser Phe Asp Tyr Ala His Ala Met Asp Tyr Trp Gly Xaa Gly Thr
               100                 105                 110
   Xaa Val Xaa Val Xaa Xaa
           115
<210> 126
   <211> 118
   <212> PRT
   <213> Homo sapiens
<220>
   <221> misc_feature
   <222> (1)..(1)
   <223> Xaa= any amino acid
<220>
   <221> misc_feature
   <222> (17)..(17)
   <223> Xaa= any amino acid
<220>
   <221> misc_feature
   <222> (25)..(25)
   <223> Xaa= any amino acid
<220>
   <221> misc_feature
   <222> (44)..(45)
   <223> Xaa= any amino acid
<220>
   <221> misc_feature
   <222> (69)..(69)
   <223> Xaa= any amino acid
<220>
   <221> misc_feature
   <222> (71)..(71)
   <223> Xaa= any amino acid
<220>
   <221> misc_feature
   <222> (80)..(80)
   <223> Xaa= any amino acid
<220>
   <221> misc_feature
   <222> (88)..(89)
   <223> Xaa= any amino acid
<220>
   <221> misc_feature
   <222> (93)..(93)
   <223> Xaa= any amino acid
<220>
   <221> misc_feature
   <222> (113)..(113)
   <223> Xaa= any amino acid
<400> 126
   Xaa Val Gln Leu Gln Glu Ser Gly Pro Gly Leu Val Lys Pro Ser Gln
   1               5                   10                  15
   Xaa Leu Ser Leu Thr Cys Thr Val Xaa Asp Tyr Ser Ile Thr Ser Asp
               20                  25                  30
   Tyr Ala Trp Asn Trp Ile Arg Gln Phe Pro Gly Xaa Xaa Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Xaa Ile Xaa Arg Asp Thr Ser Lys Asn Gln Phe Xaa
   65                  70                  75                  80
   Leu Gln Leu Asn Ser Val Thr Xaa Xaa Asp Thr Ala Xaa Tyr Tyr Cys
                   85                  90                  95
   Ala Ser Phe Asp Tyr Ala His Ala Met Asp Tyr Trp Gly Gln Gly Thr
               100                 105                 110
   Xaa Val Thr Val Ser Ser
           115
<210> 127
   <211> 118
   <212> PRT
   <213> Homo sapiens
<220>
   <221> misc_feature
   <222> (17)..(17)
   <223> Xaa= any amino acid
<220>
   <221> misc_feature
   <222> (44)..(44)
   <223> Xaa= any amino acid
<220>
   <221> misc_feature
   <222> (69)..(69)
   <223> Xaa= any amino acid
<220>
   <221> misc_feature
   <222> (71)..(71)
   <223> Xaa= any amino acid
<220>
   <221> misc_feature
   <222> (80)..(80)
   <223> Xaa= any amino acid
<220>
   <221> misc_feature
   <222> (88)..(89)
   <223> Xaa= any amino acid
<220>
   <221> misc_feature
   <222> (113)..(113)
   <223> Xaa= any amino acid
<400> 127
   Asp Val Gln Leu Gln Glu Ser Gly Pro Gly Leu Val Lys Pro Ser Gln
   1               5                   10                  15
   Xaa Leu Ser Leu Thr Cys Thr Val Thr Asp Tyr Ser Ile Thr Ser Asp
               20                  25                  30
   Tyr Ala Trp Asn Trp Ile Arg Gln Phe Pro Gly Xaa Lys Leu Glu Trp
           35                  40                  45
   Met Gly Tyr Ile Ser Tyr Ser Gly Ser Thr Ser Tyr Asn Pro Ser Leu
       50                  55                  60
   Lys Ser Arg Ile Xaa Ile Xaa Arg Asp Thr Ser Lys Asn Gln Phe Xaa
   65                  70                  75                  80
   Leu Gln Leu Asn Ser Val Thr Xaa Xaa Asp Thr Ala Thr Tyr Tyr Cys
                   85                  90                  95
   Ala Ser Phe Asp Tyr Ala His Ala Met Asp Tyr Trp Gly Gln Gly Thr
               100                 105                 110
   Xaa Val Thr Val Ser Ser
           115
<210> 128
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(12)
   <223> Xaa= any amino acid
<220>
   <221> misc_feature
   <222> (14)..(18)
   <223> Xaa= any amino acid
<220>
   <221> misc_feature
   <222> (20)..(20)
   <223> Xaa= any amino acid
<220>
   <221> misc_feature
   <222> (22)..(22)
   <223> Xaa= any amino acid
<220>
   <221> misc_feature
   <222> (24)..(24)
   <223> Xaa= any amino acid
<220>
   <221> misc_feature
   <222> (26)..(26)
   <223> Xaa= any amino acid
<220>
   <221> misc_feature
   <222> (37)..(37)
   <223> Xaa= any amino acid
<220>
   <221> misc_feature
   <222> (39)..(43)
   <223> Xaa= any amino acid
<220>
   <221> misc_feature
   <222> (45)..(45)
   <223> Xaa= any amino acid
<220>
   <221> misc_feature
   <222> (54)..(57)
   <223> Xaa= any amino acid
<220>
   <221> misc_feature
   <222> (59)..(61)
   <223> Xaa= any amino acid
<220>
   <221> misc_feature
   <222> (63)..(63)
   <223> Xaa= any amino acid
<220>
   <221> misc_feature
   <222> (65)..(65)
   <223> Xaa= any amino acid
<220>
   <221> misc_feature
   <222> (67)..(67)
   <223> Xaa= any amino acid
<220>
   <221> misc_feature
   <222> (69)..(70)
   <223> Xaa= any amino acid
<220>
   <221> misc_feature
   <222> (72)..(72)
   <223> Xaa= any amino acid
<220>
   <221> misc_feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (79)..(81)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (103)..(103)
   <223> Xaa= any amino acid
<220>
   <221> misc_feature
   <222> (105)..(109)
   <223> Xaa= any amino acid
<400> 128
   Xaa Ile Xaa Leu Xaa Gln Xaa Xaa Xaa Xaa Xaa Xaa Val Xaa Xaa Xaa
   1               5                   10                  15
   Xaa Xaa Val Xaa Phe Xaa Cys Xaa Ala Xaa Gln Ser Ile Gly Thr Ser
               20                  25                  30
   Ile His Trp Tyr Xaa Gln Xaa Xaa Xaa Xaa Xaa Pro Xaa Leu Leu Ile
           35                  40                  45
   Lys Tyr Ala Ser Glu Xaa Xaa Xaa Xaa Ile Xaa Xaa Xaa Phe Xaa Gly
       50                  55                  60
   Xaa Gly Xaa Gly Xaa Xaa Phe Xaa Leu Xaa Ile Xaa Xaa Val Xaa Xaa
   65                  70                  75                  80
   Xaa Asp Xaa Ala Asp Tyr Tyr Cys Gln Gln Ile Asn Ser Trp Pro Thr
                   85                  90                  95
   Thr Phe Gly Xaa Gly Thr Xaa Leu Xaa Xaa Xaa Xaa Xaa
               100                 105
<210> 129
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(7)
   <223> Xaa= any amino acid
<220>
   <221> misc_feature
   <222> (9)..(10)
   <223> Xaa= any amino acid
<220>
   <221> misc_feature
   <222> (12)..(12)
   <223> Xaa= any amino acid
<220>
   <221> misc_feature
   <222> (14)..(14)
   <223> Xaa= any amino acid
<220>
   <221> misc_feature
   <222> (16)..(18)
   <223> Xaa= any amino acid
<220>
   <221> misc_feature
   <222> (20)..(20)
   <223> Xaa= any amino acid
<220>
   <221> misc_feature
   <222> (22)..(22)
   <223> Xaa= any amino acid
<220>
   <221> misc_feature
   <222> (24)..(24)
   <223> Xaa= any amino acid
<220>
   <221> misc_feature
   <222> (39)..(39)
   <223> Xaa= any amino acid
<220>
   <221> misc_feature
   <222> (41)..(42)
   <223> Xaa= any amino acid
<220>
   <221> misc_feature
   <222> (54)..(54)
   <223> Xaa= any amino acid
<220>
   <221> misc_feature
   <222> (57)..(57)
   <223> Xaa= any amino acid
<220>
   <221> misc_feature
   <222> (60)..(60)
   <223> Xaa= any amino acid
<220>
   <221> misc_feature
   <222> (63)..(63)
   <223> Xaa= any amino acid
<220>
   <221> misc_feature
   <222> (65)..(65)
   <223> Xaa= any amino acid
<220>
   <221> misc_feature
   <222> (67)..(67)
   <223> Xaa= any amino acid
<220>
   <221> misc_feature
   <222> (69)..(70)
   <223> Xaa= any amino acid
<220>
   <221> misc_feature
   <222> (72)..(72)
   <223> Xaa= any amino acid
<220>
   <221> misc_feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (79)..(81)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (103)..(103)
   <223> Xaa= any amino acid
<220>
   <221> misc_feature
   <222> (105)..(109)
   <223> Xaa= any amino acid
<400> 129
   Xaa Ile Xaa Leu Xaa Gln Xaa Pro Xaa Xaa Leu Xaa Val Xaa Pro Xaa
   1               5                   10                  15
   Xaa Xaa Val Xaa Phe Xaa Cys Xaa Ala Ser Gln Ser Ile Gly Thr Ser
               20                  25                  30
   Ile His Trp Tyr Gln Gln Xaa Thr Xaa Xaa Ser Pro Arg Leu Leu Ile
           35                  40                  45
   Lys Tyr Ala Ser Glu Xaa Ile Ser Xaa Ile Pro Xaa Arg Phe Xaa Gly
       50                  55                  60
   Xaa Gly Xaa Gly Xaa Xaa Phe Xaa Leu Xaa Ile Xaa Xaa Val Xaa Xaa
   65                  70                  75                  80
   Xaa Asp Xaa Ala Asp Tyr Tyr Cys Gln Gln Ile Asn Ser Trp Pro Thr
                   85                  90                  95
   Thr Phe Gly Xaa Gly Thr Xaa Leu Xaa Xaa Xaa Xaa Xaa
               100                 105
<210> 130
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (9)..(10)
   <223> Xaa= any amino acid
<220>
   <221> misc_feature
   <222> (20)..(20)
   <223> Xaa= any amino acid
<220>
   <221> misc_feature
   <222> (39)..(39)
   <223> Xaa= any amino acid
<220>
   <221> misc_feature
   <222> (41)..(43)
   <223> Xaa= any amino acid
<220>
   <221> misc_feature
   <222> (54)..(56)
   <223> Xaa= any amino acid
<220>
   <221> misc_feature
   <222> (60)..(60)
   <223> Xaa= any amino acid
<220>
   <221> misc**_**feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (109)..(109)
   <223> Xaa= any amino acid
<400> 130
   Xaa Ile Xaa Leu Thr Gln Ser Pro Xaa Xaa Leu Ser Val Ser Pro Gly
   1               5                   10                  15
   Glu Arg Val Xaa Phe Ser Cys Arg Ala Ser Gln Ser Ile Gly Thr Ser
               20                  25                  30
   Ile His Trp Tyr Gln Gln Xaa Thr Xaa Xaa Xaa Pro Arg Leu Leu Ile
           35                  40                  45
   Lys Tyr Ala Ser Glu Xaa Xaa Xaa Gly Ile Pro Xaa Arg Phe Ser Gly
       50                  55                  60
   Ser Gly Ser Gly Thr Asp Phe Thr Leu Xaa Ile Xaa Xaa Val Glu Ser
   65                  70                  75                  80
   Glu Asp Xaa Ala Asp Tyr Tyr Cys Gln Gln Ile Asn Ser Trp Pro Thr
                   85                  90                  95
   Thr Phe Gly Xaa Gly Thr Lys Leu Glu Ile Lys Arg Xaa
               100                 105
<210> 131
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (9)..(10)
   <223> Xaa= any amino acid
<220>
   <221> misc_feature
   <222> (20)..(20)
   <223> Xaa= any amino acid
<220>
   <221> misc_feature
   <222> (39)..(39)
   <223> Xaa= any amino acid
<220>
   <221> misc_feature
   <222> (41)..(42)
   <223> Xaa= any amino acid
<220>
   <221> misc_feature
   <222> (54)..(54)
   <223> Xaa= any amino acid
<220>
   <221> misc_feature
   <222> (60)..(60)
   <223> Xaa= any amino acid
<220>
   <221> misc_feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (109)..(109)
   <223> Xaa= any amino acid
<400> 131
   Xaa Ile Xaa Leu Thr Gln Ser Pro Xaa Xaa Leu Ser Val Ser Pro Gly
   1 5 10 15
   Glu Arg Val Xaa Phe Ser Cys Arg Ala Ser Gln Ser Ile Gly Thr Ser
   20 25 30
   Ile His Trp Tyr Gln Gln Xaa Thr Xaa Xaa Ser Pro Arg Leu Leu Ile
   35 40 45
   Lys Tyr Ala Ser Glu Xaa Ile Ser Gly Ile Pro Xaa Arg Phe Ser Gly
   50 55 60
   Ser Gly Ser Gly Thr Asp Phe Thr Leu Xaa Ile Xaa Xaa Val Glu Ser
   65 70 75 80
   Glu Asp Xaa Ala Asp Tyr Tyr Cys Gln Gln Ile Asn Ser Trp Pro Thr
   85 90 95
   Thr Phe Gly Xaa Gly Thr Lys Leu Glu Ile Lys Arg Xaa
   100 105
<210> 132
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (9)..(10)
   <223> Xaa= any amino acid
<220>
   <221> misc_feature
   <222> (20)..(20)
   <223> Xaa= any amino acid
<220>
   <221> misc_feature
   <222> (39)..(39)
   <223> Xaa= any amino acid
<220>
   <221> misc_feature
   <222> (41)..(43)
   <223> Xaa= any amino acid
<220>
   <221> misc_feature
   <222> (60)..(60)
   <223> Xaa= any amino acid
<220>
   <221> misc_feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (109)..(109)
   <223> Xaa= any amino acid
<400> 132
   Xaa Ile Xaa Leu Thr Gln Ser Pro Xaa Xaa Leu Ser Val Ser Pro Gly
   1               5                   10                  15
   Glu Arg Val Xaa Phe Ser Cys Arg Ala Ser Gln Ser Ile Gly Thr Ser
               20                  25                  30
   Ile His Trp Tyr Gln Gln Xaa Thr Xaa Xaa Xaa Pro Arg Leu Leu Ile
           35                  40                  45
   Lys Tyr Ala Ser Glu Ser Ile Ser Gly Ile Pro Xaa Arg Phe Ser Gly
       50                  55                  60
   Ser Gly Ser Gly Thr Asp Phe Thr Leu Xaa Ile Xaa Xaa Val Glu Ser
   65                  70                  75                  80
   Glu Asp Xaa Ala Asp Tyr Tyr Cys Gln Gln Ile Asn Ser Trp Pro Thr
   85 90 95
   Thr Phe Gly Xaa Gly Thr Lys Leu Glu Ile Lys Arg Xaa
   100 105
<210> 133
   <211> 118
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(7)
   <223> Xaa= any amino acid
<220>
   <221> misc_feature
   <222> (9)..(17)
   <223> Xaa= any amino acid
<220>
   <221> misc_feature
   <222> (19)..(19)
   <223> Xaa= any amino acid
<220>
   <221> misc_feature
   <222> (21)..(21)
   <223> Xaa= any amino acid
<220>
   <221> misc_feature
   <222> (23)..(23)
   <223> Xaa= any amino acid
<220>
   <221> misc_feature
   <222> (25)..(25)
   <223> Xaa= any amino acid
<220>
   <221> misc_feature
   <222> (39)..(39)
   <223> Xaa= any amino acid
<220>
   <221> misc_feature
   <222> (41)..(45)
   <223> Xaa= any amino acid
<220>
   <221> misc_feature
   <222> (47)..(47)
   <223> Xaa= any amino acid
<220>
   <221> misc_feature
   <222> (60)..(60)
   <223> Xaa= any amino acid
<220>
   <221> misc_feature
   <222> (62)..(63)
   <223> Xaa= any amino acid
<220>
   <221> misc_feature
   <222> (65)..(67)
   <223> Xaa= any amino acid
<220>
   <221> misc_feature
   <222> (69)..(69)
   <223> Xaa= any amino acid
<220>
   <221> misc_feature
   <222> (71)..(71)
   <223> Xaa= any amino acid
<220>
   <221> misc_feature
   <222> (73)..(78)
   <223> Xaa= any amino acid
<220>
   <221> misc_feature
   <222> (80)..(80)
   <223> Xaa= any amino acid
<220>
   <221> misc_feature
   <222> (82)..(82)
   <223> Xaa= any amino acid
<220>
   <221> misc_feature
   <222> (84)..(85)
   <223> Xaa= any amino acid
<220>
   <221> misc_feature
   <222> (87)..(89)
   <223> Xaa= any amino acid
<220>
   <221> misc_feature
   <222> (91)..(91)
   <223> Xaa= any amino acid
<220>
   <221> misc_feature
   <222> (93)..(93)
   <223> Xaa= any amino acid
<220>
   <221> misc_feature
   <222> (110)..(110)
   <223> Xaa= any amino acid
<220>
   <221> misc_feature
   <222> (113)..(113)
   <223> Xaa= any amino acid
<220>
   <221> misc_feature
   <222> (115)..(115)
   <223> Xaa= any amino acid
<220>
   <221> misc_feature
   <222> (117)..(118)
   <223> Xaa= any amino acid
<400> 133
   Xaa Val Xaa Leu Xaa Glu Xaa Gly Xaa Xaa Xaa Xaa Xaa Xaa Xaa Xaa
   1               5                   10                  15
   Xaa Leu Xaa Leu Xaa Cys Xaa Val Xaa Asp Tyr Ser Ile Thr Ser Asp
               20                  25                  30
   Tyr Ala Trp Asn Trp Ile Xaa Gln Xaa Xaa Xaa Xaa Xaa Leu Xaa Trp
           35                  40                  45
   Met Gly Tyr Ile Ser Tyr Ser Gly Ser Thr Ser Xaa Asn Xaa Xaa Leu
       50                  55                  60
   Xaa Xaa Xaa Ile Xaa Ile Xaa Arg Xaa Xaa Xaa Xaa Xaa Xaa Phe Xaa
   65                  70                  75                  80
   Leu Xaa Leu Xaa Xaa Val Xaa Xaa Xaa Asp Xaa Ala Xaa Tyr Tyr Cys
                   85                  90                  95
   Ala Ser Phe Asp Tyr Ala His Ala Met Asp Tyr Trp Gly Xaa Gly Thr
               100                 105                 110
   Xaa Val Xaa Val Xaa Xaa
           115
<210> 134
   <211> 109
   <212> PRT
   <213> Homo sapiens
<220>
   <221> misc_feature
   <222> (1)..(1)
   <223> Xaa= any amino acid
<220>
   <221> misc_feature
   <222> (3)..(3)
   <223> Xaa= any amino acid
<220>
   <221> misc_feature
   <222> (5)..(5)
   <223> Xaa= any amino acid
<220>
   <221> misc_feature
   <222> (7)..(12)
   <223> Xaa= any amino acid
<220>
   <221> misc_feature
   <222> (14)..(18)
   <223> Xaa= any amino acid
<220>
   <221> misc_feature
   <222> (20)..(20)
   <223> Xaa= any amino acid
<220>
   <221> misc_feature
   <222> (22)..(22)
   <223> Xaa= any amino acid
<220>
   <221> misc_feature
   <222> (24)..(24)
   <223> Xaa= any amino acid
<220>
   <221> misc_feature
   <222> (26)..(26)
   <223> Xaa= any amino acid
<220>
   <221> misc_feature
   <222> (37)..(37)
   <223> Xaa= any amino acid
<220>
   <221> misc_feature
   <222> (39)..(43)
   <223> Xaa= any amino acid
<220>
   <221> misc_feature
   <222> (45)..(45)
   <223> Xaa= any amino acid
<220>
   <221> misc_feature
   <222> (54)..(57)
   <223> Xaa= any amino acid
<220>
   <221> misc_feature
   <222> (59)..(61)
   <223> Xaa= any amino acid
<220>
   <221> misc_feature
   <222> (63)..(63)
   <223> Xaa= any amino acid
<220>
   <221> misc_feature
   <222> (65)..(65)
   <223> Xaa= any amino acid
<220>
   <221> misc_feature
   <222> (67)..(67)
   <223> Xaa= any amino acid
<220>
   <221> misc_feature
   <222> (69)..(70)
   <223> Xaa= any amino acid
<220>
   <221> misc_feature
   <222> (72)..(72)
   <223> Xaa= any amino acid
<220>
   <221> misc_feature
   <222> (74)..(74)
   <223> Xaa= any amino acid
<220>
   <221> misc_feature
   <222> (76)..(77)
   <223> Xaa= any amino acid
<220>
   <221> misc_feature
   <222> (79)..(81)
   <223> Xaa= any amino acid
<220>
   <221> misc_feature
   <222> (83)..(83)
   <223> Xaa= any amino acid
<220>
   <221> misc_feature
   <222> (100)..(100)
   <223> Xaa= any amino acid
<220>
   <221> misc_feature
   <222> (103)..(103)
   <223> Xaa= any amino acid
<220>
   <221> misc_feature
   <222> (105)..(109)
   <223> Xaa= any amino acid
<400> 134
   Xaa Ile Xaa Leu Xaa Gln Xaa Xaa Xaa Xaa Xaa Xaa Val Xaa Xaa Xaa
   1               5                   10                  15
   Xaa Xaa Val Xaa Phe Xaa Cys Xaa Ala Xaa Gln Ser Ile Gly Thr Ser
               20                  25                  30
   Ile His Trp Tyr Xaa Gln Xaa Xaa Xaa Xaa Xaa Pro Xaa Leu Leu Ile
           35                  40                  45
   Lys Tyr Ala Ser Glu Xaa Xaa Xaa Xaa Ile Xaa Xaa Xaa Phe Xaa Gly
       50                  55                  60
   Xaa Gly Xaa Gly Xaa Xaa Phe Xaa Leu Xaa Ile Xaa Xaa Val Xaa Xaa
   65                  70                  75                  80
   Xaa Asp Xaa Ala Asp Tyr Tyr Cys Gln Gln Ile Asn Ser Trp Pro Thr
                   85                  90                  95
   Thr Phe Gly Xaa Gly Thr Xaa Leu Xaa Xaa Xaa Xaa Xaa
               100                 105
<210> 135
   <211> 327
   <212> DNA
   <213> Homo sapiens
<400> 135
   gaaatagtcc ttacccaatc tcccggaacc ctctcagtat ctcccggcga acgagtaacc      60
   ttttcatgta gagcatccca atccatcggc acttcaattc actggtatca gcagaaaaca     120
   ggtcaatccc cacggcttct tataaaatat gcatcagaat caatttctgg catcccagac     180
   agattttcag gttcaggatc aggcaccgat ttcacactta caatatccag agtcgaatca     240
   gaagattttg cagattacta ttgtcaacaa ataaacagct ggcccactac attcggacaa     300
   ggcacaaaac tcgaaattaa acgtacg                                         327
<210> 136
   <211> 109
   <212> PRT
   <213> Homo sapiens
<400> 136
   Glu Ile Val Leu Thr Gln Ser Pro Gly Thr Leu Ser Val Ser Pro Gly
   1               5                   10                  15
   Glu Arg Val Thr Phe Ser Cys Arg Ala Ser Gln Ser Ile Gly Thr Ser
               20                  25                  30
   Ile His Trp Tyr Gln Gln Lys Thr Gly Gln Ser Pro Arg Leu Leu Ile
           35                  40                  45
   Lys Tyr Ala Ser Glu Ser Ile Ser Gly Ile Pro Asp Arg Phe Ser Gly
       50                  55                  60
   Ser Gly Ser Gly Thr Asp Phe Thr Leu Thr Ile Ser Arg Val Glu Ser
   65                  70                  75                  80
   Glu Asp Phe Ala Asp Tyr Tyr Cys Gln Gln Ile Asn Ser Trp Pro Thr
                   85                  90                  95
   Thr Phe Gly Gln Gly Thr Lys Leu Glu Ile Lys Arg Thr
               100                 105
<210> 137
   <211> 327
   <212> DNA
   <213> Homo sapiens
<400> 137
   gaaatagttc ttactcaatc ccccggtaca ctctcagttt ccccaggcga acgcgtcact      60
   ttttcttgca gagcatcaca atcaatcggc acttcaattc attggtatca acaaaaaaca     120
   ggacaggccc cacgacttct tattaaatat gcatcagaat caatttctgg catcccagac     180
   agattttcag gttcaggatc aggcaccgat ttcacactta caatatccag agtcgaatca     240
   gaagattttg cagattacta ttgtcaacaa ataaacagct ggcccactac attcggacaa     300
   ggcacaaaac tcgaaattaa acgtacg                                         327

## Description

### TECHNICAL FIELD

This invention relates to a humanized anti-M-CSF antibody for use in treating a human subject having an atherosclerotic disease , or for use in treating HIV infection in a human subject, or for use in treating a condition associated with HIV infection in a human subject having an HIV infection.

### BACKGROUND OF THE INVENTION

Colony stimulating factor (CSF-1), also known as macrophage colony stimulating factor (M-CSF), stimulates the production and proliferation of macrophages. Macrophages are well known mediators of the atherosclerotic process and contribute to the formation of occlusive plaques by migrating into early lesions and engulfing lipid. The resulting narrowing of blood vessels, including arteries that supply the heart, brain and limbs, causes angina and other symptoms of vascular occlusion. Macrophages also may contribute to the formation of unstable plaques by secreting proteases and other bioactive molecules that cause stable plaques to become unstable. Unstable plaques play a causative role in triggering blood clotting that may cause a total blockage of the blood vessel, resulting in a myocardial infarction or stroke. Finally, macrophages may also play a role in the over-exuberant repair process that leads to restenosis after angioplasty. Despite numerous available therapies, there is still a great medical need for more effective preventative and therapeutic strategies for atherosclerotic vascular disease and its associated symptoms and damaging consequences. Thus, there remains a need in the art to identify new agents and methods for preventing or treating such diseases.

Macrophage colony-stimulating factor (M-CSF) enhances the susceptibility of macrophages to infection with human immunodeficiency virus (HIV), in part by increasing the expression of CD4 and CCR5 (Kutza, J., et al., AIDS Res Hum Retroviruses, 18(9):619-25 (2002)). Human monocyte-derived macrophages (MDMs) infected in vitro with HIV-1 endogenously produce M-CSF, with kinetics paralleling virus replication, which can lead to enhanced spreading of the infection. Studies suggest that M-CSF may function in an autocrine/paracrine manner to sustain HIV replication, and that inhibitors of M-CSF activity dramatically reduce HIV-1 replication (Kutza, J., et al., J Immunol, 164(9):4955-60 (2000). These results suggest that biologic antagonists for M-CSF may represent novel strategies for inhibiting the spread of HIV-1 by blocking virus replication in macrophages and preventing the establishment and maintenance of infected macrophages as a reservoir for HIV.

Despite numerous available therapies, there is still a great medical need for more effective therapeutic strategies for HIV infection and its associated complications. Thus, there remains a need in the art to identify new agents and methods for preventing or treating such diseases.

### SUMMARY OF THE INVENTION

The materials and methods of the present invention fulfill the aforementioned and other related needs in the art. The invention provides a humanized anti-M-CSF antibody, for use in treating a human subject having an atherosclerotic disease, or for use in treating HIV infection in a human subject, or for use in treating a condition associated with HIV infection in a human subject having an HIV infection, wherein the heavy chain comprises the heavy chain variable region sequence set forth in SEQ ID NO: 43 and an IgG1 constant region; and the light chain comprises the light chain variable region sequence set forth in SEQ ID NO: 53.

The invention also provides a humanized anti-M-CSF antibody, for use in treating a human subject having an atherosclerotic disease, or for use in treating HIV infection in a human subject, or for use in treating a condition associated with HIV infection in a human subject having an HIV infection, wherein the heavy chain comprises the heavy chain variable region sequence set forth in SEQ ID NO: 43 and an IgG1 constant region; and the light chain comprises the light chain variable region sequence set forth in SEQ ID NO: 53, wherein the antibody is for use in coordination with treatment using a second therapeutic agent.

A non-murine antibody that competes with monoclonal antibody RX1 for binding to M-CSF by more than 75%, wherein the monoclonal antibody RX1 comprises the heavy chain and light chain amino acid sequences set forth in SEQ ID NOs: 2 and 4, respectively, is disclosed herein. The aforementioned non-murine antibody specifically binds to the same epitope of M-CSF as the monoclonal antibody RX1.

The non-murine antibody may bind an epitope of M-CSF that comprises at least 4 contiguous residues of SEQ ID NO: 120 or 121. The non-murine antibody may be a monoclonal antibody, a chimeric antibody, a humanized antibody, a human engineered antibody, a human antibody, a single chain antibody, or an IgG antibody.

A non-murine antibody useful in the treatment disclosed herein may retain an affinity K_{d} (dissociation equilibrium constant) with respect to M-CSF of SEQ ID NO: 9 of, for example, at least 10⁻⁷ M or higher, at least 10⁻⁸ M or higher, or at least 10⁻⁹ M or 10⁻¹⁰ M or higher. The non-murine antibody may comprise an amino acid sequence 90% identical to SEQ ID NO: 24, or comprises SEQ ID NO: 24. The non-murine antibody may comprise at least 1, at least 2, at least 3, at least 4, at least 5, or all of (a) SEQ ID NOs: 18, 21, 24, 29, 32, and 36; or (b) SEQ ID NOs: 18, 21, 24, 32, 36 and QASQSIGTSIH.

The non-murine antibody disclosed herein may further comprise one or more CDRs from another anti-M-CSF antibody, such as SEQ ID NO: 16, 19, 22, 27, 30, or 34 from 5H4; SEQ ID NO: 17, 20, 23, 28, 31, or 35 from MC1; SEQ ID NO: 18, 21, 25, 29, 32, or 37 from MC3; or a consensus CDR as set forth in SEQ ID NOs: 18, 21, 26, 29, 33, or 38The non-murine antibody may comprise a CDR in which at least one amino acid within a CDR is substituted by a corresponding residue of a corresponding CDR of another anti-M-CSF antibody.

The non-murine antibody disclosed herein may comprise a variable light chain amino acid sequence which is at least 65% homologous to the amino acid sequence set forth in SEQ ID NO: 4, and/or a variable heavy chain amino acid sequence which is at least 65% homologous to the amino acid sequence set forth in SEQ ID NO: 2.

The non-murine antibody may comprise a constant region of a human antibody sequence and one or more heavy and light chain variable framework regions of a human antibody sequence. Exemplary human antibody sequences include an individual human sequence, a human consensus sequence, an individual human germline sequence, or a human consensus germline sequence. Exemplary human antibody sequences are found in Kabat, NCBI Ig Blast, http://www.ncbi.nlm.nih.gov/igblast/showGermline.cgi, Kabat Database http://www.bioinf.org.uk/abs/seqtest.html, FTP site for Kabat Release 5.0 (1992) ftp://ftp.ncbi.nih.gov/repository/kabat/Re15.0/, ImMunoGeneTics database (Montpellier France) http://imgt.cnusc.fr:8104/, V-Base http://www.mrccpe.cam.ac.uk/LIST.php?menu=901, Zurich University http://www.unizh.ch/∼antibody/Sequences/index.html, The Therapeutic Antibody Human Homology Project (TAHHP) http://www.path.cam.ac.uk/∼mrc7/humanisation/TAHHP.html, Protein Sequence and Structure Analysis of Antibody Domains http://how.to/AnalyseAntibodies/, Humanization by design http://people.cryst.bbk.ac.uk/∼ubcg07s/, Antibody Resources http://www.antibodyresource.com/educational.html. Antibody Engineering (by TT Wu), Humana Press. Any of the preceding described antibodies may comprise a fragment of an IgG1 constant region, optionally including a mutation within the IgG1 constant region that reduces antibody-dependent cellular cytotoxicity or complement dependent cytotoxicity activity. Alternatively, any of the preceding described antibodies may comprise a fragment of an IgG4 constant region, optionally including a mutation in the IgG4 constant region that reduces formation of half-antibodies.

The disclosed non-murine antibody may comprise a heavy chain variable region that comprises the amino acid sequence XVXLXEXGXXXXXXXXXLXLXCXVXDYSITSDYAWNWIXQXXXXXLXWMGYISY SGSTSXNXXLXXXIXIXRXXXXXXFXLXLXXVXXXDXAXYYCASFDYAHAMDYW GXGTXVXVXX, wherein X is any amino acid. The disclosed non-murine antibody may comprise a heavy chain variable region that comprises the amino acid sequence DVXLXEXGPXXVXPXXXLXLXCXVTDYSITSDYAWNWIRQXPXXKLEWMGYISYS GSTSYNPSLKXRIXIXRXTXXNXFXLXLXXVXXXDXATYYCASFDYAHAMDYWGX GTXVXVXX, wherein X is any amino acid. The disclosed non-murine antibody may comprise a heavy chain variable region that comprises the amino acid sequence XVQLQESGPGLVKPSQXLSLTCTVXDYSITSDYAWNWIRQFPGXXLEWMGYISYSGS TSYNPSLKSRIXIXRDTSKNQFXLQLNSVTXXDTAXYYCASFDYAHAMDYWGQGTX VTVSS, wherein X is any amino acid. The disclosed non-murine antibody may comprise a heavy chain variable region that comprises the amino acid sequence DVQLQESGPGLVKPSQXLSLTCTVTDYSITSDYAWNWIRQFPGXKLEWMGYISYSGS TSYNPSLKSRIXIXRDTSKNQFXLQLNSVTXXDTATYYCASFDYAHAMDYWGQGTX VTVSS, wherein X is any amino acid. The disclosed non-murine antibody may comprise a heavy chain variable region that comprises the amino acid sequence DVQLQESGPGLVKPSQTLSLTCTVTDYSITSDYAWNWIRQFPGKKLEWMGYISYSGS TSYNPSLKSRITISRDTSKNQFSLQLNSVTAADTATYYCASFDYAHAMDYWGQGTTV TVSS. the disclosed non-murine antibody may comprise a heavy chain variable region that comprises the amino acid sequence QVQLQESGPGLVKPSQTLSLTCTVSDYSITSDYAWNWIRQFPGKGLEWMGYISYSGS TSYNPSLKSRITISRDTSKNQFSLQLNSVTAADTAVYYCASFDYAHAMDYWGQGTT VTVSS.

The disclosed non-murine antibody may comprise a light chain variable region that comprises the amino acid sequence XIXLXQXXXXXXVXXXXXVXFXCXAXQSIGTSIHWYXQXXXXXPXLLIKYASEXX XXIXXXFXGXGXGXXFXLXIXXVXXXDXADYYCQQINSWPTTFGXGTXLXXXXX, wherein X is any amino acid. The disclosed non-murine antibody may comprise a light chain variable region that comprises the amino acid sequence XIXLXQXPXXLXVXPXXXVXFXCXASQSIGTSIHWYQQXTXXSPRLLIKYASEXISXI PXRFXGXGXGXXFXLXIXXVXXXDXADYYCQQINSWPTTFGXGTXLXXXXX, wherein X is any amino acid. The disclosed non-murine antibody comprises a light chain variable region that comprises the amino acid sequence XIXLTQSPXXLSVSPGERVXFSCRASQSIGTSIHWYQQXTXXXPRLLIKYASEXXXGIP XRFSGSGSGTDFTLXIXXVESEDXADYYCQQINSWPTTFGXGTKLEIKRX, wherein X is any amino acid.

The disclosed non-murine antibody may comprise a light chain variable region that comprises the amino acid sequence XIXLTQSPXXLSVSPGERVXFSCRASQSIGTSIHWYQQXTXXSPRLLIKYASEXISGIPX RFSGSGSGTDFTLXIXXVESEDXADYYCQQINSWPTTFGXGTKLEIKRX, wherein X is any amino acid. The disclosed non-murine antibody may comprise a light chain variable region that comprises the amino acid sequence XIXLTQSPXXLSVSPGERVXFSCRASQSIGTSIHWYQQXTXXXPRLLIKYASESISGIPX RFSGSGSGTDFTLXIXXVESEDXADYYCQQINSWPTTFGXGTKLEIKRX, wherein X is any amino acid. The disclosed non-murine antibody may comprise a light chain variable region that comprises the amino acid sequence EIVLTQSPGTLSVSPGERVTFSCRASQSIGTSIHWYQQKTGQAPRLLIKYASESISGIPD RFSGSGSGTDFTLTISRVESEDFADYYCQQINSWPTTFGQGTKLEIKRT.

The disclosed non-murine antibody may comprise a light chain variable region that comprises the amino acid sequence EIVLTQSPGTLSVSPGERVTFSCRASQSIGTSIHWYQQKTGQAPRLLIKYASERATGIP DRFSGSGSGTDFTLTISRVESEDFADYYCQQINSWPTTFGQGTKLEIKRT. The disclosed non-murine antibody may comprise a light chain variable region that comprises the amino acid sequence EIVLTQSPGTLSVSPGERVTFSCRASQSIGTSIHWYQQKTGQSPRLLIKYASERISGIPD RFSGSGSGTDFTLTISRVESEDFADYYCQQINSWPTTFGQGTKLEIKRT.

At least one X of the aforementioned antibody is the same as an amino acid at the same corresponding position in SEQ ID NOs: 2 or 4 using Kabat numbering. In any of the preceding described antibodies, at least one X is a conservative substitution of an amino acid at the same corresponding position in SEQ ID NOs: 2 or 4 using Kabat numbering. Moreover, in any of the preceding described antibodies, at least one X is a non-conservative substitution of an amino acid at the same corresponding position in SEQ ID NOs: 2 or 4 using Kabat numbering. In examples of the preceding described antibodies, at least one X is an amino acid at the same corresponding position within a human antibody sequence, using Kabat numbering. The aforementioned human antibody sequence may be, for example, a human consensus sequence, human germline sequence, human consensus germline sequence, or any one of the human antibody sequences in Kabat.

The aforementioned Human Engineered™ antibody is derived from, based on, or contains part of the human antibody consensus sequence, human germline sequence, human consensus germline sequence, or any one of the human antibody sequences in Kabat, NCBI Ig Blast, http://www.ncbi.nlm.nih.gov/igblast/showGermline.cgi, Kabat Database http://www.bioinf.org.uk/abs/seqtest.html, FTP site for Kabat Release 5.0 (1992) ftp://ftp.ncbi.nih.gov/repository/kabat/Re15.0/, ImMunoGeneTics database (Montpellier France) http://imgt.cnusc.fr:8104/, V-Base http://www.mrc-cpe.cam.ac.uk/LIST.php?menu=901, Zurich University http://www.unizh.ch/∼antibody/Sequences/index.html, The Therapeutic Antibody Human Homology Project (TAHHP) http://www.path.cam.ac.uk/∼mrc7/humanisation/TAHHP.html, Protein Sequence and Structure Analysis of Antibody Domains http://how.to/AnalyseAntibodies/, Humanization by design http://people.cryst.bbk.ac.uk/∼ubcg07s/, Antibody Resources http://www.antibodyresource.com/educational.html, Antibody Engineering (by TT Wu), Humana Press.

The aforementioned non-murine antibody may comprise any one of the heavy chain sequences set forth in SEQ ID NOS: 114, 116, or 119. The disclosed non-murine antibody may comprise any one of the heavy chain variable region sequences set forth in SEQ ID NOS: 41 or 43. The disclosed non-murine antibody may comprise any one of the light chain sequences set forth in SEQ ID NOS: 45, 47, 48, 51, 53 or 136The disclosed non-murine antibody may comprise the heavy chain sequence set forth in SEQ ID NO: 114 and the light chain sequence set forth in SEQ ID NO: 47. The disclosed non-murine antibody may comprise the heavy chain sequence set forth in SEQ ID NO: 116 and the light chain sequence set forth in SEQ ID NO: 47. The disclosed non-murine antibody may comprise the heavy chain sequence set forth in SEQ ID NO: 119 and the light chain sequence set forth in SEQ ID NO: 47.

In any of the preceding described antibodies, the non-murine antibody may comprise a variable heavy chain amino acid sequence which is at least 65%, or at least 80%, identical to the variable heavy chain amino acid sequence set forth in SEQ ID NOs: 41 or 43, and/or a variable light chain amino acid sequence which is at least 65%, or at least 80%, identical to the variable light chain amino acid sequence set forth in SEQ ID NOs: 45, 47, 48, 51, or 53.

Any and all combinations of the preceding described exemplary light chain variable regions or heavy chain variable regions may be used.

The antibody may, for example, be administered at a dose between about 1 µg/kg to 100 mg/kg body weight, between about 2 µg/kg to 30 mg/kg body weight, between about 0.1 mg/kg to 30 mg/kg body weight, or between about 0.1 mg/kg to 10 mg/kg body weight. In other aspects, the aforementioned antibodies for use may further comprise administering a second therapeutic agent.

The invention also provides use of a humanized anti-M-CSF antibody, in the manufacture of a medicament for treating a human subject having an atherosclerotic disease, or treating HIV infection in a human subject, or treating a condition associated with HIV infection in a human subject having an HIV infection, wherein the heavy chain comprises the heavy chain variable region sequence set forth in SEQ ID NO: 43 and an IgG1 constant region; and the light chain comprises the light chain variable region sequence set forth in SEQ ID NO: 53, wherein the medicament further comprises a second therapeutic agent.

Also disclosed herein is a kit comprising a therapeutically effective amount of the aforementioned antibody, either in lyophilized or solution form, packaged in a container, such as a vial or bottle or prefilled syringe. The container further may comprise a label attached to or packaged with the container, the label describing the contents of the container and providing indications and/or instructions regarding use of the contents of the container to treat a macrophage-associated disease. The container optionally comprises another vial with suitable solution for reconstituting lyophilized antibody.

### BRIEF DESCRIPTION OF THE DRAWINGS

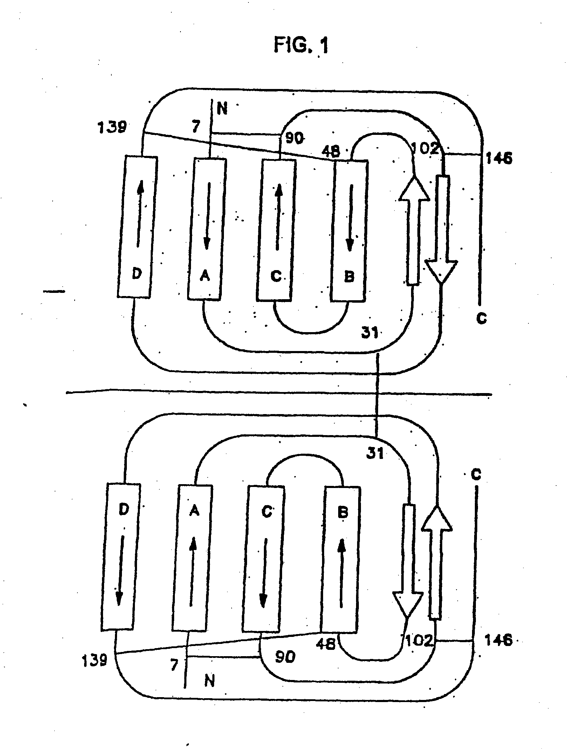

Figure 1 is a topology diagram showing the disulfide bonds in truncated dimeric M-CSF.

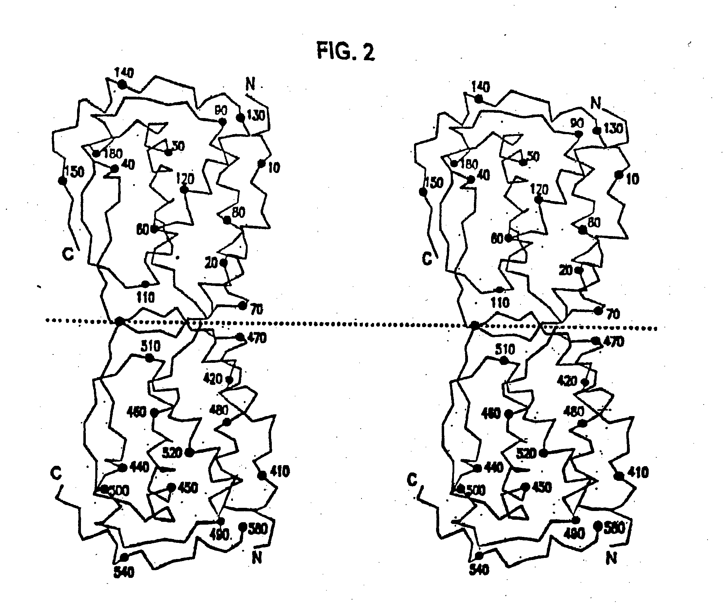

Figure 2 is a stereodiagram of the C-alpha backbone of M-CSF with every tenth residue labeled and with the non-crystallographic symmetry axis indicated by a dotted line.
Figure 3A shows the amino acid sequence of M-CSF-specific murine antibody RX1 (SEQ ID NOs: 2 and 4) (encoded by the cDNA insert of the plasmid deposited with the American Type Culture Collection, Manassas, VA, USA, under ATCC deposit number PTA-6113) and a corresponding nucleic acid sequence (SEQ ID NOs: 1 and 3). The CDR regions are numbered and shown in bold.
Figures 3B and 3C show the amino acid sequences of M-CSF specific murine antibody RX1 light (SEQ ID NO: 5) and heavy chains (SEQ ID NO: 6), respectively, with high risk (bold), moderate risk (underline), and low risk residues identified according to Studnicka et al., WO93/11794.
Figure 4A shows that M-CSF antibodies RX1 and 5A1 are species specific.
Figure 4B shows the M-CSF neutralization activity of antibodies MC1 and MC3.
Figure 5 is the amino acid sequence of M-CSFα (SEQ ID NO: 7).

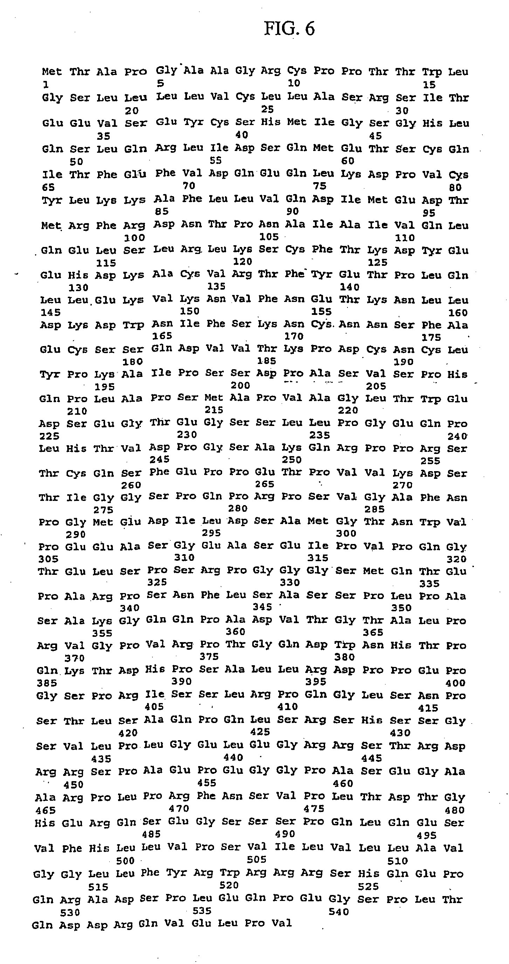

Figure 6 is the amino acid sequence of M-CSFβ (SEQ ID NO: 8).
Figure 7 is the amino acid sequence of M-CSFγ (SEQ ID NO: 9). A number of polymorphisms in the DNA sequence may result in amino acid differences. For example, a common polymorphism provides an Ala rather than Pro at position 104.
Figures 8, 9, and 10 show the amino acid sequences of M-CSF-specific murine antibodies 5H4 (SEQ ID NOs: 10 and 11), MC1 (SEQ ID NOs: 12 and 13) (produced by the hybridoma deposited under ATCC deposit number PTA-6263) and MC3 (SEQ ID NOs: 14 and 15) (produced by the hybridoma deposited under ATCC deposit number PTA-6264), respectively.
Figures 11A and 11B are an alignment of CDR regions of the heavy and light chain amino acid sequences of human M-CSF specific murine antibodies RX1; 5H4; MC1; and MC3 (SEQ ID NOs: 16-38).
Figure 11C shows the neutralization activities of intact versus Fab fragments for RX1 versus 5H4.
Figure 12 shows the structure of M-CSF with RX1, 5H4, and MC3 epitopes highlighted (SEQ ID NOs: 120, 122, and 123).

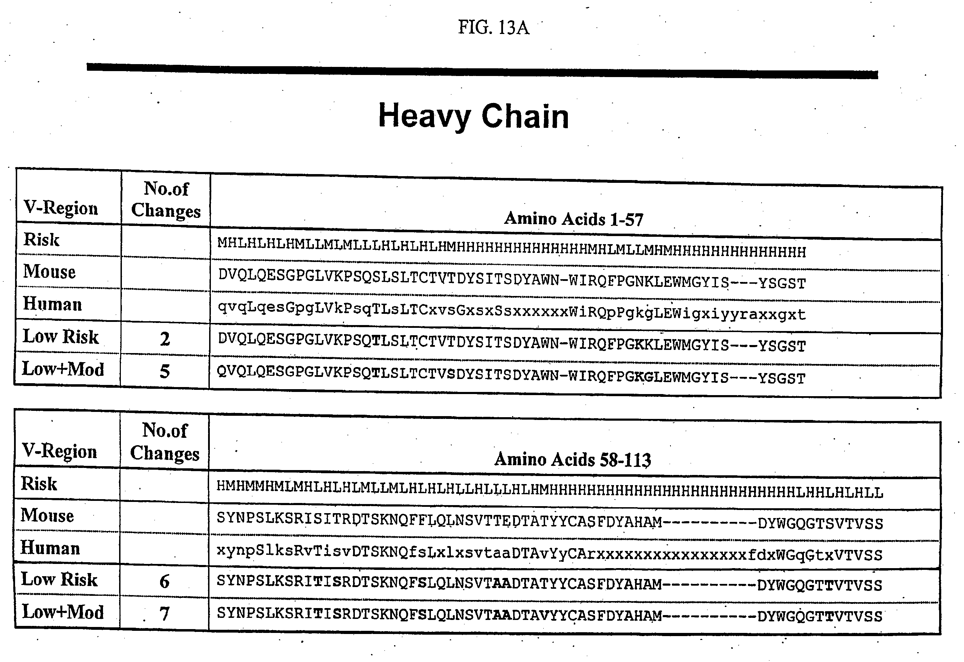

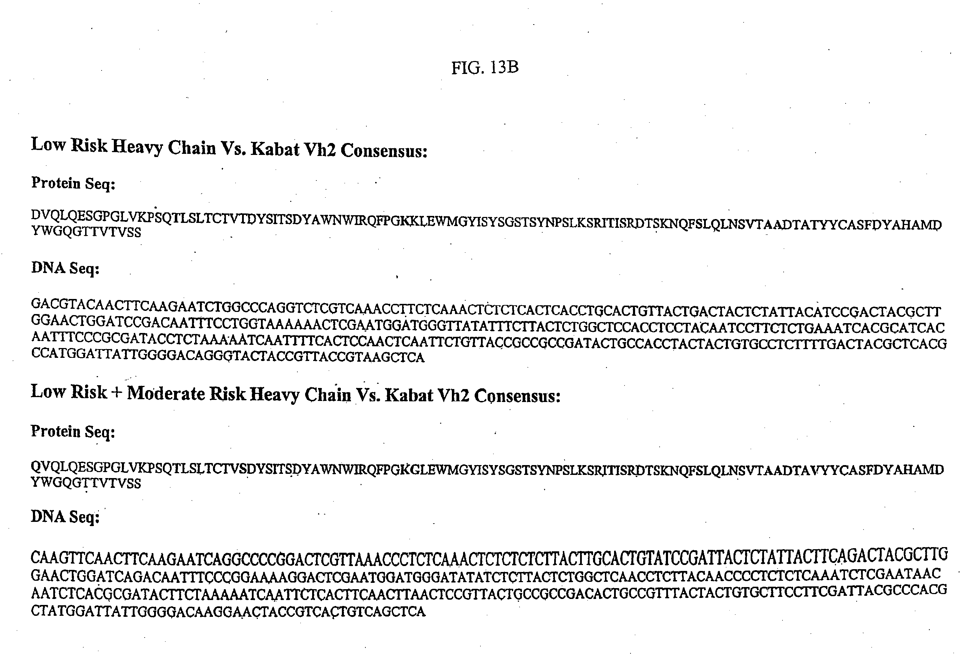

Figure 13A shows (a) the risk line for the murine RX1 heavy chain (H=high risk, M=moderate risk, L=low risk), (b) the RX1 heavy chain amino acid sequence (SEQ ID NO: 6), (c) the amino acid sequence of the closest human consensus sequence, Kabat Vh2 consensus, aligned to RX1 (SEQ ID NO: 39) and (d) changes that were made to produce two exemplary Human Engineered™ sequences (SEQ ID NOs: 41 and 43). Figure 13B shows the amino acid sequences of the two exemplary heavy chain Human Engineered™ sequences (SEQ ID NOs: 41 and 43), designated "low risk" and "low+moderate risk" as well as corresponding nucleic acid sequences (SEQ ID NOs: 40 and 42).

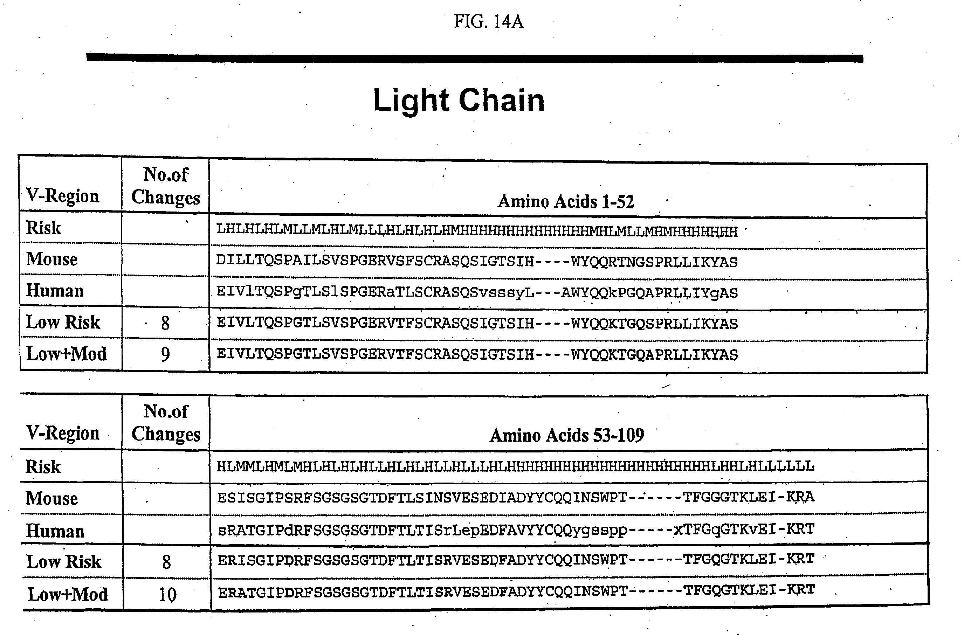

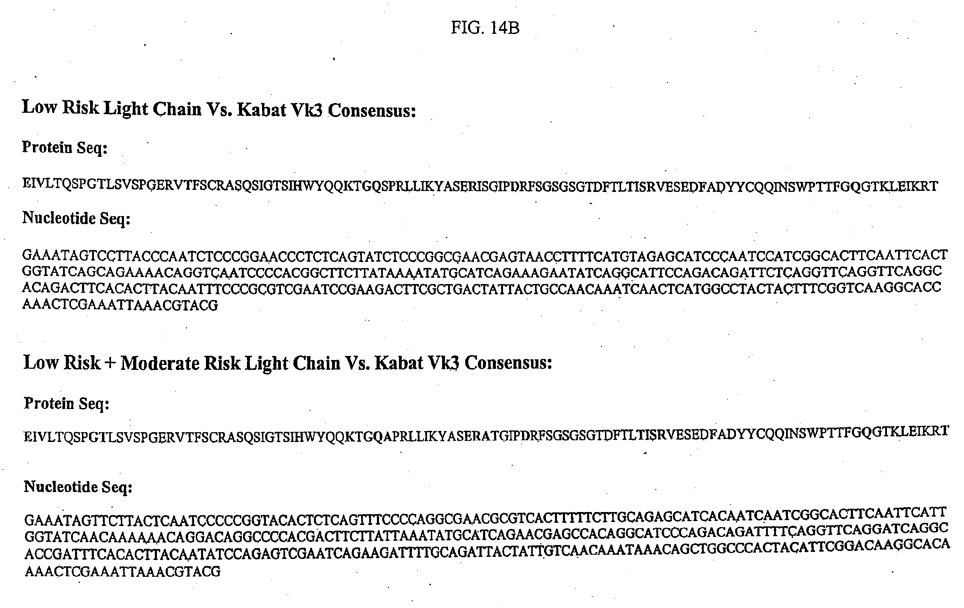

Figure 14A shows (a) the risk line for the murine RX1 light chain (H=high risk, M=moderate risk, L=low risk), (b) the RX1 light chain amino acid sequence (SEQ ID NO: 5), (c) the amino acid sequence of the closest human consensus sequence, Kabat Vk3 consensus, aligned to RX1 (SEQ ID NO: 49) and (d) changes that were made to produce two exemplary Human Engineered™ sequences (SEQ ID NOs: 45 and 47). Figure 14B shows the amino acid sequences of the two exemplary light chain Human Engineered™ sequences (SEQ ID NOs: 45 and 47), designated "low risk" and "low+moderate risk" as well as corresponding nucleic acid sequences (SEQ ID NOs: 44 and 46).

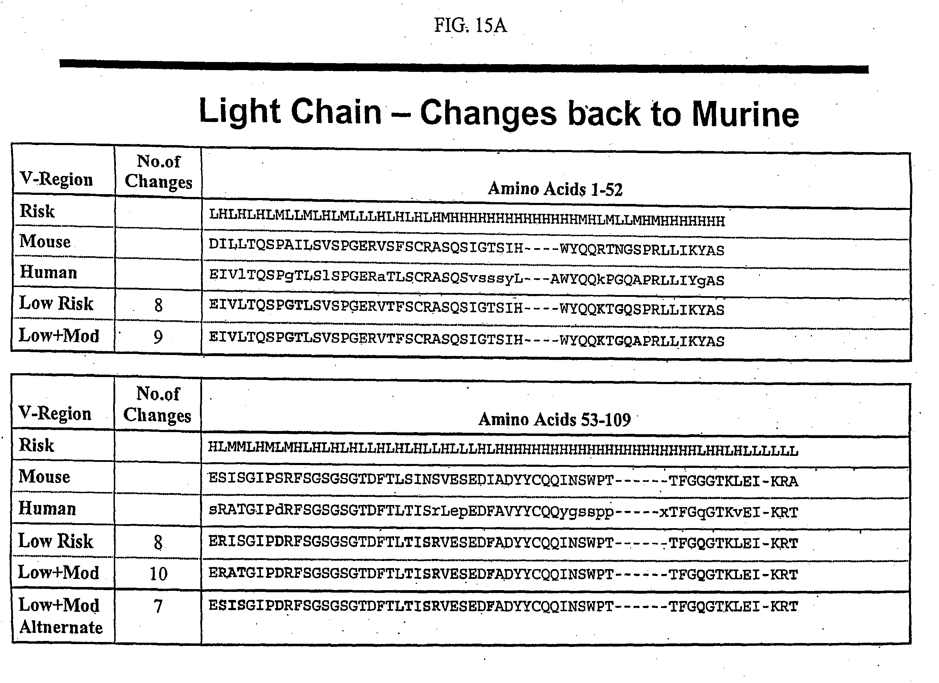

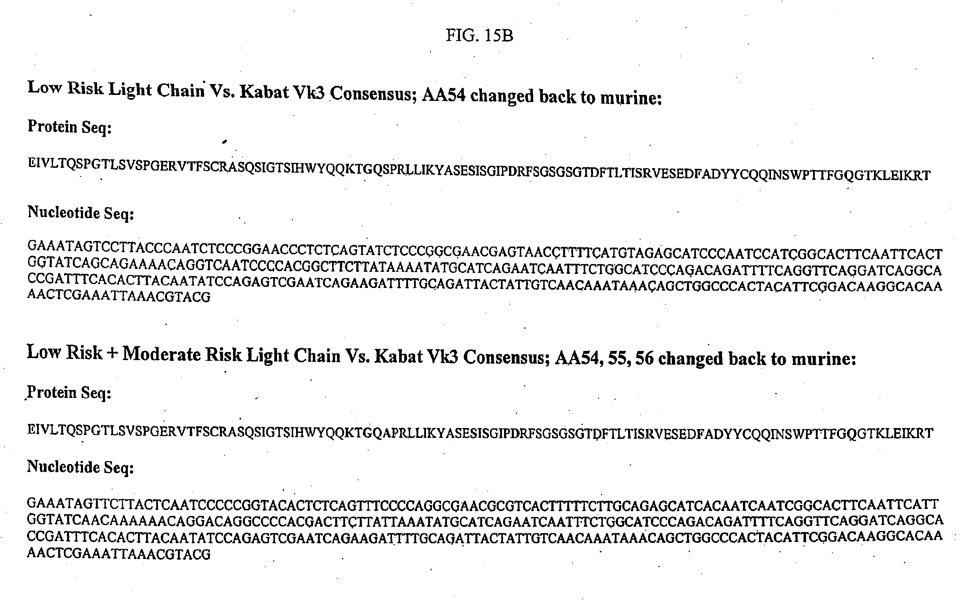

Figure 15A shows (a) the risk line for the murine RX1 light chain (H=high risk, M=moderate risk, L=low risk), (b) the RX1 light chain amino acid sequence (SEQ ID NO: 5), (c) the amino acid sequence of the closest human consensus sequence, Kabat Vk3 consensus, aligned to RX1 (SEQ ID NO: 49) and (d) an alternate exemplary amino acid sequence in which positions 54-56 were not changed (i.e. remained the murine sequence) (SEQ ID NO: 48). Figure 15B shows the amino acid sequences of two exemplary alternate light chain Human Engineered™ sequences (SEQ ID NOs: 48, 136), as well as corresponding nucleic acid sequences (SEQ ID NOs: 137 and 135).

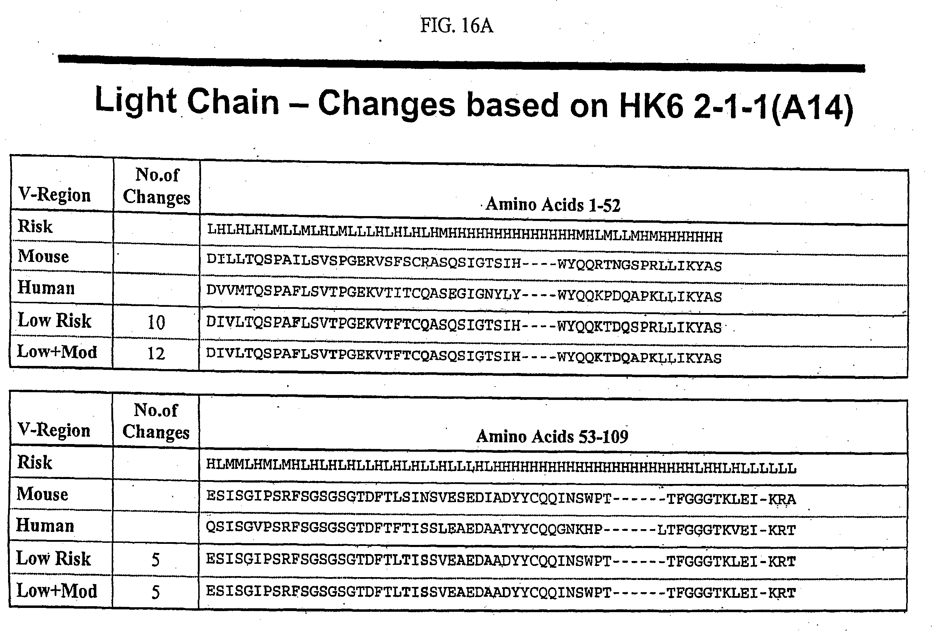

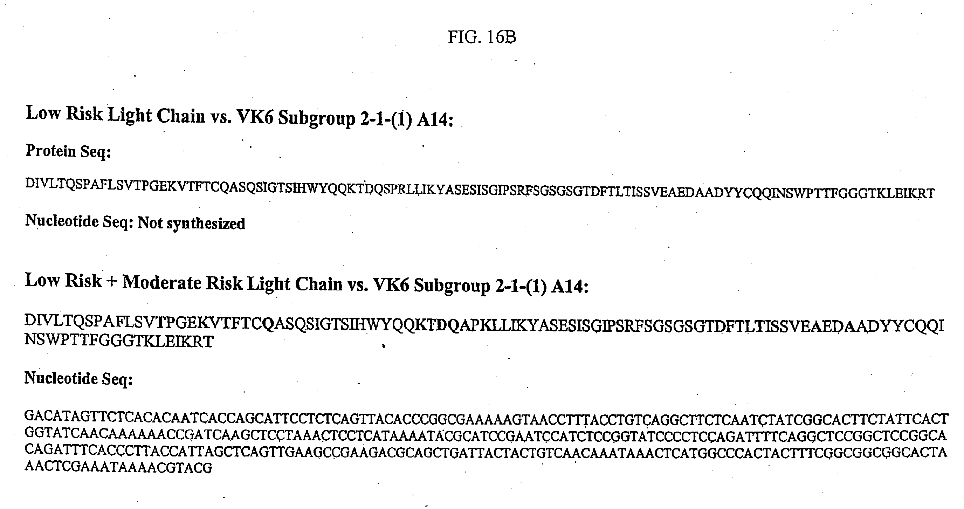

Figure 16A shows (a) the risk line for the murine RX1 light chain (H=high risk, M=moderate risk, L=low risk), (b) the RX1 light chain amino acid sequence (SEQ ID NO: 5), (c) the amino acid sequence of the closest human consensus germline sequence, Vk6 Subgroup 2-1-(1) A14, aligned to RX1 (SEQ ID NO: 50)and (d) changes that were made to produce two exemplary Human Engineered™ sequences (SEQ ID NOs: 51 and 53). Figure 16B shows the amino acid sequences of the two exemplary light chain Human Engineered™ sequences (SEQ ID NOs: 51 and 53), designated "low risk" and "low+moderate risk" as well as the corresponding nucleic acid sequence (SEQ ID NO: 52).

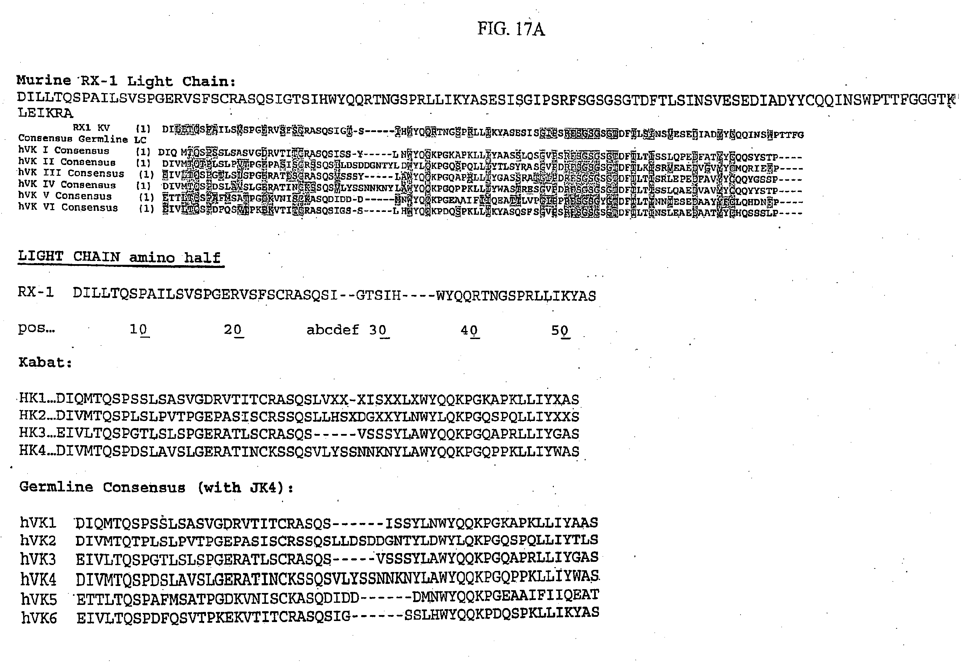

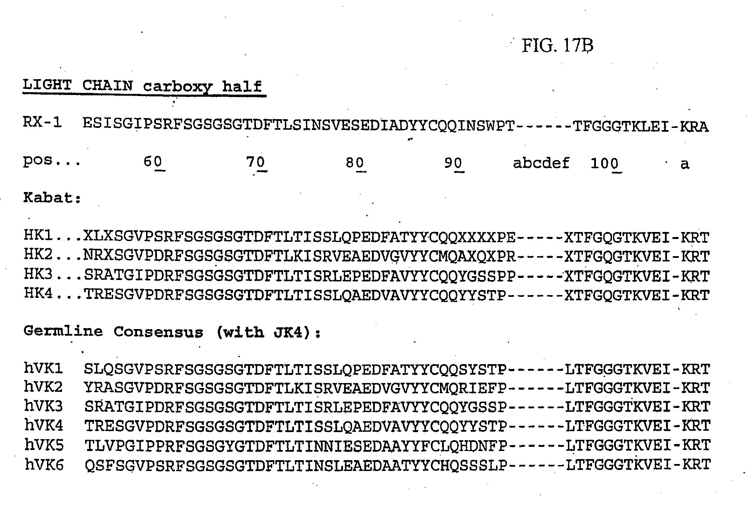

Figures 17A and 17B show the alignment of murine RX1 light chain amino acid sequence (SEQ ID NO: 54) with various human consensus and human germline consensus sequences using the Kabat numbering system (amino acid numbering indicated in line designated "POS") (SEQ ID NOs: 55-82).
Figures 18A and 18B show the alignment of murine RX1 heavy chain amino acid sequence (SEQ ID NO: 83) with various human consensus and human germline consensus sequences using the Kabat numbering system (amino acid numbering indicated in line designated "POS") (SEQ ID NOs: 84-112). Figures 18C-18E show how the amino acid residues of antibodies 5H4, MC1 and MC3 correspond to the Kabat numbering system (SEQ ID NOs: 10 and 11; SEQ ID NOs: 12 and 13; SEQ ID NOs: 14 and 15, respectively).
Figure 19A shows the amino acid (SEQ ID NO: 114) and nucleotide sequence (SEQ ID NO: 113) for heRX1-1.IgG1 with low risk amino acid changes. Figure 19B shows the amino acid (SEQ ID NO: 116) and nucleotide sequence (SEQ ID NO: 115) for heRX1-10.IgG1 with low + moderate risk amino acid changes.
Figure 20 shows the amino acid (SEQ ID NO: 119) and nucleotide sequence (cDNA (SEQ ID NO: 118) and genomic DNA (SEQ ID NO: 117)) for heRX1-1.IgG4 with low risk amino acid changes.

### DETAILED DESCRIPTION

Colony stimulating factor (CSF-1), also known as macrophage colony stimulating factor (M-CSF), is expressed by bone marrow stromal cells, osteoclasts and other cells. M-CSF has been shown to stimulate the production and proliferation of macrophages and osteoclasts, among other cells. Although macrophage activity is beneficial in many situations, macrophage activity is deleterious in a number of situations. Macrophages have been shown to play a role in formation of atherosclerotic plaques, destabilization of plaques and restenosis after angioplasty. Macrophages and production of M-CSF have also been shown to be associated with HIV replication, and infected macrophages may serve as a reservoir of the virus.

Anti-M-CSF-antibodies may be used to prevent or treat macrophage-associated diseases. "Macrophage-associated diseases" as used herein means conditions or disorders associated with or caused by deleterious macrophage activity. Exemplary macrophage-associated diseases include atherosclerotic diseases, or HIV infection and conditions associated therewith. The anti-M-CSF antibodies contemplated for use in such methods include any antibody described herein, including RX1-derived antibodies, RX1-competing antibodies, 5H4-derived antibodies, 5H4-competing antibodies, MC1-derived antibodies, MC1-competing antibodies, MC3-derived antibodies and MC3-competing antibodies.

"Atherosclerotic diseases" include atherosclerosis in any blood vessel, diseases or conditions resulting from atherosclerosis, and conditions associated with increased risk of vessel occlusion or thrombosis, for example, hypertension, diabetes, and other risk factors for myocardial infarction or stroke. Exemplary atherosclerotic diseases include arterial thrombosis, stenosis or ischemia; cardiovascular disease, including occlusive cardiovascular diseases such as angina; arterial thrombosis, such as coronary artery thrombosis or resulting myocardial ischemia or myocardial infarction; restenosis, particularly following angiography or angioplasty; cerebral artery thrombosis or resulting cerebral ischemia or stroke; intracardiac thrombosis (due to, e.g., atrial fibrillation) or resulting stroke; peripheral vascular disease or peripheral arterial thrombosis or occlusion; neointimal hyperplasia, disruption of intercellular junctions in vascular endothelium, or vessel injury.

Diseases or conditions associated with HIV include, but are not limited to, pneumonia, *Pneumocystis carinii, Streptococcus pneumoniae, Haemophilus influenzae, Staphylococcus aureus, Rhodococeus equi, Nocardia asteroids, Histoplasma capsulatum, Coccidioides immitis, Candida species, Aspergillus species, Mycobacterium* avium-complex, *Toxoplasma gondii, Strongyloides stercoralis,* cytomegalovirus, herpes simplex virus,lymphoid interstitial pneumonitis, odynophagia, hairy leukoplakia, erosive gingivitis, aphthous ulcers, gastrointestinal/colitis, *Salmonella* species, *Shigella* species, *Campylobacter jejuni, Cryptosporidium* species, *Isospora* belli, *Blastocystis hominis, Entamoeba histolytica, Giardia lamblia, Microsporida, Strongyloides stercoralis,* adenovirus, nonspecific enteropathy, proctitis, *Chlamydia trachomatis, Neisseria gonorrhoeae, Treponema pallidum,* cholecystitis, extrahepatic obstruction and sclerosing cholangitis, bacillary peliosis hepatitis, encephalitis or dementia, subacute encephalopathy, progressive multifocal leukeoncephalopathy, varicella-zoster virus, *Treponema pallidum,* neoplasm, kaposi's sarcoma, primary or metastatic lymphoma, *Cryptococcus neoformans,* Coccidioides immitis, *Candida albicans, Mycobacterium tuberculosis, Nocardia asteroids,* meningitis, *Listeria rrionocytogenes, Lymphomatous meningitis,* myelitis or neuropathy, vacuolar myelopathy, chronic inflammatory polyneuropathy, distal symmetric sensory motor neuropathy, varicella-zoster virus radiculitis, retinitis, keratoconjunctivitis, cat-scratch disease, non-Hodgkin's lymphoma.

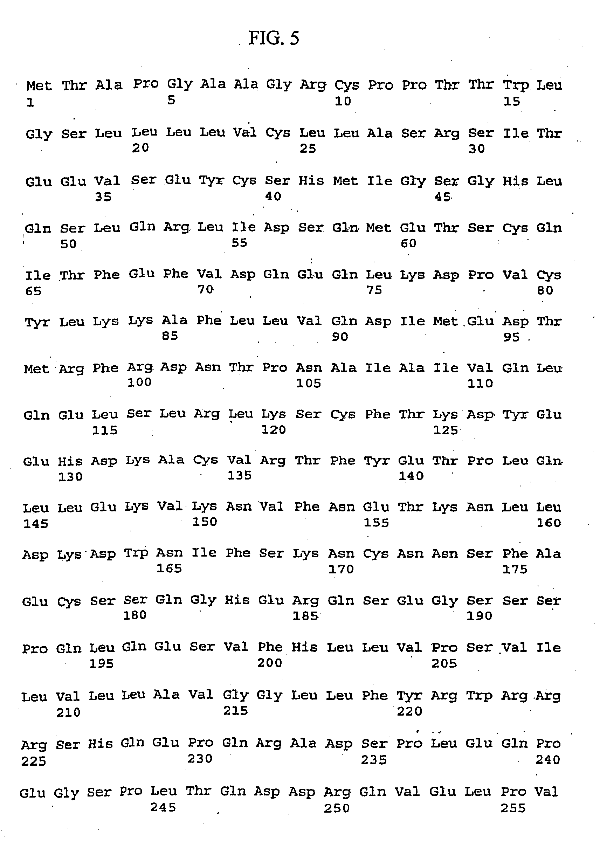

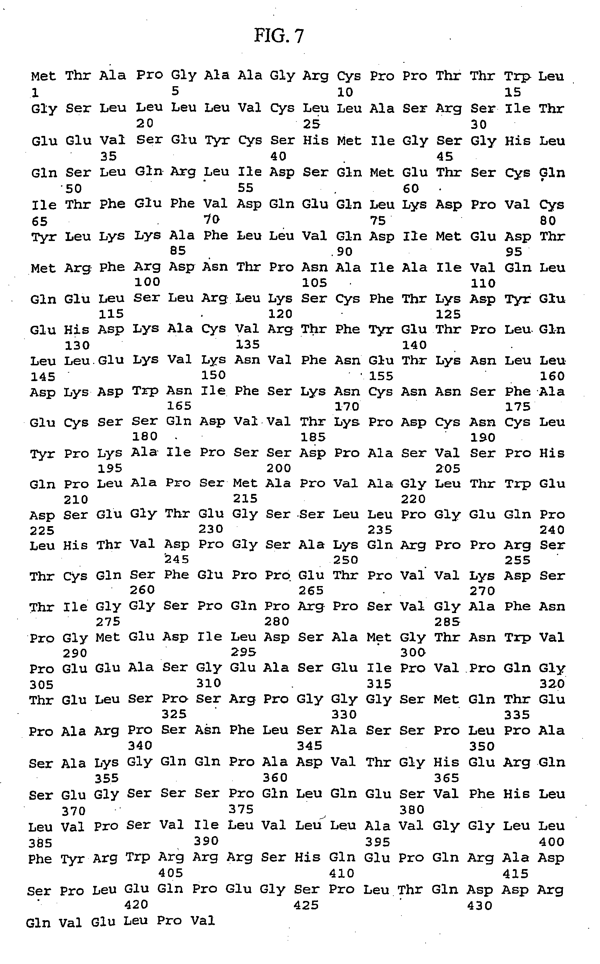

The full-length human M-CSF mRNA encodes a precursor protein of 554 amino acids. Through alternative mRNA splicing and differential post-translational proteolytic processing, M-CSF can either be secreted into the circulation as a glycoprotein or chondroitin sulfate containing proteoglycan or be expressed as a membrane spanning glycoprotein on the surface of M-CSF producing cells. The three-dimensional structure of the bacterially expressed amino terminal 150 amino acids of human M-CSF, the minimal sequence required for full in vitro biological activity, indicates that this protein is a disulfide linked dimer with each monomer consisting of four alpha helical bundles and an anti-parallel beta sheet (Pandit et al., Science 258: 1358-62 (1992)). Three distinct M-CSF species are produced through alternative mRNA splicing. The three polypeptide precursors are M-CSFα of 256 amino acids, M-CSFβ of 554 amino acids, and M-CSFγ of 438 amino acids. M-CSFβ is a secreted protein that does not occur in a membrane-bound form. M-CSFα is expressed as an integral membrane protein that is slowly released by proteolytic cleavage. M-CSFα is cleaved at amino acids 191-197 of the sequence set out in Figure 5. The membrane-bound form of M-CSF can interact with receptors on nearby cells and therefore mediates specific cell-to-cell contacts. The term "M-CSF" may also include amino acids 36-438 of Figure 7.

Various forms of M-CSF function by binding to its receptor M-CSFR (also known as CSF-1R) on target cells. M-CSFR is a membrane spanning molecule with five extracellular immunoglobulin-like domains, a transmembrane domain and an intracellular interrupted Src related tyrosine kinase domain. M-CSFR is encoded by the *c-fms* proto-oncogene. Binding of M-CSF to the extracellular domain of M-CSFR leads to dimerization of the receptor, which activates the cytoplasmic kinase domain, leading to autophosphorylation and phosphorylation of other cellular proteins (Hamilton J. A., J Leukoc Biol.,62(2):145-55 (1997); Hamilton J, A., Immuno Today., 18(7): 313-7(1997).

Phosphorylated cellular proteins induce a cascade of biochemical events leading to cellular responses: mitosis, secretion of cytokines, membrane ruffling, and regulation of transcription of its own receptor (Fixe and Praloran, Cytokine 10: 32-37 (1998)).

An anti-M-CSF antibody designated murine RX1 and having the sequence set forth in SEQ ID NO: 1 was discovered to have superior M-CSF-neutralizing properties compared to other antibodies. Murine RX1 antibody was modified to be less immunogenic in humans based on the Human Engineering™ method of Studnicka et al. Preferably, 8 to 12 surface exposed amino acid residues of the heavy chain variable region and 16 to 19 surface exposed residues in the light chain region were modified to human residues in positions determined to be unlikely to adversely effect either antigen binding or protein folding, while reducing its immunogenicity with respect to a human environment. Synthetic genes containing modified heavy and/or light chain variable regions were constructed and linked to human γ heavy chain and/or kappa light chain constant regions. Any human heavy chain and light chain constant regions may be used in combination with the Human Engineered™ antibody variable regions. The human heavy and light chain genes were introduced into mammalian cells and the resultant recombinant immunoglobulin products were obtained and characterized. Other exemplary anti-M-CSF antibodies such as 5H4, MC1, or MC3 are similarly Human Engineered™.

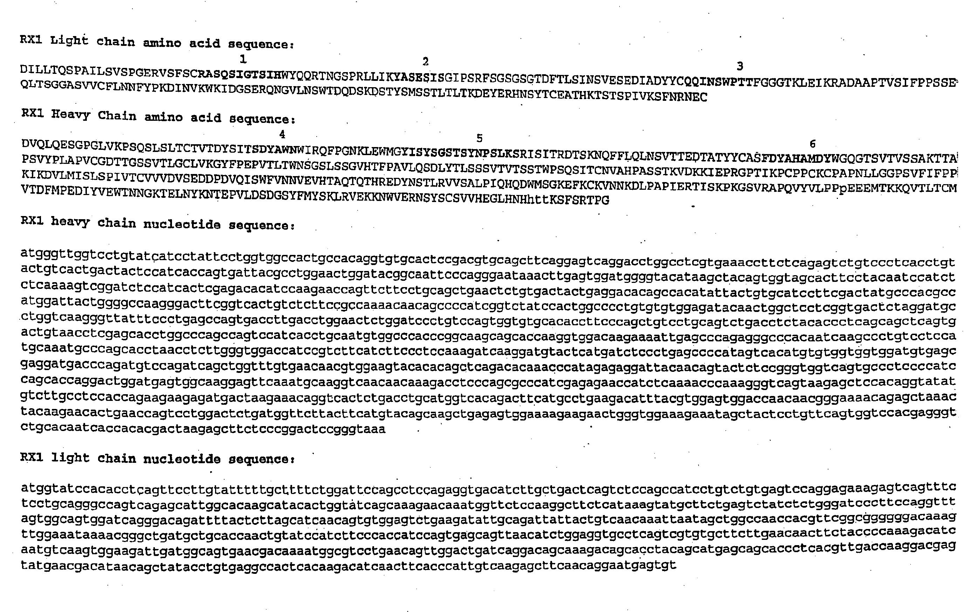

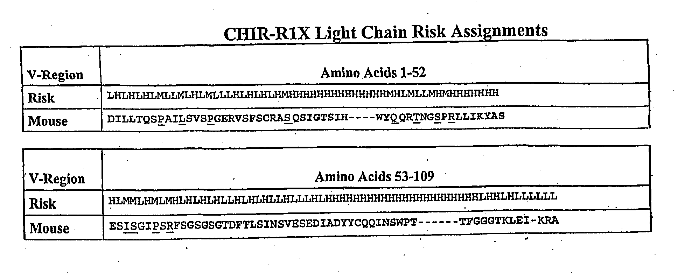

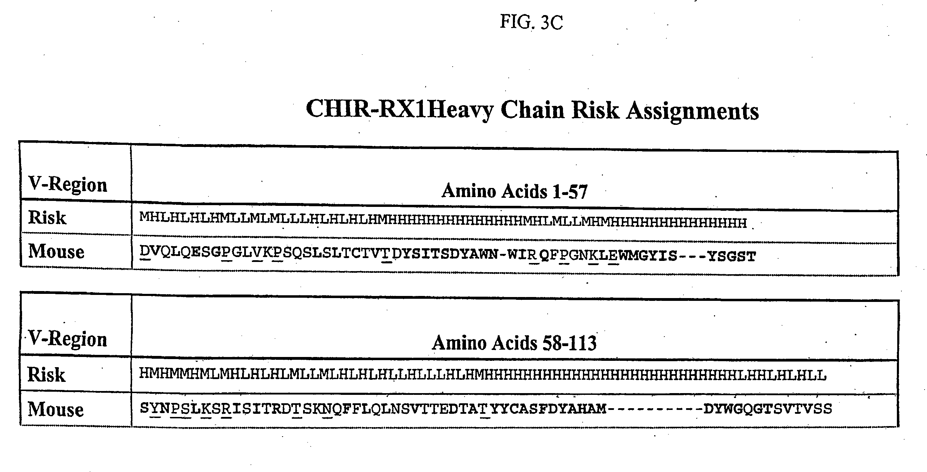

The term "RX 1-derived antibody" includes any one of the following:
1) an amino acid variant of murine antibody RX1 having the amino acid sequence set out in Figure 3, including variants comprising a variable heavy chain amino acid sequence which is at least 60, 65, 70, 75, 80, 85, 90, 91, 92, 93, 94, 95, 96, 97, 98, or 99% homologous to the amino acid sequence as set forth in Figure 3, and/or comprising a variable light chain amino acid sequence which is at least 60, 65, 70, 75, 80, 85, 90, 91, 92, 93, 94, 95, 96, 97, 98, or 99% homologous to the amino acid sequence as set forth in Figure 3, taking into account similar amino acids for the homology determination;
2) M-CSF-binding polypeptides (excluding murine antibody RX 1) comprising one or more complementary determining regions (CDRs) of murine antibody RX 1 having the amino acid sequence set out in Figure 3, preferably comprising at least CDR3 of the RX 1 heavy chain, and preferably comprising two or more, or three or more, or four or more, or five or more, or all six CDRs;
3) Human Engineered™ antibodies having the heavy and light chain amino acid sequences set out in Figures 13B through 16B or variants thereof comprising a heavy or light chain having at least 60% amino acid sequence identity with the original Human Engineered™ heavy or the light chain of Figures 13B through 16B, more preferably at least 80%, more preferably at least 85%, more preferably at least 90%, and most preferably at least 95%, including for example, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, and 100% identical;
4) M-CSF-binding polypeptides (excluding murine antibody RX1) comprising the high risk residues of one or more CDRs of the Human Engineered™ antibodies of Figures 13B through 16B, and preferably comprising high risk residues of two or more, or three or more, or four or more, or five or more, or all six CDRs;
5) Human Engineered™ antibodies or variants retaining the high risk amino acid residues set out in Figure 3B, and comprising one or more changes at the low or moderate risk residues set out in Figure 3B;
   for example, comprising one or more changes at a low risk residue and conservative substitutions at a moderate risk residue set out in Figure 3B, or
   for example, retaining the moderate and high risk amino acid residues set out in Figure 3B and comprising one or more changes at a low risk residue,
   where changes include insertions, deletions or substitutions and may be conservative substitutions or may cause the engineered antibody to be closer in sequence to a human light chain or heavy chain sequence, a human germline light chain or heavy chain sequence, a consensus human light chain or heavy chain sequence, or a consensus human germline light chain or heavy chain sequence;
that retain ability to bind M-CSF. Such antibodies preferably bind to M-CSF with an affinity of at least 10⁻⁷, 10⁻⁸ or 10⁻⁹ or higher and preferably neutralize the desired biological activity of M-CSF.

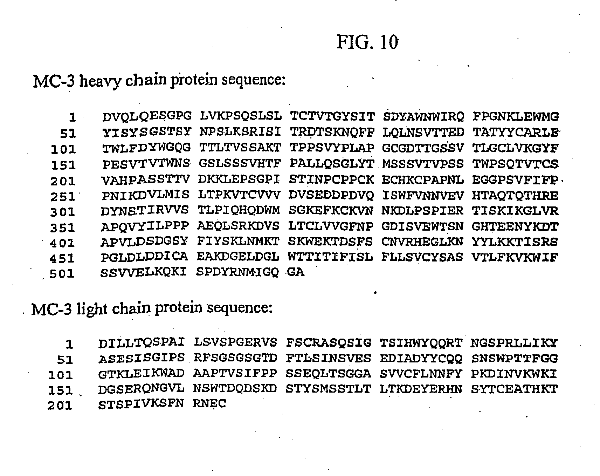

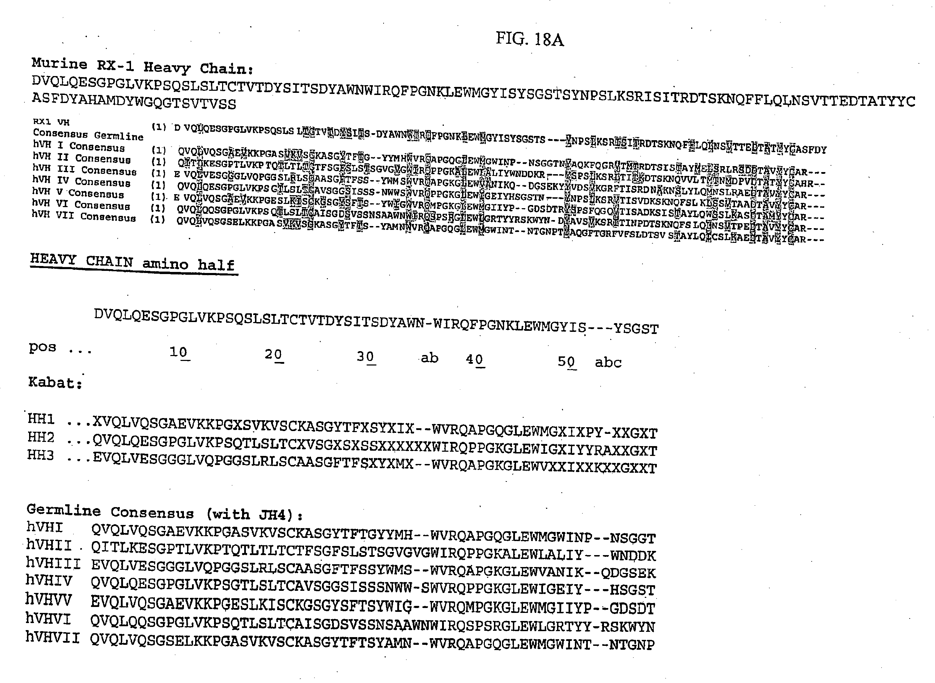

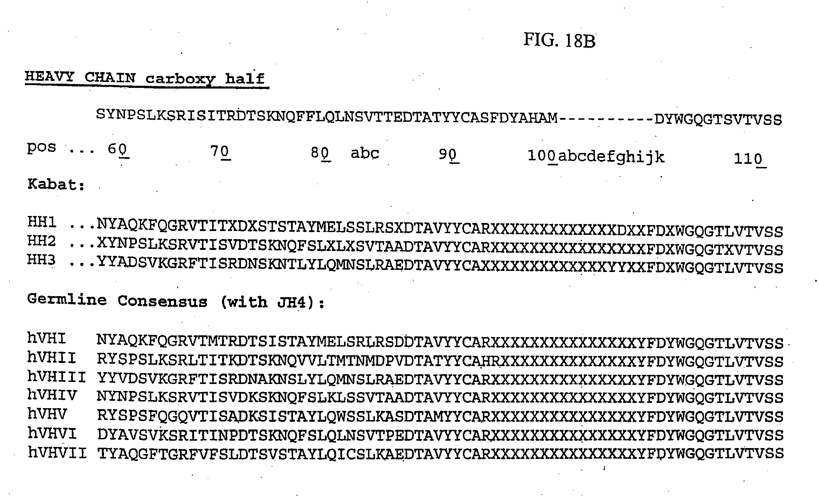

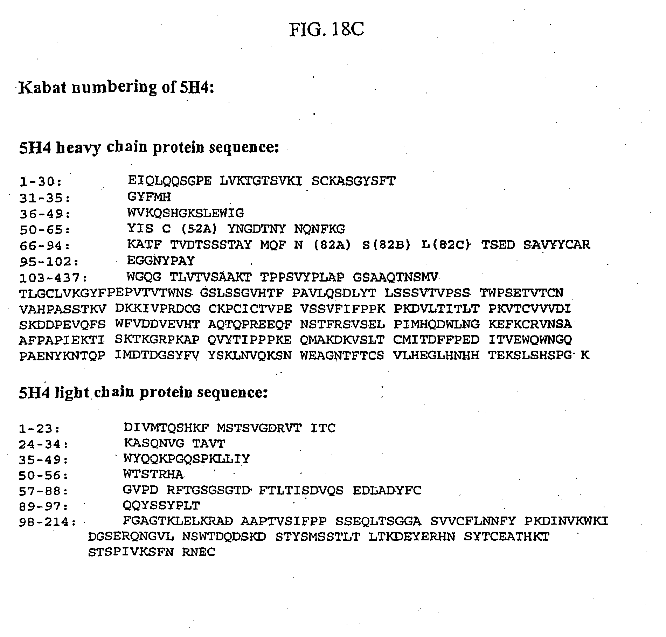

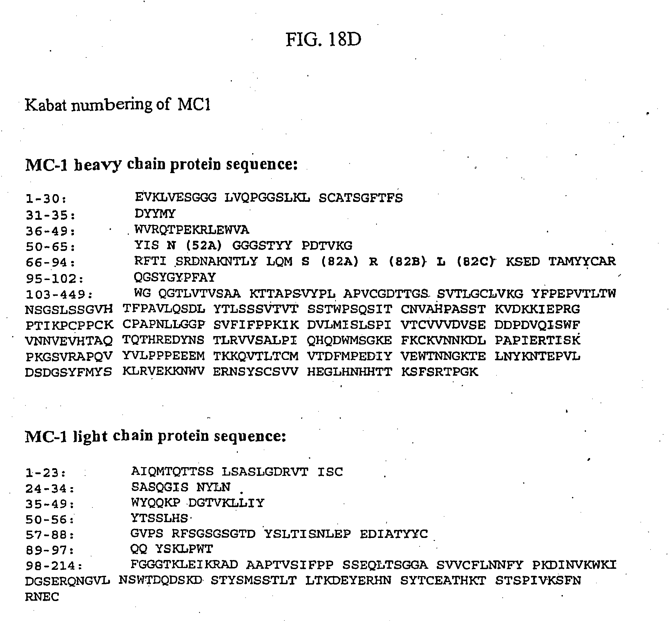

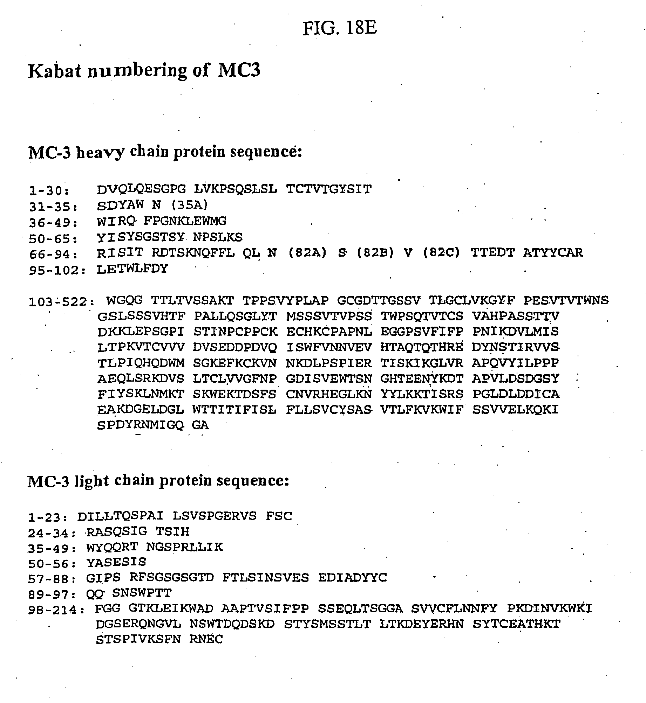

Similarly, the term "MC3-derived antibody" includes any one of the following:
1) an amino acid variant of murine antibody MC3 having the amino acid sequence set out in Figure 10, including variants comprising a variable heavy chain amino acid sequence which is at least 60, 65, 70, 75, 80, 85, 90, 91, 92, 93, 94, 95, 96, 97, 98, or 99% homologous to the amino acid sequence as set forth in Figure 10, and/or comprising a variable light chain amino acid sequence which is at least 60, 65, 70, 75, 80, 85, 90, 91, 92, 93, 94, 95, 96, 97, 98, or 99% homologous to the amino acid sequence as set forth in Figure 10, taking into account similar amino acids for the homology determination;
2) M-CSF-binding polypeptides (optionally including or excluding murine antibody MC3) comprising one or more complementary determining regions (CDRs) of murine antibody MC3 having the amino acid sequence set out in Figure 10, preferably comprising at least CDR3 of the MC3 heavy chain, and preferably comprising two or more, or three or more, or four or more, or five or more, or all six CDRs;
3) Human Engineered™ antibodies generated by altering the murine sequence according to the methods set forth in Studnicka et al., U.S. Patent No. 5,766,886 and Example 3 herein, using the Kabat numbering set forth in figures 18C-18E to identify low, moderate and high risk residues; such antibodies comprising at least one of the following heavy chains and at least one of the following light chains: (a) a heavy chain in which all of the low risk residues have been modified, if necessary, to be the same residues as a human reference immunoglobulin sequence or (b) a heavy chain in which all of the low and moderate risk residues have been modified, if necessary, to be the same residues as a human reference immunoglobulin sequence, (c) a light chain in which all of the low risk residues have been modified, if necessary, to be the same residues as a human reference immunoglobulin sequence or (b) a light chain in which all of the low and moderate risk residues have been modified, if necessary, to be the same residues as a human reference immunoglobulin sequence
4) variants of the aforementioned antibodies in preceding paragraph (3) comprising a heavy or light chain having at least 60% amino acid sequence identity with the original Human Engineered™ heavy or the light chain, more preferably at least 80%, more preferably at least 85%, more preferably at least 90%, and most preferably at least 95%, including for example, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, and 100% identical;
5) M-CSF-binding polypeptides (optionally including or excluding murine antibody MC3) comprising the high risk residues of one or more CDRs of the murine MC3 antibody of Figure 10, and preferably comprising high risk residues of two or more, or three or more, or four or more, or five or more, or all six CDRs;
6) Human Engineered™ antibodies or variants retaining the high risk amino acid residues of murine MC3 antibody, and comprising one or more changes at the low or moderate risk residues;
   for example, comprising one or more changes at a low risk residue and conservative substitutions at a moderate risk residue, or
   for example, retaining the moderate and high risk amino acid residues and comprising one or more changes at a low risk residue,
   where changes include insertions, deletions or substitutions and may be conservative substitutions or may cause the engineered antibody to be closer in sequence to a human light chain or heavy chain sequence, a human germline light chain or heavy chain sequence, a consensus human light chain or heavy chain sequence, or a consensus human germline light chain or heavy chain sequence;
that retain ability to bind M-CSF. Such antibodies preferably bind to M-CSF with an affinity of at least 10⁻⁷, 10⁻⁸ or 10⁻⁹ or higher and preferably neutralize the desired biological activity of M-CSF.

The term "5H4-derived antibody" or "MC1-derived antibody" is similarly defined according to the above description.

Anti-M-CSF antibodies, such asRX1, 5H4, MC1 or MC3-derived antibodies, including Human Engineered™ antibodies or variants, may be of different isotypes, such as IgG, IgA, IgM or IgE. Antibodies of the IgG class may include a different constant region, e.g. an IgG2 antibody may be modified to display an IgG1 or IgG4 constant region. , Human Engineered™ antibodies or variants comprising a modified or unmodified IgG1 or IgG4 constant region are disclosed herein. In the case of IgG1, modifications to the constant region, particularly the hinge or CH2 region, may increase or decrease effector function, including ADCC and/or CDC activity. An IgG2 constant region may be modified to decrease antibody-antigen aggregate formation. In the case of IgG4, modifications to the constant region, particularly the hinge region, may reduce the formation of half-antibodies. In a specific example, mutating the IgG4 hinge sequence Cys-Pro-Ser-Cys to the IgG1 hinge sequence Cys-Pro-Pro-Cys isdisclosed.

Human Engineered™ antibodies containing IgG1 or IgG4 constant regions have improved properties compared to Human Engineered™ antibodies containing IgG2 constant regions. Choice of the IgG1 or IgG4 Fc region improved binding affinity and M-CSF neutralization activity. In addition, choice of the IgG1 or IgG4 Fc region provided antigen-antibody complexes that more closely resembled those formed by the parent murine antibody.

The mobility at the hinge region thus appears to markedly affect binding of antibody to the dimeric antigen M-CSF as well as neutralization activity of the antibody. It is contemplated that preparation of antibodies containing a heavy chain comprising a modified or unmodified IgG1 or IgG4 constant region, particularly the hinge and CH2 domains, or preferably at least the hinge domains, improves binding affinity and/or slows dissociation of antibody from dimeric antigens.

The term "RX1-competing antibody" includes
1) a non-murine or non-rodent monoclonal antibody that binds to the same epitope of M-CSF as murine RX1 having the complete light and heavy chain sequences set out in Figure 3;
2) a non-murine or non-rodent monoclonal antibody that binds to at least 4 contiguous amino acids of amino acids 98-105 of the M-CSF of Figure 7; and
3) a non-murine or non-rodent monoclonal antibody that competes with murine antibody RX1 having the complete sequence set out in Figure 3 for binding to M-CSF, by more than 75%, more than 80%, or more than 81 %, 82%, 83%, 84%, 85%, 86%, 87%, 88%, 89%, 90%, 91%, 92%, 93%, 94% or 95%. Such antibodies preferably bind to M-CSF with an affinity of at least 10⁻⁷, 10⁻⁸ or 10⁻⁹ or higher and preferably neutralize the desired biological activity of M-CSF.

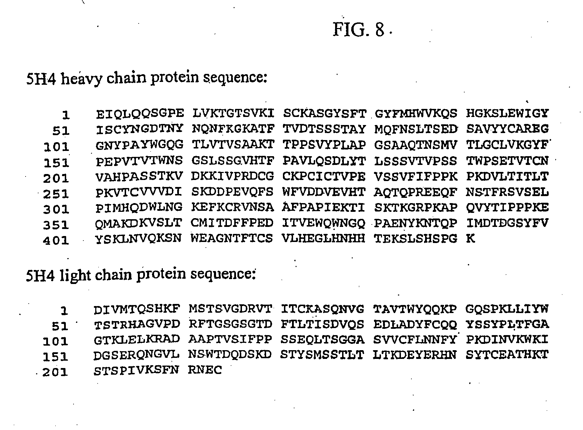

The term "MC1-competing antibody" or "MC3-competing antibody" or "5H4-competing antibody" is similarly defined with reference to the murine 5H4, MC1 or MC3 antibodies having the complete light and heavy chain sequences set out in Figure 8, 9 or 10, respectively, and with reference to the epitope of M-CSF bound by the antibody, e.g. amino acids 65-73 or 138-144 of Figure 7 (corresponding to M-CSF epitopes recognized by 5H4 or MC3).

Optionally, any chimeric, human or humanized M-CSF antibody publicly disclosed before the filing date hereof, or disclosed in an application filed before the filing date hereof, is excluded from the scope of the invention.

"Non-rodent" monoclonal antibody is any antibody, as broadly defined herein, that is not a complete intact rodent monoclonal antibody generated by a rodent hybridoma. Thus, non-rodent antibodies specifically include, but are not limited to, variants of rodent antibodies, rodent antibody fragments, linear antibodies, chimeric antibodies, humanized antibodies, Human Engineered™ antibodies and human antibodies, including human antibodies produced from transgenic animals or via phage display technology. Similarly, non-murine antibodies include but are not limited to variants of murine antibodies, murine antibody fragments, linear antibodies, chimeric, humanized, Human Engineered™ and human antibodies.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. Treatment of patients suffering from clinical, biochemical, radiological or subjective symptoms of the disease, such as a macrophage-associated disease, may include alleviating some or all of such symptoms or reducing the predisposition to the disease. The "pathology" of the disease includes all phenomena that compromise the well being of the patient.

"Mammal" for purposes of treatment refers to any animal classified as a mammal, including humans, domestic and farm animals, and zoo, sports, or pet animals, such as dogs, horses, cats, cows, etc. Preferably, the mammal is human.

As used herein, the phrase "therapeutically effective amount" is meant to refer to an amount of therapeutic or prophylactic M-CSF antibody that would be appropriate, that will elicit the desired therapeutic or prophylactic effect or response, including alleviating some or all of such symptoms of disease or reducing the predisposition to the disease, when administered in accordance with the desired treatment regimen.

Human "M-CSF" as used herein refers to a human polypeptide having substantially the same amino acid sequence as the mature human M-CSFα, M-CSFβ, or M-CSFγ polypeptides described in Kawasaki et al., Science 230:291 (1985), Cerretti et al., Molecular Immunology, 25:761 (1988), or Ladner et al., EMBO Journal 6:2693 (1987. Such terminology reflects the understanding that the three mature M-CSFs have different amino acid sequences, as described above, and that the active form of M-CSF is a disulfide bonded dimer; thus, when the term "M-CSF" refers to the biologically active form, the dimeric form is intended. "M-CSF dimer" refers to two M-CSF polypeptide monomers that have dimerized and includes both homodimers (consisting of two of the same type of M-CSF monomer) and heterodimers (consisting of two different monomers). M-CSF monomers may be converted to M-CSF dimers in vitro as described in U.S. Pat. No. 4,929,700.

### Anti-M-CSF antibodies

The present disclosure provides methods of treating subjects suffering from macrophage-associated diseases using the M-CSF-specific antibodies described herein, including preparation of a medicament for treating subjects suffering from macrophage-associated diseases. The term "antibody" is used in the broadest sense and includes fully assembled antibodies, monoclonal antibodies, polyclonal antibodies, multispecific antibodies (e.g., bispecific antibodies), antibody fragments that can bind antigen (e.g., Fab', F'(ab)2, Fv, single chain antibodies, diabodies), and recombinant peptides comprising the forgoing as long as they exhibit the desired biological activity. In addition to intact, full-length molecules, the term "antibody" also refers to fragments thereof (such as, e.g., scFv, Fv, Fd, Fab, Fab' and F(ab)'2 fragments) or multimers or aggregates of intact molecules and/or fragments that bind to M-CSF (or M-CSFR). These antibody fragments bind antigen and may be derivatized to exhibit structural features that facilitate clearance and uptake, e.g., by incorporation of galactose residues.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that are typically include different antibodies directed against different determinants (epitopes), each monoclonal antibody is directed against a single determinant on the antigen. In addition to their specificity, the monoclonal antibodies are advantageous in that they are synthesized by the homogeneous culture, uncontaminated by other immunoglobulins with different specificities and characteristics.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al., Nature, 256:495 [19751, or may be made by recombinant DNA methods (see, e.g., U.S. Patent No. 4,816,567). The "monoclonal antibodies" may also be isolated from phage antibody libraries using the techniques described inClackson et al.,Nature,352:624628[1991] end Marks et al., J. Mol. Biol., 222:581-597 (1991), for example.

Depending on the amino acid sequence of the constant domain of their heavy chains, immunoglobulins can be assigned to different classes. There are five major classes, IgA, IgD, IgE, IgG and IgM, and several of these may be further divided into subclasses or isotypes, e.g. IgG1, IgG2, IgG3, IgG4, IgA1 and IgA2. The heavy-chain constant domains that correspond to the different classes of immunoglobulins are called alpha, delta, epsilon, gamma and mu respectively. The subunit structures and three-dimensional configurations of different classes of immunoglobulins are well known. Different isotypes have different effector functions; for example, IgG1 and IgG3 isotypes have ADCC activity.

"Antibody fragments" comprise a portion of an intact full length antibody, preferably the antigen binding or variable region of the intact antibody. Examples of antibody fragments include Fab, Fab', F(ab')2, and Fv fragments; diabodies; linear antibodies (Zapata et al., Protein Eng.,8(10):1057-1062 (1995)); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two "Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains that enables the Fv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "hypervariable" region refers to the amino acid residues of an antibody which are responsible for antigen-binding. The hypervariable region comprises amino acid residues from a complementarity determining region or CDR [i.e., residues 24-34 (L1), 50-56 (L2) and 89-97 (L3) in the light chain variable domain and 31-35 (H1), 50-65 (H2) and 95-102 (H3) in the heavy chain variable domain as described by Kabat et al., Sequences of Proteins of Immunological Interest, 5th Ed. Public Health Service, National Institutes of Health, Bethesda, Md. (1991)] and/or those residues from a hypervariable loop *(i.e.,* residues 26-32 (L1), 50-52 (L2) and 91-96 (L3) in the light chain variable domain and 26-32 (H1), 53-55 (H2) and 96-101 (H3) in the heavy chain variable domain as described by [Chothia et al., J. Mol.Biol. 196: 901-917 (1987)].

"Framework" or FR residues are those variable domain residues other than the hypervariable region residues.

The term "diabodies" refers to small antibody fragments with two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and 30 Hollinger et al., Proc. Natl. Acad. Sci. USA, 90:6444-6448 (1993).

It may be desirable to generate multispecific (e.g. bispecific) monoclonal antibody including monoclonal, human, humanized, Human Engineered™ or variant anti-M-CSF antibodies having binding specificities for at least two different epitopes. Exemplary bispecific antibodies may bind to two different epitopes of M-CSF. Alternatively, an anti-M-CSF arm may be combined with an arm which binds to a triggering molecule on a leukocyte such as a T-cell receptor molecule (e.g., CD2 or CD3), or Fc receptors for IgG (FcγR), such as FcγRI (CD64), FcγRII (CD32) and FcγRIII (CD16) so as to focus cellular defense mechanisms to the M-CSF-expressing cell. Bispecific antibodies may also be used to localize therapeutic agents to cells which express M-CSF. These antibodies possess an M-CSF-binding arm and an arm which binds the therapeutic agent. Bispecific antibodies can be prepared as full length antibodies or antibody fragments (e.g., F(ab').sub.2 bispecific antibodies).

According to another approach for making bispecific antibodies, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers. See WO96/27011 published Sep. 6, 1996.

Bispecific antibodies include cross-linked or "heteroconjugate" antibodies. For example, one of the antibodies in the heteroconjugate can be coupled to avidin, the other to biotin. Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes. Fab'-SH fragments directly recovered from E. coli may be chemically coupled in vitro to form bispecific antibodies. (Shalaby et al., J. Exp. Med. 175:217-225 (1992))

Shalaby et al., J. Exp. Med. 175:217-225 (1992) describe the production of a fully humanized bispecific antibody F(ab')₂ molecule. Each Fab' fragment was separately secreted from *E.coli* and subjected to directed chemical coupling in vitro to form the bispecfic antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. (Kostelny et al., J. Immunol. 148(5):1547-1553 (1992)) The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments.

The fragments comprise a heavy chain variable region (V_{H}) connected to a light-chain variable region (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. *See* Gruber et al., J. Immunol. 152: 5368 (1994).

Alternatively, the bispecific antibody may be a "linear antibody" produced as described in Zapata et al. Protein Eng. 8(10):1057-1062 (1995). Briefly, these antibodies comprise a pair of tandem Fd segments (V_{H} -C_{H}1-V_{H} -C_{H}1) which form a pair of antigen binding regions. Linear antibodies can be bispecific or monospecific.

Antibodies with more than two valencies are also contemplated. For example, trispecific antibodies can be prepared. (Tutt et al., J. Immunol. 147:60 (1991))

The monoclonal, human, humanized, Human Engineered™ or variant anti-M-CSF antibody may be an antibody fragment, such as an RX1, 5H4, MC1, or MC3 antibody fragment. Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992) and Brennan et al., Science 229:81 (1985)). However, these fragments can now be produced directly by recombinant host cells. Better et al., Science 240: 1041-1043 (1988) disclose secretion of functional antibody fragments from bacteria *(see, e.g.,* Better et al., Skerra et al. Science 240: 1038-1041 (1988)). For example, Fab'-SH fragments can be directly recovered from E. coli and chemically coupled to form F(ab')₂ fragments (Carter et al., Bio/Technology 10:163-167 (1992)). The F(ab')₂ is formed using the leucine zipper GCN4 to promote assembly of the F(ab')₂ molecule. According to another approach, Fv, Fab or F(ab')₂ fragments can be isolated directly from recombinant host cell culture. Other techniques for the production of antibody fragments will be apparent to the skilled practitioner.

An "isolated" antibody is one that has been identified and separated and recovered from a component of its natural environment. Contaminant components of its natural environment are materials that would interfere with diagnostic or therapeutic uses for the antibody, and may include enzymes, hormones, and other proteinaceous or nonproteinaceous solutes. The antibody may be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

For a detailed description of the structure and generation of antibodies, see Roth, D.B., and Craig, N.L., Cell, 94:411-414 (1998), and United States Patent No. 6,255,458. Briefly, the process for generating DNA encoding the heavy and light chain immunoglobulin genes occurs primarily in developing B-cells. Prior to the rearranging and joining of various immunoglobulin gene segments, the V, D, J and constant (C) gene segments are found generally in relatively close proximity on a single chromosome. During B-cell-differentiation, one of each of the appropriate family members of the V, D, J (or only V and J in the case of light chain genes) gene segments are recombined to form functionally rearranged heavy and light immunoglobulin genes. This gene segment rearrangement process appears to be sequential. First, heavy chain D-to-J joints are made, followed by heavy chain V-to-DJ joints and light chain V-to-J joints.

The recombination of variable region gene segments to form functional heavy and light chain variable regions is mediated by recombination signal sequences (RSS's) that flank recombinationally competent V, D and J segments. RSS's necessary and sufficient to direct recombination, comprise a dyad-symmetric heptamer, an AT-rich nonamer and an intervening spacer region of either 12 or 23 base pairs. These signals are conserved among the different loci and species that carry out D-J (or V-J) recombination and are functionally interchangeable. See Oettinger, et al. (1990), Science, 248, 1517-1523 and references cited therein. The heptamer comprises the sequence CACAGTG or its analogue followed by a spacer of unconserved sequence and then a nonamer having the sequence ACAAAAACC or its analogue. These sequences are found on the J, or downstream side, of each V and D gene segment. Immediately preceding the germline D and J segments are again two recombination signal sequences, first the nonamer and then the heptamer again separated by an unconserved sequence. The heptameric and nonameric sequences following a V_{L}, V_{H} or D segment are complementary to those preceding the J_{L}, D or J_{H} segments with which they recombine. The spacers between the heptameric and nonameric sequences are either 12 base pairs long or between 22 and 24 base pairs long.

In addition to the rearrangement of V, D and J segments, further diversity is generated in the primary repertoire of immunoglobulin heavy and light chain by way of variable recombination at the locations where the V and J segments in the light chain are joined and where the D and J segments of the heavy chain are joined. Such variation in the light chain typically occurs within the last codon of the V gene segment and the first codon of the J segment. Similar imprecision in joining occurs on the heavy chain chromosome between the D and J_{H} segments and may extend over as many as 10 nucleotides. Furthermore, several nucleotides may be inserted between the D and J_{H} and between the V_{H} and D gene segments which are not encoded by genomic DNA. The addition of these nucleotides is known as N-region diversity.

The net effect of such rearrangements in the variable region gene segments and the variable recombination which may occur during such joining is the production of a primary antibody repertoire.

"Fv" is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen binding site on the surface of the VH VI dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them.

By "neutralizing antibody" is meant an antibody molecule that is able to eliminate or significantly reduce an effecter function of a target antigen to which is binds. Accordingly, a "neutralizing" anti-target antibody is capable of eliminating or significantly reducing an effecter function, such as enzyme activity, ligand binding, or intracellular signaling.

As disclosed herein, the compositions for and methods of treating macrophage-associated diseases may utilize one or more antibody used singularly or in combination with other therapeutics to achieve the desired effects. Antibodies according to the present disclosure may be isolated from an animal producing the antibody as a result of either direct contact with an environmental antigen or immunization with the antigen. Alternatively, antibodies may be produced by recombinant DNA methodology using one of the antibody expression systems well known in the art (See, e.g., Harlow and Lane, Antibodies: A Laboratory Manual, Cold Spring Harbor Laboratory (1988)). Such antibodies may include recombinant IgGs, chimeric fusion proteins having immunoglobulin derived sequences or "Human Engineered™" antibodies that may all be used for the treatment of macrophage-associated diseases according to the presentdisclosure.

M-CSF monoclonal antibodies may be prepared essentially as described in Halenbeck et al. U.S. Pat. No. 5,491,065 (1997). Exemplary M-CSF monoclonal antibodies include those that bind to an apparent conformational epitope associated with recombinant or native dimeric M-CSF with concomitant neutralization of biological activity. These antibodies are substantially unreactive with biologically inactive forms of M-CSF including monomeric and chemically derivatized dimeric M-CSF.

Human Engineered™ anti-M-CSF monoclonal antibodies are disclosed herein. The phrase "Human Engineered™ antibody" refers to an antibody derived from a non-human antibody, typically a mouse monoclonal antibody. Alternatively, a Human Engineered™ antibody may be derived from a chimeric antibody that retains or substantially retains the antigen binding properties of the parental, non-human, antibody but which exhibits diminished immunogenicity as compared to the parental antibody when administered to humans. The phrase "chimeric antibody," as used herein, refers to an antibody containing sequence derived from two different antibodies (see, e.g., U.S. Patent No. 4,816,567) which typically originate from different species. Most typically, chimeric antibodies comprise human and murine antibody fragments, generally human constant and mouse variable regions.

The phrase "complementarity determining region" or the term "CDR" refers to amino acid sequences which together define the binding affinity and specificity of the natural Fv region of a native immunoglobulin binding site (See, e.g., Chothia et al., J. Mol. Biol. 196:901 917 (1987); Kabat et al., U.S. Dept. of Health and Human Services NIH Publication No. 91 3242 (1991)). The phrase "constant region" refers to the portion of the antibody molecule that confers effector functions. In the present disclosure, mouse constant regions are preferably substituted by human constant regions. The constant regions of the subject antibodies are derived from human immunoglobulins. The heavy chain constant region can be selected from any of the five isotypes: alpha, delta, epsilon, gamma or mu.

The antibodies of the present disclosure are said to be immunospecific or specifically binding if they bind to antigen with a Kₐ of greater than or equal to about 10⁶M⁻¹ preferably greater than or equal to about 10⁷M⁻¹, more preferably greater than or equal to about 10⁸M⁻¹, and most preferably greater than or equal to about 10⁹M⁻¹, 10¹⁰M⁻¹, 10¹¹M⁻¹ or 10¹²M⁻¹. The anti-M-CSF antibodies may bind to different naturally occurring forms of M-CSF. The monoclonal antibodies disclosed herein, such as RX1, 5H4, MC1, or MC3 antibody, have affinity for M-CSF and are characterized by a dissociation equilibrium constant (Kd) of at least 10⁻⁴ M, preferably at least about 10⁻⁷ M to about 10⁻⁸ M, more preferably at least about 10⁻⁹ M, 10⁻¹⁰M, 10⁻¹¹M or 10⁻¹²M. Such affinities may be readily determined using conventional techniques, such as by equilibrium dialysis; by using the BIAcore 2000 instrument, using general procedures outlined by the manufacturer; by radioimmunoassay using ¹²⁵I labeled M-CSF; or by another method known to the skilled artisan. The affinity data may be analyzed, for example, by the method of Scatchard et al., Ann N.Y. Acad. Sci., 51:660 (1949). Thus, it will be apparent that preferred M-CSF antibodies will exhibit a high degree of specificity for M-CSF and will bind with substantially lower affinity to other molecules. Preferred antibodies bind M-CSF with a similar affinity as murine RX1 of Figure 3 binds to M-CSF, exhibit low immunogenicity, and inhibit macrophage-associated diseases when tested in animal models. Other exemplary antibodies bind M-CSF with a similar affinity as murine 5H4, MC1 or MC3 of Figure 8, 9 or 10, respectively, binds to M-CSF.

The antigen to be used for production of antibodies may be, e.g., intact M-CSF or a fragment of M-CSF that retains the desired epitope, optionally fused to another polypeptide that allows the epitope to be displayed in its native conformation. Alternatively, cells expressing M-CSF at their cell surface can be used to generate antibodies. Such cells can be transformed to express M-CSF or may be other naturally occurring cells that express M-CSF. Other forms of M-CSF useful for generating antibodies will be apparent to those skilled in the art.

### Polyclonal Antibodies

Polyclonal antibodies are preferably raised in animals by multiple subcutaneous (sc) or intraperitoneal (ip) injections of the relevant antigen and an adjuvant. An improved antibody response may be obtained by conjugating the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride or other agents known in the art.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to {fraction (1/10)} the original amount of peptide or conjugate in Freund's complete adjuvant by subcutaneous injection at multiple sites. At 7-14 days post-booster injection, the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### Monoclonal Antibodies

Monoclonal antibodies may be made using the hybridoma method first described by Kohler et al., Nature, 256:495 (1975), or may be made by recombinant DNA methods.

In the hybridoma method, a mouse or other appropriate host animal, such as a hamster or macaque monkey, is immunized as herein described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium. Human myeloma and mouse-human heteromyeloma cell lines also have been described for the production of human monoclonal antibodies (Kozbor, J. Immunol., 133: 3001 (1984) ;Brodeur et al., Monoclonal Antibody Production Techniques and Applications, pp. 51-63 (Marcel Dekker, Inc., New York, 1987)). Exemplary murine myeloma lines include those derived from MOP-21 and M.C.-11 mouse tumors available from the Salk Institute Cell Distribution Center, San Diego, Calif. USA, and SP-2 or X63-Ag8-653 cells available from the American Type Culture Collection, Rockville, Md. USA.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by Scatchard analysis (Munson et al., Anal. Biochem., 107:220 (1980)).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

### Recombinant Production of Antibodies

DNA encoding the monoclonal antibodies may be isolated and sequenced from the hybridoma cells using conventional procedures (e.g., by using oligonucleotide probes that are capable of binding specifically to genes encoding the heavy and light chains of the monoclonal antibodies). Sequence determination will generally require isolation of at least a portion of the gene or cDNA of interest. Usually this requires cloning the DNA or, preferably, mRNA (i.e., cDNA) encoding the monoclonal antibodies. Cloning is carried out using standard techniques (see, e.g., Sambrook et al. (1989) Molecular Cloning: A Laboratory Guide, Vols 1-3, Cold Spring Harbor Press). For example, a cDNA library may be constructed by reverse transcription of polyA+ mRNA, preferably membrane-associated mRNA, and the library screened using probes specific for human immunoglobulin polypeptide gene sequences. Preferably, however, the polymerase chain reaction (PCR) is used to amplify cDNAs (or portions of full-lenght cDNAs) encoding an immunoglobulin gene segment of interest (e.g., a light chain variable segment). The amplified sequences can be readily cloned into any suitable vector, e.g., expression vectors, minigene vectors, or phage display vectors. It will be appreciated that the particular method of cloning used not critical, so long as it is possible to determine the sequence of some portion of the immunoglobulin polypeptide of interest. As used herein, an "isolated" nucleic acid molecule or "isolated" nucleic acid sequence is a nucleic acid molecule that is either (1) identified and separated from at least one contaminant nucleic acid molecule with which it is ordinarily associated in the natural source of the nucleic acid or (2) cloned, amplified, tagged, or otherwise distinguished from background nucleic acids such that the sequence of the nucleic acid of interest can be determined, is considered isolated. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

One source for RNA used for cloning and sequencing is a hybridoma produced by obtaining a B cell from the transgenic mouse and fusing the B cell to an immortal cell. An advantage of using hybridomas is that they can be easily screened, and a hybridoma that produces a human monoclonal antibody of interest selected. Alternatively, RNA can be isolated from B cells (or whole spleen) of the immunized animal. When sources other than hybridomas are used, it may be desirable to screen for sequences encoding immunoglobulins or immunoglobulin polypeptides with specific binding characteristics. One method for such screening is the use of phage display technology. Phage display is described in e.g., Dower et al., WO 91/17271, McCafferty et al., WO 92/01047, and Caton and Koprowski, Proc. Natl. Acad. Sci. USA, 87:6450-6454 (1990). Using phage display technology, cDNA from an immunized transgenic mouse (e.g., total spleen cDNA) may be isolated, the polymerase chain reaction may be used to amplify a cDNA sequences that encode a portion of an immunoglobulin polypeptide, e.g., CDR regions, and the amplified sequences may be inserted into a phage vector. cDNAs encoding peptides of interest, e.g., variable region peptides with desired binding characteristics, are identified by standard techniques such as panning.

The sequence of the amplified or cloned nucleic acid is then determined. Typically the sequence encoding an entire variable region of the immunoglobulin polypeptide is determined, however, it will sometimes by adequate to sequence only a portion of a variable region, for example, the CDR-encoding portion. Typically the portion sequenced will be at least 30 bases in length, more often based coding for at least about one-third or at least about one-half of the length of the variable region will be sequenced.

Sequencing can be carried out on clones isolated from a cDNA library, or, when PCR is used, after subcloning the amplified sequence or by direct PCR sequencing of the amplified segment. Sequencing is carried out using standard techniques (see, e.g., Sambrook et al. (1989) Molecular Cloning: A Laboratory Guide, Vols 1-3, Cold Spring Harbor Press, and Sanger, F. et al. (1977) Proc. Natl. Acad. Sci. USA 74: 5463-5467). By comparing the sequence of the cloned nucleic acid with published sequences of human immunoglobulin genes and cDNAs, one of skill will readily be able to determine, depending on the region sequenced, (i) the germline segment usage of the hybridoma immunoglobulin polypeptide (including the isotype of the heavy chain) and (ii) the sequence of the heavy and light chain variable regions, including sequences resulting from N-region addition and the process of somatic mutation. One source of immunoglobulin gene sequence information is the National Center for Biotechnology Information, National Library of Medicine, National Institutes of Health, Bethesda, Md.

Once isolated, the DNA may be placed into expression vectors, which are then transfected into host cells such as *E. coli* cells, simian COS cells, human embryonic kidney 293 cells (e.g., 293E cells), Chinese hamster ovary (CHO) cells, or myeloma cells that do not otherwise produce immunoglobulin protein, to obtain the synthesis of monoclonal antibodies in the recombinant host cells. Recombinant production of antibodies is well known in the art.

Expression control sequences refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is operably linked when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, operably linked means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

Cell, cell line, and cell culture are often used interchangeably and all such designations herein include progeny. Transformants and transformed cells include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

The amino acid sequence of an immunoglobulin of interest may be determined by direct protein sequencing. Suitable encoding nucleotide sequences can be designed according to a universal codon table.

Amino acid sequence variants of the desired antibody may be prepared by introducing appropriate nucleotide changes into the encoding DNA, or by peptide synthesis. Such variants include, for example, deletions from, and/or insertions into and/or substitutions of, residues within the amino acid sequences of the antibodies. Any combination of deletion, insertion, and substitution is made to arrive at the final construct, provided that the final construct possesses the desired characteristics. The amino acid changes also may alter post-translational processes of the monoclonal, human, humanized, Human Engineered™ or variant antibody, such as changing the number or position of glycosylation sites.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Also disclosed herein are isolated nucleic acid encoding antibodies of the disclosure, optionally operably linked to control sequences recognized by a host cell, vectors and host cells comprising the nucleic acids, and recombinant techniques for the production of the antibodies, which may comprise culturing the host cell so that the nucleic acid is expressed and, optionally, recovering the antibody from the host cell culture or culture medium.

For recombinant production of the antibody, the nucleic acid encoding it is isolated and inserted into a replicable vector for further cloning (amplification of the DNA) or for expression. DNA encoding the monoclonal antibody is readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more selective marker genes, an enhancer element, a promoter, and a transcription termination sequence.

### (1) Signal sequence component

The antibody of the disclosure may be produced recombinantly not only directly, but also as a fusion polypeptide with a heterologous polypeptide, which is preferably a signal sequence or other polypeptide having a specific cleavage site at the N-terminus of the mature protein or polypeptide. The signal sequence selected preferably is one that is recognized and processed (i.e., cleaved by a signal peptidase) by the host cell. If prokaryotic host cells do not recognize and process the native antibody signal sequence, the signal sequence may be substituted by a signal sequence selected, for example, from the group of the pectate lyase (e.g., pelB) alkaline phosphatase, penicillinase, lpp, or heat-stable enterotoxin II leaders. For yeast secretion the native signal sequence may be substituted by, *e.g.,* the yeast invertase leader, α factor leader (including Saccharomyces and Kluyveromyces α-factor leaders), or acid phosphatase leader, the C. albicans glucoamylase leader, or the signal described in WO90/13646. In mammalian cell expression, mammalian signal sequences as well as viral secretory leaders, for example, the herpes simplex gD signal, are available.

The DNA for such precursor region is ligated in reading frame to DNA encoding the antibody.

### (2) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2 µ plasmid origin is suitable for yeast, and various viral origins are useful for cloning vectors in mammalian cells. Generally, the origin of replication component is not needed for mammalian expression vectors (the SV40 origin may typically be used only because it contains the early promoter).

### (3) Selective marker component

Expression and cloning vectors may contain a selective gene, also termed a selectable marker. Typical selection genes encode proteins that (a) confer resistance to antibiotics or other toxins, e.g., ampicillin, neomycin, methotrexate, tetracycline, G418, geneticin, histidinol, or mycophenolic acid (b) complement auxotrophic deficiencies, or (c) supply critical nutrients not available from complex media, e.g., the gene encoding D-alanine racemase for Bacilli.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs methotrexate, neomycin, histidinol, puromycin, mycophenolic acid and hygromycin.

Another example of suitable selectable markers for mammalian cells are those that enable the identification of cells competent to take up the antibody-encoding nucleic acid, such as DHFR, thymidine kinase, metallothionein-I and -II, preferably primate metallothionein genes, adenosine deaminase, ornithine decarboxylase, etc.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding antibody of the disclosure, wild-type DHFR protein, and another selectable marker such as aminoglycoside 3'-phosphotransferase (APH) can be selected by cell growth in medium containing a selection agent for the selectable marker such as an aminoglycosidic antibiotic, e.g., kanamycin, neomycin, or G418. *See* U.S. Patent No. 4,965,199.

A suitable selection gene for use in yeast is the trp1 gene present in the yeast plasmid YRp7 (Stinchcomb et al., Nature, 282: 39 (1979)). The trp1 gene provides a selection marker for a mutant strain of yeast lacking the ability to grow in tryptophan, for example, ATCC No. 44076 or PEP4-1. Jones, Genetics, 85: 12 (1977). The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene. *Ura*3-deficient yeast strains are complemented by plasmids bearing the *ura*3 gene.

In addition, vectors derived from the 1.6 µm circular plasmid pKD1 can be used for transformation of Kluyveromyces yeasts. Alternatively, an expression system for large-scale production of recombinant calf chymosin was reported for K. lactis. Van den Berg, Bio/Technology, 8: 135 (1990). Stable multi-copy expression vectors for secretion of mature recombinant human serum albumin by industrial strains of Kluyveromyces have also been disclosed. Fleer et al, Bio/Technology, 9: 968-975 (1991).

### (4) Promoter component

Expression and cloning vectors usually contain a promoter that is recognized by the host organism and is operably linked to the antibody-encoding nucleic acid. Promoters suitable for use with prokaryotic hosts include the arabinose (e.g., araB) promoter phoA promoter, β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the antibody of the disclosure.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Antibody transcription from vectors in mammalian host cells is controlled, for example, by promoters obtained from the genomes of viruses such as Abelson leukemia virus, polyoma virus, fowlpox virus, adenovirus (such as Adenovirus 2), bovine papilloma virus, avian sarcoma virus, most preferably cytomegalovirus, a retrovirus, hepatitis-B virus, Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. *See* also Reyes et al., Nature 297: 598-601 (1982) on expression of human β-interferon cDNA in mouse cells under the control of a thymidine kinase promoter from herpes simplex virus. Alternatively, the rous sarcoma virus long terminal repeat can be used as the promoter.

### (5) Enhancer element component

Transcription of a DNA encoding the antibody by higher eukaryotes is often increased by inserting an enhancer sequence into the vector. Many enhancer sequences are now known from mammalian genes (globin, elastase, albumin, alpha-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. *See* also Yaniv, Nature 297: 17-18 (1982) on enhancing elements for activation of eukaryotic promoters. The enhancer may be spliced into the vector at a position 5' or 3' to the antibody-encoding sequence, but is preferably located at a site 5' from the promoter.

### (6) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding antibody. One useful transcription termination component is the bovine growth hormone polyadenylation region. *See* WO94/11026 and the expression vector disclosed therein. Another is the mouse immunoglobulin light chain transcription terminator.

### (7) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, *Enterobacteriaceae* such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis (e.g., B. licheniformis* 41 P disclosed in DD 266,710 published Apr. 12, 1989), *Pseudomonas* such as *P. aeruginosa,* and *Streptomyces.* One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e.g., K. lactis, K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906), *K. thermotolerans, and K. marxianus; yarrowia* (EP 402,226); *Pichia pastors* (EP 183,070); *Candida; Trichoderma reesia* (EP 244,234); *Neurospora crassa; Schwanniomyces* such as *Schwanniomyces occidentalis;* and filamentous fungi such as, *e.g., Neurospora, Penicillium, Tolypocladium,* and *Aspergillus* hosts such as *A. nidulans* and *A. niger.*

Suitable host cells for the expression of glycosylated antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present disclosure, particularly for transfection of Spodoptera frugiperda cells.

Plant cell cultures of cotton, corn, potato, soybean, petunia, tomato, tobacco, lemna, and other plant cells can also be utilized as hosts.

However, interest has been greatest in vertebrate cells, and propagation of vertebrate cells in culture (tissue culture) has become routine procedure. Examples of useful mammalian host cell lines are Chinese hamster ovary cells, including CHOK1 cells (ATCC CCL61), DXB-11, DG-44, and Chinese hamster ovary cells/-DHFR (CHO, Urlaub et al., Proc. Natl. Acad. Sci. USA 77: 4216 (1980)); monkey kidney CV1 line transformed by SV40 (COS-7, ATCC CRL 1651); human embryonic kidney line (293 or 293 cells subcloned for growth in suspension culture, [Graham et al., J. Gen Virol. 36: 59 (1977)]; baby hamster kidney cells (BHK, ATCC CCL 10); mouse sertoli cells (TM4, Mather, Biol. Reprod. 23: 243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y Acad. Sci. 383: 44-68 (1982)); MRC 5 cells; FS4 cells; and a human hepatoma line (Hep G2).

Host cells are transformed or transfected with the above-described expression or cloning vectors for antibody production and cultured in conventional nutrient media modified as appropriate for inducing promoters, selecting transformants, or amplifying the genes encoding the desired sequences. In addition, novel vectors and transfected cell lines with multiple copies of transcription units separated by a selective marker are particularly useful and preferred for the expression of antibodies that target M-CSF.

### (8) Culturing the host cells

The host cells used to produce the antibody may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58: 44 (1979), Barnes et al., Anal. Biochem. 102: 255 (1980), U.S. Patent Nos. 4,767,704; 4,657,866; 4,927,762; 4,560,655; or 5,122,469; WO90103430; WO 87/00195; or U.S. Patent Re. No. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as Gentamycin™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (9) Purification of antibody

When using recombinant techniques, the antibody can be produced intracellularly, in the periplasmic space, or directly secreted into the medium, including from microbial cultures. If the antibody is produced intracellularly, as a first step, the particulate debris, either host cells or lysed fragments, is removed, for example, by centrifugation or ultrafiltration. Better et al. Science 240: 1041-1043 (1988); ICSU Short Reports 10: 105 (1990); and Proc. Natl. Acad. Sci. USA 90: 457-461 (1993) describe a procedure for isolating antibodies which are secreted to the periplasmic space of *E*. *coli. (See* also, [Carter et al., Bio/Technology 10: 163-167 (1992)].

The antibody composition prepared from microbial or mammalian cells can be purified using, for example, hydroxylapatite chromatography cation or avian exchange chromatography, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62: 1-13 (1983)). Protein G is recommended for all mouse isotypes and for human γ3 (Guss et al., EMBO J. 5: 15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H} 3 domain, the Bakerbond ABX™resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

### Chimeric and humanized antibodies

Because chimeric or humanized antibodies are less immunogenic in humans than the parental mouse monoclonal antibodies, they can be used for the treatment of humans with far less risk of anaphylaxis. Thus, these antibodies may be preferred in therapeutic applications that involve in vivo administration to a human.

Chimeric monoclonal antibodies, in which the variable Ig domains of a mouse monoclonal antibody are fused to human constant Ig domains, can be generated using standard procedures known in the art (See Morrison, S. L., et al. (1984) Chimeric Human Antibody Molecules; Mouse Antigen Binding Domains with Human Constant Region Domains, Proc. Natl. Acad. Sci. USA 81, 6841-6855; and, Boulianne, G. L., et al, Nature 312, 643-646. (1984)). Although some chimeric monoclonal antibodies have proved less immunogenic in humans, the mouse variable Ig domains can still lead to a significant human anti-mouse response.

Humanized antibodies may be achieved by a variety of methods including, for example: (1) grafting the non-human complementarity determining regions (CDRs) onto a human framework and constant region (a process referred to in the art as humanizing through "CDR grafting"), or, alternatively, (2) transplanting the entire non-human variable domains, but "cloaking" them with a human-like surface by replacement of surface residues (a process referred to in the art as "veneering"). In the present disclosure, humanized antibodies will include both "humanized" and "veneered" antibodies. These methods are disclosed in, e.g., Jones et al., Nature 321:522 525 (1986); Morrison et al., Proc. Natl. Acad. Sci., U.S.A., 81:6851 6855 (1984); Morrison and Oi, Adv. Immunol., 44:65 92 (1988); Verhoeyer et al., Science 239:1534 1536 (1988); Padlan, Molec. Immun. 28:489 498 (1991); Padlan, Molec. Immunol. 31(3):169 217 (1994); and Kettleborough, C.A. et al., Protein Eng. 4(7):773 83 (1991).

In particular, a rodent antibody on repeated in vivo administration in man either alone or as a conjugate will bring about an immune response in the recipient against the rodent antibody; the so-called HAMA response (Human Anti Mouse Antibody). The HAMA response may limit the effectiveness of the pharmaceutical if repeated dosing is required. The immunogenicity of the antibody may be reduced by chemical modification of the antibody with a hydrophilic polymer such as polyethylene glycol or by using the methods of genetic engineering to make the antibody binding structure more human like. For example, the gene sequences for the variable domains of the rodent antibody which bind CEA can be substituted for the variable domains of a human myeloma protein, thus producing a recombinant chimaeric antibody. These procedures are detailed in EP 194276, EP 0120694, EP 0125023, EP 0171496, EP 0173494 and WO 86/01533. Alternatively the gene sequences of the CDRs of the rodent antibody may be isolated or synthesized and substituted for the corresponding sequence regions of a homologous human antibody gene, producing a human antibody with the specificity of the original rodent antibody. These procedures are described in EP 023940, WO 90/07861 and WO91/09967. Alternatively a large number of the surface residues of the variable domain of the rodent antibody may be changed to those residues normally found on a homologous human antibody, producing a rodent antibody which has a surface 'veneer' of residues and which will therefore be recognized as self by the human body. This approach has been demonstrated by Padlan et.al. (1991) Mol. Immunol. 28, 489.

CDR grafting involves introducing one or more of the six CDRs from the mouse heavy and light chain variable Ig domains into the appropriate four framework regions of human variable Ig domains is also called CDR grafting. This technique (Riechmann, L., et al., Nature 332, 323 (1988)), utilizes the conserved framework regions (FR1-FR4) as a scaffold to support the CDR loops which are the primary contacts with antigen. A disadvantage of CDR grafting, however, is that it can result in a humanized antibody that has a substantially lower binding affinity than the original mouse antibody, because amino acids of the framework regions can contribute to antigen binding, and because amino acids of the CDR loops can influence the association of the two variable Ig domains. To maintain the affinity of the humanized monoclonal antibody, the CDR grafting technique can be improved by choosing human framework regions that most closely resemble the framework regions of the original mouse antibody, and by site-directed mutagenesis of single amino acids within the framework or CDRs aided by computer modeling of the antigen binding site (e.g., Co, M. S., et al. (1994), J. Immunol. 152, 2968-2976).

One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region which disturb the structure of the CDRs until the predicted conformation of the humanized sequence model closely approximates the conformation of the non-human CDRs of the parent non-human antibody. Such humanized antibodies may be further derivatized to facilitate uptake and clearance, e.g., via Ashwell receptors (See, e.g., U.S. Patent Nos. 5,530,101 and 5,585,089).

A number of humanizations of mouse monoclonal antibodies by rational design have been reported (See, for example, US 20020091240 published July 11, 2002, WO 92/11018 and U.S. Patent No., 5,693,762, U.S. Patent No. 5,766,866.

### Amino acid sequence variants

A useful method for identification of certain residues or regions of the antibody that are preferred locations for mutagenesis is called "alanine scanning mutagenesis," as described by Cunningham and Wells Science, 244:1081-1085 (1989). Here, a residue or group of target residues are identified (e.g., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intra-sequence insertions of single or multiple amino acid residues. Examples of terminal insertions include an antibody with an N-terminal methionyl residue or the antibody (including antibody fragment) fused to an epitope tag or a salvage receptor epitope. Other insertional variants of the antibody molecule include the fusion to a polypeptide which increases the serum half-life of the antibody, e.g. at the N-terminus or C-terminus.

The term "epitope tagged" refers to the antibody fused to an epitope tag. The epitope tag polypeptide has enough residues to provide an epitope against which an antibody there against can be made, yet is short enough such that it does not interfere with activity of the antibody. The epitope tag preferably is sufficiently unique so that the antibody there against does not substantially cross-react with other epitopes. Suitable tag polypeptides generally have at least 6 amino acid residues and usually between about 8-50 amino acid residues (preferably between about 9-30 residues). Examples include the flu HA tag polypeptide and its antibody 12CA5 [Field et al., Mol. Cell. Biol. 8: 2159-2165 (1988)]; the c-myc tag and the 8F9, 3C7, 6E10, G4, B7 and 9E10 antibodies thereto [Evan et al., Mol. Cell. Biol. 5(12): 3610-3616 (1985)]; and the Herpes Simplex virus glycoprotein D (gD) tag and its antibody [Paborsky et al., Protein Engineering 3(6): 547-553 (1990)]. Other exemplary tags are a poly-histidine sequence, generally around six histidine residues, that permits isolation of a compound so labeled using nickel chelation. Other labels and tags, such as the FLAG® tag (Eastman Kodak, Rochester, NY), well known and routinely used in the art, are disclosed herein.

As used herein, the term "salvage receptor binding epitope" refers to an epitope of the Fc region of an IgG molecule (e.g., IgG₁, IgG₂, IgG₃, or IgG₄) that is responsible for increasing the in vivo serum half-life of the IgG molecule.

Another type of variant is an amino acid substitution variant. These variants have at least one amino acid residue in the antibody molecule removed and a different residue inserted in its place. Substitutional mutagenesis within any of the hypervariable or CDR regions or framework regions is contemplated. Conservative substitutions are shown in Table 1. The most conservative substitution is found under the heading of "preferred substitutions". If such substitutions result in no change in biological activity, then more substantial changes, denominated "exemplary substitutions" in Table 1, or as further described below in reference to amino acid classes, may be introduced and the products screened.

**TABLE 1**

| **Original** | **Exemplary** | **Preferred Residue Substitutions** |
|---|---|---|
| Ala (A) | val; leu; ile | val |
| Arg (R) | lys; gln; asn | lys |
| Asn (N) | gln; his; asp, lys; gln | arg |
| Asp (D) | glu; asn | glu |
| Cys (C) | ser; ala | ser |
| Gln (Q) | asn; glu | asn |
| Glu (E) | asp; gln | asp |
| Gly (G) | ala | |
| His (H) | asn; gln; lys; arg | |
| Ile (I) | leu; val; met; ala; phe; | leu norleucine |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | |
| Pro (P) | ala | |
| Ser (S) | thr | |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Conservative substitutions involve replacing an amino acid with another member of its class. Non-conservative substitutions involve replacing a member of one of these classes with a member of another class.

Any cysteine residue not involved in maintaining the proper conformation of the monoclonal, human, humanized, Human Engineered™ or variant antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

Affinity maturation involves preparing and screening antibody variants that have substitutions within the CDRs of a parent antibody and selecting variants that have improved biological properties such as binding affinity relative to the parent antibody. A convenient way for generating such substitutional variants is affinity maturation using phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity).

Alanine scanning mutagenesis can be performed to identify hypervariable region residues that contribute significantly to antigen binding. Alternatively, or in addition, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Antibody variants can also be produced that have a modified glycosylation pattern relative to the parent antibody, for example, deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. The presence of either of these tripeptide sequences in a polypeptide creates a potential glycosylation site. Thus, N-linked glycosylation sites may be added to an antibody by altering the amino acid sequence such that it contains one or more of these tripeptide sequences. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. O-linked glycosylation sites may be added to an antibody by inserting or substituting one or more serine or threonine residues to the sequence of the original antibody. By way of example, the amino acids of RX1 at positions 41-43 of Figure 3A (NGS) may be retained. Alternatively, only amino acids 41 and 42 (NG) may be retained.

Ordinarily, amino acid sequence variants of the Human Engineered™ antibody will have an amino acid sequence having at least 60% amino acid sequence identity with the original Human Engineered™ antibody amino acid sequences of either the heavy or the light chain *(e.g.,* as in any of Figures 13B through 16B) more preferably at least 80%, more preferably at least 85%, more preferably at least 90%, and most preferably at least 95%, including for example, 80%, 81%, 82%, 83%, 84%, 85%, 86%, 87%, 88%, 89%, 90%, 91%, 92%, 93%, 94%, 95%, 96%, 97%, 98%, 99%, and 100%. Identity or homology with respect to this sequence is defined herein as the percentage of amino acid residues in the candidate sequence that are identical with the Human Engineered™ residues, after aligning the sequences and introducing gaps, if necessary, to achieve the maximum percent sequence identity, and not considering any conservative substitutions (as defined in Table 1 above) as part of the sequence identity. None of N-terminal, C-terminal, or internal extensions, deletions, or insertions into the antibody sequence shall be construed as affecting sequence identity or homology. Thus, sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences, or along a pre-determined portion of one or both sequences). The programs provide a default opening penalty and a default gap penalty, and a scoring matrix such as PAM 250 [a standard scoring matrix; see Dayhoff et al., in Atlas of Protein Sequence and Structure, vol. 5, supp. 3 (1978)] can be used in conjunction with the computer program. For example, the percent identity can then be calculated as: the total number of identical matches multiplied by 100 and then divided by the sum of the length of the longer sequence within the matched span and the number of gaps introduced into the longer sequences in order to align the two sequences.

Other modifications of the antibody are contemplated. For example, it may be desirable to modify the antibody with respect to effector function, so as to enhance the effectiveness of the antibody in treating macrophage-associated diseases, for example. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See* Caron et al., J. Exp Med. 176: 1191-1195 (1992) and Shopes, B. J. Immunol. 148: 2918-2922 (1992). Homodimeric antibodies with enhanced activity may also be prepared using heterobifunctional cross-linkers as described in Wolff et al., Cancer Research 53: 2560-2565 (1993). Alternatively, an antibody can be engineered which has dual Fc regions and may thereby have enhanced complement lysis and ADCC capabilities. *See* Stevenson et al., Anti-Cancer Drug Design 3: 219-230 (1989). In addition, it has been shown that sequences within the CDR can cause an antibody to bind to MHC Class II and trigger an unwanted helper T-cell response. A conservative substitution can allow the antibody to retain binding activity yet lose its ability to trigger an unwanted T-cell response. Also see Steplewski et al., Proc Natl Acad Sci USA. 1988;85(13):4852-6, which described chimeric antibodies wherein a murine variable region was joined with human gamma 1, gamma 2, gamma 3, and gamma 4 constant regions.

It may be desirable to use an antibody fragment, rather than an intact antibody, to increase tissue penetration, for example. In this case, it may be desirable to modify the antibody fragment in order to increase its serum half-life, for example, adding molecules such as PEG or other water soluble polymers, including polysaccharide polymers, to antibody fragments to increase the half-life. This may also be achieved, for example, by incorporation of a salvage receptor binding epitope into the antibody fragment (e.g., by mutation of the appropriate region in the antibody fragment or by incorporating the epitope into a peptide tag that is then fused to the antibody fragment at either end or in the middle, *e.g.,* by DNA or peptide synthesis) *(see, e.g.,* WO96/32478).

The salvage receptor binding epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (e.g., of an IgG) and transferred to the CH1, CH3, or VH region, or more than one such region, of the antibody. Alternatively, the epitope is taken from the CH2 domain of the Fc region and transferred to the C.sub.L region or V.sub.L region, or both, of the antibody fragment. See also International applications WO 97/34631 and WO 96/32478 which describe Fc variants and their interaction with the salvage receptor.

Thus, antibodies disclosed herein may comprise a human Fc portion, a human consensus Fc portion, or a variant thereof that retains the ability to interact with the Fc salvage receptor, including variants in which cysteines involved in disulfide bonding are modified or removed, and/or in which the a met is added at the N-terminus and/or one or more of the N-terminal 20 amino acids are removed, and/or regions that interact with complement, such as the C1q binding site, are removed, and/or the ADCC site is removed [see, e.g., Molec. Immunol. 29 (5): 633-9 (1992)].

Previous studies mapped the binding site on human and murine IgG for FcR primarily to the lower hinge region composed of IgG residues 233-239. Other studies proposed additional broad segments, e.g. Gly316-Lys338 for human Fc receptor I, Lys274-Arg301 and Tyr407-Arg416 for human Fc receptor III, or found a few specific residues outside the lower hinge, e.g. Asn297 and Glu318 for murine IgG2b interacting with murine Fc receptor II. The report of the 3.2-Å crystal structure of the human IgG1 Fc fragment with human Fc receptor IIIA delineated IgG1 residues Leu234-Ser239, Asp265-Glu269, Asn297-Thr299, and Ala327-Ile332 as involved in binding to Fc receptor IIIA. It has been suggested based on crystal structure that in addition to the lower hinge (Leu234-Gly237), residues in IgG CH2 domain loops FG (residues 326-330) and BC (residues 265-271) might play a role in binding to Fc receptor IIA. See Shields et al., J. Biol. Chem., 276(9):6591-6604 (2001). Mutation of residues within Fc receptor binding sites can result in altered effector function, such as altered ADCC or CDC activity, or altered half-life. As described above, potential mutations include insertion, deletion or substitution of one or more residues, including substitution with alanine, a conservative substitution, a non-conservative substitution, or replacement with a corresponding amino acid residue at the same position from a different IgG subclass (e.g. replacing an IgG1 residue with a corresponding IgG2 residue at that position).

Shields et al. reported that IgG1 residues involved in binding to all human Fc receptors are located in the CH2 domain proximal to the hinge and fall into two categories as follows: 1) positions that may interact directly with all FcR include Leu234-Pro238, Ala327, and Pro329 (and possibly Asp265); 2) positions that influence carbohydrate nature or position include Asp265 and Asn297. The additional IgG1 residues that affected binding to Fc receptor II are as follows: (largest effect) Arg255, Thr256, Glu258, Ser267, Asp270, Glu272, Asp280, Arg292, Ser298, and (less effect) His268, Asn276, His285, Asn286, Lys290, Gln295, Arg301, Thr307, Leu309, Asn315, Lys322, Lys326, Pro331, Ser337, Ala339, Ala378, and Lys414. A327Q, A327S, P329A, D265A and D270A reduced binding. In addition to the residues identified above for all FcR, additional IgG1 residues that reduced binding to Fc receptor IIIA by 40% or more are as follows: Ser239, Ser267 (Gly only), His268, Glu293, Gln295, Tyr296, Arg301, Val303, Lys338, and Asp376. Variants that improved binding to FcRIIIA include T256A, K290A, S298A, E333A, K334A, and A339T. Lys414 showed a 40% reduction in binding for FcRIIA and FcRIIB, Arg416 a 30% reduction for FcRIIA and FcRIIIA, Gln419 a 30% reduction to FcRIIA and a 40% reduction to FcRIIB, and Lys360 a 23% improvement to FcRIIIA. See also Presta et al., Biochem. Soc. Trans. (2001) 30, 487-190.

For example, United States Patent No. 6,194,551 describes variants with altered effector function containing mutations in the human IgG Fc region, at amino acid position 329, 331 or 322 (using Kabat numbering), some of which display reduced C1q binding or CDC activity. As another example, United States Patent No. 6,737,056 describes variants with altered effector or Fc-gamma-receptor binding containing mutations in the human IgG Fc region, at amino acid position 238, 239, 248, 249, 252, 254, 255, 256, 258, 265, 267, 268, 269, 270, 272, 276, 278, 280, 283, 285, 286, 289, 290, 292, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439 (using Kabat numbering), some of which display receptor binding profiles associated with reduced ADCC or CDC activity. Of these, a mutation at amino acid position 238, 265, 269, 270, 327 or 329 are stated to reduce binding to FcRI, a mutation at amino acid position 238, 265, 269,270, 292, 294, 295, 298, 303, 324, 327, 329, 333, 335, 338, 373, 376, 414, 416, 419, 435, 438 or 439 are stated to reduce binding to FcRII, and a mutation at amino acid position 238, 239, 248, 249, 252, 254, 265, 268, 269, 270, 272, 278, 289, 293, 294, 295, 296, 301, 303, 322, 327, 329, 338, 340, 373, 376, 382, 388, 389, 416, 434, 435 or 437 is stated to reduce binding to FcRIII.

United States Patent No. 5,624,821 reports that Clq binding activity of an murine antibody can be altered by mutating amino acid residue 318, 320 or 322 of the heavy chain and that replacing residue 297 (Asn) results in removal of lytic activity.

United States Application Publication No. 20040132101, describes variants with mutations at amino acid positions 240, 244, 245, 247, 262, 263, 266, 299, 313, 325, 328, or 332 (using Kabat numbering) or positions 234, 235, 239, 240, 241, 243, 244, 245, 247, 262, 263, 264, 265, 266, 267, 269, 296, 297, 298, 299, 313, 325, 327, 328, 329, 330, or 332 (using Kabat numbering), of which mutations at positions 234, 235, 239, 240, 241, 243, 244, 245, 247, 262, 263, 264, 265, 266, 267, 269, 296, 297, 298, 299, 313, 325, 327, 328, 329, 330, or 332 may reduce ADCC activity or reduce binding to an Fc gamma receptor.

Chappel et al., Proc Natl Acad Sci USA. 1991;88(20):9036-40 report that cytophilic activity of IgG1 is an intrinsic property of its heavy chain CH2 domain. Single point mutations at any of amino acid residues 234-237 of IgG1 significantly lowered or abolished its activity. Substitution of all of IgG1 residues 234-237 (LLGG) into IgG2 and IgG4 were required to restore full binding activity. An IgG2 antibody containing the entire ELLGGP sequence (residues 233-238) was observed to be more active than wild-type IgG1.

Isaacs et al., J Immunol. 1998;161(8):3862-9 report that mutations within a motif critical for Fc gammaR binding (glutamate 233 to proline, leucine/phenylalanine 234 to valine, and leucine 235 to alanine) completely prevented depletion of target cells. The mutation glutamate 318 to alanine eliminated effector function of mouse IgG2b and also reduced the potency of human IgG4.

Armour et al., Mol Immunol. 2003;40(9):585-93 identified IgG1 variants which react with the activating receptor, FcgammaRIIa, at least 10-fold less efficiently than wildtype IgG1 but whose binding to the inhibitory receptor, FcgammaRIIb, is only four-fold reduced. Mutations were made in the region of amino acids 233-236 and/or at amino acid positions 327, 330 and 331. See also WO 99/58572.

Xu et al., J Biol Chem. 1994;269(5):3469-74 report that mutating IgG1 Pro331 to Ser markedly decreased C1q binding and virually eliminated lytic activity. In contrast, the substitution of Pro for Ser331 in IgG4 bestowed partial lytic activity (40%) to the IgG4 Pro331 variant.

Schuurman et al,. Mol Immunol. 2001;38(1):1-8report that mutating one of the hinge cysteines involved in the inter-heavy chain bond formation, Cys226, to serine resulted in a more stable inter-heavy chain linkage. Mutating the IgG4 hinge sequence Cys-Pro-Ser-Cys to the IgG1 hinge sequence Cys-Pro-Pro-Cys also markedly stabilizes the covalent interaction between the heavy chains.

Angal et al., Mol Immunol. 1993;30(1):105-8 report that mutating the serine at amino acid position 241 in IgG4 to proline (found at that position in IgG1 and IgG2) led to the production of a homogeneous antibody, as well as extending serum half-life and improving tissue distribution compared to the original chimeric IgG4.

### Human and Human Engineered™ antibodies

### Human Engineering™

Human Engineering™ of antibody variable domains has been described by Studnicka [See, e.g., Studnicka et al. U.S. Patent No. 5,766,886; Studnicka et al. Protein Engineering 7: 805-814 (1994)] as a method for reducing immunogenicity while maintaining binding activity of antibody molecules. According to the method, each variable region amino acid has been assigned a risk of substitution. Amino acid substitutions are distinguished by one of three risk categories : (1) low risk changes are those that have the greatest potential for reducing immunogenicity with the least chance of disrupting antigen binding; (2) moderate risk changes are those that would further reduce immunogenicity, but have a greater chance of affecting antigen binding or protein folding; (3) high risk residues are those that are important for binding or for maintaining antibody structure and carry the highest risk that antigen binding or protein folding will be affected. Due to the three-dimensional structural role of prolines, modifications at prolines are generally considered to be at least moderate risk changes, even if the position is typically a low risk position.

Variable regions of the light and heavy chains of a rodent antibody are Human Engineered™ as follows to substitute human amino acids at positions determined to be unlikely to adversely effect either antigen binding or protein folding, but likely to reduce immunogenicity in a human environment. Amino acid residues that are at "low risk" positions and that are candidates for modification according to the method are identified by aligning the amino acid sequences of the rodent variable regions with a human variable region sequence. Any human variable region can be used, including an individual VH or VL sequence or a human consensus VH or VL sequence or an individual or consensus human germline sequence. The amino acid residues at any number of the low risk positions, or at all of the low risk positions, can be changed. For example, at each low risk position where the aligned murine and human amino acid residues differ, an amino acid modification is introduced that replaces the rodent residue with the human residue. Alternatively, the amino acid residues at all of the low risk positions and at any number of the moderate risk positions can be changed. Ideally, to achieve the least immunogenicity all of the low and moderate risk positions are changed from rodent to human sequence.

Synthetic genes containing modified heavy and/or light chain variable regions are constructed and linked to human γ heavy chain and/or kappa light chain constant regions. Any human heavy chain and light chain constant regions may be used in combination with the Human Engineered™ antibody variable regions, including IgA (of any subclass, such as IgA1 or IgA2), IgD, IgE, IgG (of any subclass, such as IgG1, IgG2, IgG3, or IgG4), or IgM. The human heavy and light chain genes are introduced into host cells, such as mammalian cells, and the resultant recombinant immunoglobulin products are obtained and characterized.

### Human antibodies from transgenic animals

Human antibodies to M-CSF can also be produced using transgenic animals that have no endogenous immunoglobulin production and are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/741 also discloses transgenic non-primate mammalian hosts capable of mounting an immune response to an immunogen, wherein the antibodies have primate constant and/or variable regions, and wherein the endogenous immunoglobulin encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions.

Using a transgenic animal described above, an immune response can be produced to a selected antigenic molecule, and antibody producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. This publication discloses monoclonal antibodies against a variety of antigenic molecules including IL 6, IL 8, TNFa, human CD4, L selectin, gp39, and tetanus toxin. The monoclonal antibodies can be tested for the ability to inhibit or neutralize the biological activity or physiological effect of the corresponding protein. WO 96/33735 discloses that monoclonal antibodies against IL-8, derived from immune cells of transgenic mice immunized with IL-8, blocked IL-8 induced functions of neutrophils. Human monoclonal antibodies with specificity for the antigen used to immunize transgenic animals are also disclosed in WO 96/34096 and U.S. patent application no. 20030194404; and U.S. patent application no. 20030031667).

See also Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Pat. No. 5,591,669, U.S. Patent No. 5,589,369, U.S. Patent No. 5,545,807; and U.S Patent Application No. 20020199213. U.S. Patent Application No. and 20030092125 describes methods for biasing the immune response of an animal to the desired epitope. Human antibodies may also be generated by *in vitro* activated B cells (see U.S. Pat. Nos. 5,567,610 and 5,229,275).

### Human antibodies from phage display technology

The development of technologies for making repertoires of recombinant human antibody genes, and the display of the encoded antibody fragments on the surface of filamentous bacteriophage, has provided a means for making human antibodies directly. The antibodies produced by phage technology are produced as antigen binding fragments-usually Fv or Fab fragments-in bacteria and thus lack effector functions. Effector functions can be introduced by one of two strategies: The fragments can be engineered either into complete antibodies for expression in mammalian cells, or into bispecific antibody fragments with a second binding site capable of triggering an effector function.

Typically, the Fd fragment (V_{H}-C_{H}1) and light chain (V_{L}-C_{L}) of antibodies are separately cloned by PCR and recombined randomly in combinatorial phage display libraries, which can then be selected for binding to a particular antigen. The Fab fragments are expressed on the phage surface, i.e., physically linked to the genes that encode them. Thus, selection of Fab by antigen binding co-selects for the Fab encoding sequences, which can be amplified subsequently. By several rounds of antigen binding and re-amplification, a procedure termed panning, Fab specific for the antigen are enriched and finally isolated.

In 1994, an approach for the humanization of antibodies, called "guided selection", was described. Guided selection utilizes the power of the phage display technique for the humanization of mouse monoclonal antibody (See Jespers, L. S., et al., Bio/Technology 12, 899-903 (1994)). For this, the Fd fragment of the mouse monoclonal antibody can be displayed in combination with a human light chain library, and the resulting hybrid Fab library may then be selected with antigen. The mouse Fd fragment thereby provides a template to guide the selection. Subsequently, the selected human light chains are combined with a human Fd fragment library. Selection of the resulting library yields entirely human Fab.

A variety of procedures have been described for deriving human antibodies from phage-display libraries (See, for example, Hoogenboom et al., J. Mol. Biol., 227:381 (1991); Marks et al., J. Mol. Biol, 222:581-597 (1991); U.S. Pat. Nos. 5,565,332 and 5,573,905; Clackson, T., and Wells, J. A., TIBTECH 12, 173-184 (1994)). In particular, in vitro selection and evolution of antibodies derived from phage display libraries has become a powerful tool (See Burton, D. R., and Barbas III, C. F., Adv. Immunol. 57, 191-280 (1994); and, Winter, G., et al., Annu. Rev. Immunol. 12, 433-455 (1994); U.S. patent application no. 20020004215 and WO92/01047; U.S. patent application no. 20030190317 published October 9, 2003 and U.S. Patent No. 6,054,287; U.S. Patent No. 5,877,293.

Watkins, "Screening of Phage-Expressed Antibody Libraries by Capture Lift," Methods in Molecular Biology, Antibody Phage Display: Methods and Protocols 178: 187-193, and U.S. patent application no. 20030044772 published March 6, 2003 describe methods for screening phage-expressed antibody libraries or other binding molecules by capture lift, a method involving immobilization of the candidate binding molecules on a solid support.

The antibody products may be screened for activity and for suitability in the treatment methods disclosed herein using assays as described in the section entitled "Screening Methods" herein or using any suitable assays known in the art.

### Other covalent modifications

Covalent modifications of the antibody are also included within the scope of thisdisclosure. They may be made by chemical synthesis or by enzymatic or chemical cleavage of the antibody, if applicable. Other types of covalent modifications of the antibody are introduced into the molecule by reacting targeted amino acid residues of the antibody with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, .alpha.-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino-terminal residues are reacted with succinic or other carboxylic acid anhydrides. Derivatization with these agents has the effect of reversing the charge of the lysinyl residues. Other suitable reagents for derivatizing .alpha.-amino-containing residues include imidoesters such as methyl picolinimidate, pyridoxal phosphate, pyridoxal, chloroborohydride, trinitrobenzenesulfonic acid, O-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay.

Carboxyl side groups (aspartyl or glutamyl) are selectively modified by reaction with carbodiimides (R-N.dbd.C.dbd.N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of thisdisclosure.

Other modifications include hydroxylation of proline and lysine, phosphorylation of hydroxyl groups of seryl or threonyl residues, methylation of the .alpha.-amino groups of lysine, arginine, and histidine side chains (T. E. Creighton, Proteins: Structure and Molecular Properties, W.H. Freeman & Co., San Francisco, pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO87/05330 published 11 Sep. 1987, and in Aplin and Wriston, CRC Crit. Rev. Biochem., pp. 259-306 (1981).

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Hakimuddin, et al. Arch. Biochem. Biophys. 259: 52 (1987) and by Edge et al. Anal. Biochem., 118: 131 (1981). Enzymatic cleavage of carbohydrate moieties on antibodies can be achieved by the use of a variety of endo- and exo-glycosidases as described by Thotakura et al. Meth. Enzymol. 138: 350 (1987).

Another type of covalent modification of the antibody comprises linking the antibody to one of a variety of nonproteinaceous polymers, e.g., polyethylene glycol, polypropylene glycol, polyoxyethylated polyols, polyoxyethylated sorbitol, polyoxyethylated glucose, polyoxyethylated glycerol, polyoxyalkylenes, or polysaccharide polymers such as dextran. Such methods are known in the art, see, e.g. U.S. Patent Nos. 4,640,835; 4,496,689; 4,301,144; 4,670,417; 4,791,192, 4,179,337, 4,766,106, 4,179,337, 4,495,285, 4,609,546or EP 315 456.

### Gene Therapy

Delivery of a therapeutic antibody to appropriate cells can be effected via gene therapy ex vivo, in situ, or in vivo by use of any suitable approach known in the art, including by use of physical DNA transfer methods (e.g., liposomes or chemical treatments) or by use of viral vectors (e.g., adenovirus, adeno-associated virus, or a retrovirus). For example, for in vivo therapy, a nucleic acid encoding the desired antibody, either alone or in conjunction with a vector, liposome, or precipitate may be injected directly into the subject, and may be injected at the site where the expression of the antibody compound is desired. For ex vivo treatment, the subject's cells are removed, the nucleic acid is introduced into these cells, and the modified cells are returned to the subject either directly or, for example, encapsulated within porous membranes which are implanted into the patient. See, e.g. U.S. Pat. Nos. 4,892,538 and 5,283,187. There are a variety of techniques available for introducing nucleic acids into viable cells. The techniques vary depending upon whether the nucleic acid is transferred into cultured cells in vitro, or in vivo in the cells of the intended host. Techniques suitable for the transfer of nucleic acid into mammalian cells in vitro include the use of liposomes, electroporation, microinjection, cell fusion, DEAE-dextran, and calcium phosphate precipitation. A commonly used vector for ex vivo delivery of a nucleic acid is a retrovirus.

Other in vivo nucleic acid transfer techniques include transfection with viral vectors (such as adenovirus, Herpes simplex I virus, or adeno-associated virus) and lipid-based systems. The nucleic acid and transfection agent are optionally associated with a microparticle. Exemplary transfection agents include calcium phosphate or calcium chloride co-precipitation, DEAE-dextran-mediated transfection, quaternary ammonium amphiphile DOTMA ((dioleoyloxypropyl) trimethylammonium bromide, commercialized as Lipofectin by GIBCO-BRL))(Felgner et al, (1987) Proc. Natl. Acad. Sci. USA 84, 7413-7417; Malone et al. (1989) Proc. Natl Acad. Sci. USA 86 6077-6081); lipophilic glutamate diesters with pendent trimethylammonium heads (Ito et al. (1990) Biochem. Biophys. Acta 1023, 124-132); the metabolizable parent lipids such as the cationic lipid dioctadecylamido glycylspermine (DOGS, Transfectam, Promega) and dipalmitoylphosphatidyl ethanolamylspermine (DPPES)(J. P. Behr (1986) Tetrahedron Lett. 27, 5861-5864; J. P. Behr et al. (1989) Proc. Natl. Acad. Sci. USA 86, 6982-6986); metabolizable quaternary ammonium salts (DOTB, N-(1-[2,3-dioleoyloxy]propyl)-N,N,N-trimethylammonium methylsulfate (DOTAP)(Boehringer Mannheim), polyethyleneimine (PEI), dioleoyl esters, ChoTB, ChoSC, DOSC)(Leventis et al. (1990) Biochim. Inter. 22, 235-241); 3beta[N-(N', N'-dimethylaminoethane)-carbamoyl]cholesterol (DC-Chol), dioleoylphosphatidyl ethanolamine (DOPE)/3beta[N-(N',N'-dimethylaminoethane)-carbamoyl]cholesterolDC-Chol in one to one mixtures (Gao et al., (1991) Biochim. Biophys. Acta 1065, 8-14), spermine, spermidine, lipopolyamines (Behr et al., Bioconjugate Chem, 1994, 5: 382-389), lipophilic polylysines (LPLL) (Zhou et al., (1991) Biochim. Biophys. Acta 939, 8-18), [[(1,1,3,3-tetramethylbutyl)cre- soxy]ethoxy]ethyl]dimethylbe nzylammonium hydroxide (DEBDA hydroxide) with excess phosphatidylcholine/cholesterol (Ballas et al., (1988) Biochim. Biophys. Acta 939, 8-18), cetyltrimethylammonium bromide (CTAB)/DOPE mixtures (Pinnaduwage et al, (1989) Biochim. Biophys. Acta 985, 33-37), lipophilic diester of glutamic acid (TMAG) with DOPE, CTAB, DEBDA, didodecylammonium bromide (DDAB), and stearylamine in admixture with phosphatidylethanolamine (Rose et al., (1991) Biotechnique 10, 520-525), DDAB/DOPE (TransfectACE, GIBCO BRL), and oligogalactose bearing lipids. Exemplary transfection enhancer agents that increase the efficiency of transfer include, for example, DEAE-dextran, polybrene, lysosome-disruptive peptide (Ohmori N I et al, Biochem Biophys Res Commun Jun. 27, 1997;235(3):726-9), chondroitan-based proteoglycans, sulfated proteoglycans, polyethylenimine, polylysine (Pollard H et al. J Biol Chem, 1998 273 (13):7507-11), integrin-binding peptide CYGGRGDTP, linear dextran nonasaccharide, glycerol, cholesteryl groups tethered at the 3'-terminal internucleoside link of an oligonucleotide (Letsinger, R. L. 1989 Proc Natl Acad Sci USA 86: (17):6553-6), lysophosphatide, lysophosphatidylcholine, lysophosphatidylethanolamine, and 1-oleoyl lysophosphatidylcho line.

In some situations it may be desirable to deliver the nucleic acid with an agent that directs the nucleic acid-containing vector to target cells. Such "targeting" molecules include antibodies specific for a cell-surface membrane protein on the target cell, or a ligand for a receptor on the target cell. Where liposomes are employed, proteins which bind to a cell-surface membrane protein associated with endocytosis may be used for targeting and/or to facilitate uptake. Examples of such proteins include capsid proteins and fragments thereof tropic for a particular cell type, antibodies for proteins which undergo internalization in cycling, and proteins that target intracellular localization and enhance intracellular half-life.Alternatively, receptor-mediated endocytosis may be used. Such methods are described, for example, in Wu et al., 1987 or Wagner et al., 1990. For review of the currently known gene marking and gene therapy protocols, see Anderson 1992. See also WO 93/25673 and the references cited therein. For additional reviews of gene therapy technology, see Friedmann, Science, 244: 1275-1281 (1989); Anderson, Nature, supplement to vol. 392, no 6679, pp. 25-30 (1998); Verma, Scientific American: 68-84 (1990); and Miller, Nature, 357: 455460 (1992).

### Screening Methods

Effective therapeutics depend on identifying efficacious agents devoid of significant toxicity. Antibodies may be screened for binding affinity by methods known in the art. For example, gel-shift assays, Western blots, radiolabeled competition assay, co-fractionation by chromatography, co-precipitation, cross linking, ELISA, and the like may be used, which are described in, for example, Current Protocols in Molecular Biology (1999) John Wiley & Sons, NY.

To initially screen for antibodies which bind to the desired epitope on M-CSF *(e.g.,* those which block binding of RX1, 5H4, MC1 and/or MC3 to M-CSF), a routine cross-blocking assay such as that described in Antibodies, A Laboratory Manual, Cold Spring Harbor Laboratory, Ed Harlow and David Lane (1988), can be performed. Routine competitive binding assays may also be used, in which the unknown antibody is characterized by its ability to inhibit binding of M-CSF to an M-CSF specific antibody of the disclosure. Intact M-CSF, fragments thereof, or linear epitopes such as represented by amino acids 98-105 of M-CSF of Figure 7, or amino acids 65-73 or 138-144 of Figure 7 (corresponding to M-CSF epitopes recognized by 5H4 or MC3), can be used. Epitope mapping is described in Champe et al., J. Biol. Chem. 270: 1388-1394 (1995).

It is further contemplated that the antibodies are next tested for their effect on M-CSF biological activity with respect to inducing production or proliferation of macrophages, followed by administration to animals. Compounds potentially useful in macrophage-associated diseases may be screened using various assays. For instance, a candidate antagonist may first be characterized in a cultured cell system to determine its ability to neutralize M-CSF biological activity. Such a system may include the co-culture of mouse stromal cell lines (e.g., MC3T3-G2/PA6 and ST2) and mouse spleen cells (Udagawa et al., Endocrinology 125: 1805 13, 1989), and the co-culture of ST2 cells and bone marrow cells, peripheral blood mononuclear cells or alveolar macrophages (Udagawa et al., Proc. Natl. Acad. Sci. USA 87: 7260 4, 1990; Sasaki et al., Cancer Res. 58: 462 7, 1998; Mancino et al., J. Surg. Res.100: 18-24, 2001). Efficacy of a given M-CSF antibody in reducing the effect of M-CSF on production or proliferation of macrophages, or in preventing or treating macrophage-associated diseases such as atherosclerotic diseases, or HIV infection and conditions associated therewith, may also be tested in any of the *in vitro* assays or animal model systems familiar to those skilled in the art. Such model systems are described in, for example, Bhattacharyya and Strong, Exp Mol Pathol., 74(3):291-7 (2003), Lewin et al., AIDS, 12(7):719-27 (1998), Dereuddre-Bosquet N et al., AIDS Res Hum Retroviruses, 13(11):961-6 (1997), Zuber et al., AIDS Res Hum Retroviruses, 17(7):631-5 (2001), and North et al., J Virol., 79(12):7349-54 (2005).

In one variation of an *in vitro* assay, disclosed herein is a method comprising the steps of (a) contacting an immobilized M-CSF with a candidate antibody and (b) detecting binding of the candidate antibody to the M-CSF. Alternatively, the candidate antibody may be immobilized and binding of M-CSF is detected. Immobilization is accomplished using any of the methods well known in the art, including covalent bonding to a support, a bead, or a chromatographic resin, as well as non-covalent, high affinity interaction such as antibody binding, or use of streptavidin/biotin binding wherein the immobilized compound includes a biotin moiety. Detection of binding can be accomplished (i) using a radioactive label on the compound that is not immobilized, (ii) using a fluorescent label on the non-immobilized compound, (iii) using an antibody immunospecific for the non-immobilized compound, (iv) using a label on the non-immobilized compound that excites a fluorescent support to which the immobilized compound is attached, as well as other techniques well known and routinely practiced in the art.

Antibodies that modulate (i.e., increase, decrease, or block) the activity or expression of M-CSF may be identified by incubating a putative modulator with a cell expressing a M-CSF and determining the effect of the putative modulator on the activity or expression of the M-CSF. The selectivity of an antibody that modulates the activity of a M-CSF polypeptide or polynucleotide can be evaluated by comparing its effects on the M-CSF polypeptide or polynucleotide to its effect on other related compounds. Selective modulators may include, for example, antibodies and other proteins, peptides, or organic molecules which specifically bind to M-CSF polypeptides or to a nucleic acid encoding a M-CSF polypeptide. Modulators of M-CSF activity will be therapeutically useful in treatment of diseases and physiological conditions in which normal or aberrant activity of M-CSF polypeptide is involved.

The disclosure also comprehends high throughput screening (HTS) assays to identify antibodies that interact with or inhibit biological activity (i.e., inhibit enzymatic activity, binding activity, etc.) of a M-CSF polypeptide. HTS assays permit screening of large numbers of compounds in an efficient manner. Cell-based HTS systems are contemplated to investigate the interaction between M-CSF polypeptides and their binding partners. HTS assays are designed to identify "hits" or "lead compounds" having the desired property, from which modifications can be designed to improve the desired property. Chemical modification of the "hit" or "lead compound" is often based on an identifiable structure/activity relationship between the "hit" and M-CSF polypeptides.

Another aspect of the disclosure is directed to methods of identifying antibodies which modulate (i.e., decrease) activity of a M-CSF comprising contacting a M-CSF with an antibody, and determining whether the antibody modifies activity of the M-CSF. The activity in the presence of the test antibody is compared to the activity in the absence of the test antibody. Where the activity of the sample containing the test antibody is lower than the activity in the sample lacking the test antibody, the antibody will have inhibited activity.

A variety of heterologous systems is available for functional expression of recombinant polypeptides that are well known to those skilled in the art. Such systems include bacteria (Strosberg, et al., Trends in Pharmacological Sciences (1992) 13:95-98), yeast (Pausch, Trends in Biotechnology (1997) 15:487-494), several kinds of insect cells (Vanden Broeck, Int. Rev. Cytology (1996) 164:189-268), amphibian cells (Jayawickreme et al., Current Opinion in Biotechnology (1997) 8: 629-634) and several mammalian cell lines (CHO, HEK293, COS, etc.; see Gerhardt, et al., Eur. J. Pharmacology (1997) 334:1-23). These examples do not preclude the use of other possible cell expression systems, including cell lines obtained from nematodes (PCT application WO 98/37177).

Also disclosed herein are methods of screening for antibodies which modulate the activity of M-CSF comprise contacting test antibodies with a M-CSF polypeptide and assaying for the presence of a complex between the antibody and the M-CSF. In such assays, the ligand is typically labeled. After suitable incubation, free ligand is separated from that present in bound form, and the amount of free or uncomplexed label is a measure of the ability of the particular antibody to bind to the M-CSF or M-CSFR polypeptide.

High throughput screening for antibody fragments or CDRs having suitable binding affinity to a M-CSF polypeptide may be employed. Briefly, large numbers of different small peptide test compounds are synthesized on a solid substrate. The peptide test antibodies are contacted with a M-CSF polypeptide and washed. Bound M-CSF polypeptides are then detected by methods well known in the art. Purified polypeptides of the disclosure can also be coated directly onto plates for use in the aforementioned drug screening techniques. In addition, non-neutralizing antibodies can be used to capture the protein and immobilize it on the solid support.

### Combination Therapy

Having identified more than one M-CSF antibody that is effective in an animal model, it may be further advantageous to mix two or more such M-CSF antibodies together to provide still improved efficacy against macrophage-associated disease. Compositions comprising one or more M-CSF antibody may be administered to persons or mammals suffering from, or predisposed to suffer from, macrophage-associated diseases. Concurrent administration of two therapeutic agents does not require that the agents be administered at the same time or by the same route, as long as there is an overlap in the time period during which the agents are exerting their therapeutic effect. Simultaneous or sequential administration is contemplated, as is administration on different days or weeks.

The administration of single anti-M-CSF antibodies, as well as combinations, or "cocktails", of different antibodies is contemplated herein. Such antibody cocktails may have certain advantages inasmuch as they contain antibodies which exploit different effector mechanisms. Such antibodies in combination may exhibit synergistic or additive therapeutic effects.

Combining RX1 or Human Engineered™ derivative of RX1 antibody with other therapeutics can have an effect on a patient experiencing macrophage-associated diseases. For example, one could use anti-M-CSF antibody in the manufacture of a medicament for treating a patient having an atherosclerotic disease or a disease associated with HIV, or treating a patient that has been pre-treated with a second therapeutic agent, or a patient that is not responsive to treatment with a second therapeutic agent. "Pre-treatment" means that a patient had been treated with the second therapeutic agent within 2 years, 1 year, 6 months, 3 months 2 months, 1 month, 2 weeks, 1 week, or at least one day before treatment with M-CSF antibody. Such a medicament containing anti-M-CSF antibody may be a medicament that is coordinated with treatment using a second therapeutic agent or a procedure, such as angioplasty. Alternatively, one could use the second therapeutic agent in the manufacture of a medicament that is coordinated with treatment using the anti-M-CSF antibody. The combination might also have a synergistic effect in a treated patient. The two therapeutics need not be administered simultaneously; for example, they can be administered within 1 day, 1 week, 2 weeks, 4 weeks, 2 months, 3 months, 6 months, 1 year or two years of each other.

Exemplary second therapeutic agents for treating diseases associated with atherosclerosis include a second anti-M-CSF antibody, a drug which beneficially alters the serum lipid profile (e.g., statins such as lovastatin, simvastatin and pravastatin, fluvastatin, atorvastatin, cerivastatin and rosuvastatin, drugs that lower intestinal absorption of cholesterol such as ezetimibe, fibrates, cholestyramine or colestipol resins, or nicotinic acid, or drugs containing highly polyunsaturated or omega-3 fatty acids, e.g. eicosapentaenoic acid and docosahexaenoic acid from fish oil), anti-anginal agents such as nitrates, beta-blockers, angiotensin converting enzyme inhibitors, angiotensin receptor blockers, calcium channel antagonists, anti-platelet agents, or anticoagulants.

Exemplary second therapeutic agents for treating diseases associated with HIV infection include a second anti-M-CSF antibody, or agents used in highly active antiretroviral therapy (HAART) as described in Barbaro G, et al., Curr Pharm Des.;11(14):1805-43 (2005), herein incorporated by reference in its entirety. For example, any reverse transcriptase inhibitor or protease inhibitor known in the art may be used.

Prodrug refers to a precursor or derivative form of a pharmaceutically active substance that is less cytotoxic or non-cytotoxic to cells compared to the parent drug and is capable of being enzymatically activated or converted into an active or the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug.

### Administration and preparation

The anti-M-CSF antibodies for use in the practice of the invention may be formulated into pharmaceutical compositions comprising a carrier suitable for the desired delivery method. Suitable carriers include any material which, when combined with the anti-M-CSF antibodies, retains the desired activity of the antibody and is nonreactive with the subject's immune systems. Examples include, but are not limited to, any of a number of standard pharmaceutical carriers such as sterile phosphate buffered saline solutions, bacteriostatic water, and the like. A variety of aqueous carriers may be used, e.g., water, buffered water, 0.4% saline, 0.3% glycine and the like, and may include other proteins for enhanced stability, such as albumin, lipoprotein, globulin, etc., subjected to mild chemical modifications or the like.

Therapeutic formulations of the antibody are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

The antibody is administered by any suitable means, including parenteral, subcutaneous, intraperitoneal, intrapulmonary, and intranasal, and, if desired for local treatment, intralesional administration. Parenteral infusions include intravenous, intraarterial, intraperitoneal, intramuscular, intradermal or subcutaneous administration. In addition, the antibody is suitably administered by pulse infusion, particularly with declining doses of the antibody. Preferably the dosing is given by injections, most preferably intravenous or subcutaneous injections. Other administration methods are contemplated, including topical, particularly transdermal, transmucosal, rectal, oral or local administration e.g. through a catheter placed close to the desired site.

For nasal administration, the pharmaceutical formulations and medicaments may be a spray or aerosol containing an appropriate solvent(s) and optionally other compounds such as, but not limited to, stabilizers, antimicrobial agents, antioxidants, pH modifiers, surfactants, bioavailability modifiers and combinations of these. A propellant for an aerosol formulation may include compressed air, nitrogen, carbon dioxide, or a hydrocarbon based low boiling solvent.

Injectable dosage forms generally include aqueous suspensions or oil suspensions which may be prepared using a suitable dispersant or wetting agent and a suspending agent. Injectable forms may be in solution phase or in the form of a suspension, which is prepared with a solvent or diluent. Acceptable solvents or vehicles include sterilized water, Ringer's solution, or an isotonic aqueous saline solution. Alternatively, sterile oils may be employed as solvents or suspending agents. Preferably, the oil or fatty acid is nonvolatile, including natural or synthetic oils, fatty acids, mono-, di- or tri-glycerides.

For injection, the pharmaceutical formulation and/or medicament may be a powder suitable for reconstitution with an appropriate solution as described above. Examples of these include, but are not limited to, freeze dried, rotary dried or spray dried powders, amorphous powders, granules, precipitates, or particulates. For injection, the formulations may optionally contain stabilizers, pH modifiers, surfactants, bioavailability modifiers and combinations of these.

Sustained-release preparations may be prepared. Suitable examples of sustained-release preparations include semipermeable matrices of solid hydrophobic polymers containing the antibody, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and y ethyl-L-glutamate, non-degradable ethylene-vinyl acetate, degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions. Other strategies known in the art may be used.

The formulations of the disclosure may be designed to be short-acting, fast-releasing, long-acting, or sustained-releasing as described herein. Thus, the pharmaceutical formulations may also be formulated for controlled release or for slow release.

The instant compositions may also comprise, for example, micelles or liposomes, or some other encapsulated form, or may be administered in an extended release form to provide a prolonged storage and/or delivery effect. Therefore, the pharmaceutical formulations and medicaments may be compressed into pellets or cylinders and implanted intramuscularly or subcutaneously as depot injections or as implants such as stents. Such implants may employ known inert materials such as silicones and biodegradable polymers.

Besides those representative dosage forms described above, pharmaceutically acceptable excipients and carries are generally known to those skilled in the art and are thus included in the instant disclosure. Such excipients and carriers are described, for example, in "Remingtons Pharmaceutical Sciences" Mack Pub. Co., New Jersey (1991).

Specific dosages may be adjusted depending on conditions of disease, the age, body weight, general health conditions, genotype, sex, and diet of the subject, dose intervals, administration routes, excretion rate, and combinations of drugs. Any of the above dosage forms containing effective amounts are well within the bounds of routine experimentation and therefore, well within the scope of the instant disclosure.

M-CSF antibodies useful as therapeutics for macrophage-associated diseases will often be prepared substantially free of other naturally occurring immunoglobulins or other biological molecules. Preferred M-CSF antibodies will also exhibit minimal toxicity when administered to a mammal afflicted with, or predisposed to suffer from macrophage-associated diseases.

The compositions of the disclosure may be sterilized by conventional, well known sterilization techniques. The resulting solutions may be packaged for use or filtered under aseptic conditions and lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride and stabilizers (e.g., 1 20% maltose, etc.).

The M-CSF antibodies for use in the present invention may also be administered via liposomes, which are small vesicles composed of various types of lipids and/or phospholipids and/or surfactant which are useful for delivery of a drug (such as the antibodies disclosed herein and, optionally, a chemotherapeutic agent). Liposomes include emulsions, foams, micelles, insoluble monolayers, phospholipid dispersions, lamellar layers and the like, and can serve as vehicles to target the M-CSF antibodies to a particular tissue as well as to increase the half life of the composition. A variety of methods are available for preparing liposomes, as described in, e.g., U.S. Patent Nos. 4,837,028 and 5,019,369.

Liposomes containing the antibody are prepared by methods known in the art, such as described in Epstein et al., Proc. Natl. Acad. Sci. USA 82: 3688 (1985); Hwang et al., Proc. Natl Acad. Sci. USA 77: 4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody can be conjugated to the liposomes as described in Martin et al., J. Biol. Chem. 257: 286-288 (1982) via a disulfide interchange reaction. A chemotherapeutic agent (such as Doxorubicin) is optionally contained within the liposome *[see, e.g.,* Gabizon et al., J. National Cancer Inst. 81(19): 1484 (1989)].

The concentration of the M-CSF antibody in these compositions can vary widely, i.e., from less than about 10%, usually at least about 25% to as much as 75% or 90% by weight and will be selected primarily by fluid volumes, viscosities, etc., in accordance with the particular mode of administration selected. Actual methods for preparing orally, topically and parenterally administrable compositions will be known or apparent to those skilled in the art and are described in detail in, for example, Remington's Pharmaceutical Science, 19th ed., Mack Publishing Co., Easton, PA (1995).

Determination of an effective amount of a composition to treat macrophage-associated diseases in a patient can be accomplished through standard empirical methods which are well known in the art. For example, the in vivo neutralizing activity of sera from a subject treated with a given dosage of M-CSF antibody may be evaluated using an assay that determines the ability of the sera to block M-CSF induced proliferation and survival of murine monocytes (CD11b+ cell, a subset of CD 11 cells, which expresses high levels of receptor to M-CSF) in vitro as described in Cenci et al., J Clin. Invest. 1055: 1279-87, 2000.

Compositions are administered to a mammal already suffering from, or predisposed to or at risk of a macrophage-associated disease in an amount sufficient to prevent or at least partially arrest the development of disease. An amount adequate to accomplish this is defined as a "therapeutically effective dose." Effective amounts of a M-CSF antibody will vary and depend on the severity of the disease and the weight and general state of the patient being treated, but generally range from about 1.0 µg/kg to about 100 mg/kg body weight. Exemplary doses may range from about 10 µg/kg to about 30 mg/kg, or from about 0.1 mg/kg to about 20 mg/kg or from about 1 mg/kg to about 10 mg/kg per application. Antibody may also be dosed by body surface area (e.g. up to 4.5 g/square meter). Other exemplary doses of antibody include up to 8g total in a single administration (assuming a body weight of 80 kg or body surface area of 1.8 square meters).

Administration may be by any means known in the art. For example, antibody may be administered by one or more separate bolus administrations, or by short or long term infusion over a period of, e.g., 5, 10, 15, 30, 60, 90 or 120 minutes. Following an initial treatment period, and depending on the patient's response and tolerance of the therapy, maintenance doses may be administered, e.g., weekly, biweekly, every 3 weeks, every 4 weeks, monthly, bimonthly, every 3 months, or every 6 months, as needed to maintain patient response. More frequent dosages may be needed until a desired suppression of disease symptoms occurs, and dosages may be adjusted as necessary. The progress of this therapy is easily monitored by conventional techniques and assays. The therapy may be for a defined period or may be chronic and continue over a period of years until disease progression or death.

Single or multiple administrations of the compositions can be carried out with the dose levels and pattern being selected by the treating physician. For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

In any event, the formulations should provide a quantity of M-CSF antibody over time that is sufficient to effectively prevent or minimize the severity of macrophage-associated disease. The compositions of the present disclosure may be administered alone or as an adjunct therapy in conjunction with other therapeutics known in the art for the treatment of macrophage-associated disease.

The antibody composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutically effective amount of the antibody to be administered will be governed by such considerations, and is the minimum amount necessary to prevent, ameliorate, or treat the M-CSF mediated disease, condition or disorder. Such amount is preferably below the amount that is toxic to the host or renders the host significantly more susceptible to infections.

The antibody need not be, but is optionally formulated with one or more agents currently used to prevent or treat the disorder in question. The effective amount of such other agents depends on the amount of antibody present in the formulation, the type of disease, condition or disorder or treatment, and other factors discussed above. These are generally used in the same dosages and with administration routes as used hereinbefore or about from 1 to 99% of the heretofore employed dosages.

Also disclosed herein is an article of manufacture containing materials useful for the treatment of a macrophage-associated disease. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the antibody. The label on, or associated with, the container indicates that the composition is used for treating the condition of choice. The article of manufacture may further comprise a second container containing a second therapeutic agent (including any of the second therapeutic agents for macrophage-associated diseases discussed herein or known in the art). The article of manufacture may further comprise another container containing a pharmaceutically-acceptable buffer, such as phosphate-buffered saline, Ringer's solution or dextrose solution for reconstituting a lyophilized antibody formulation. It may further include other materials desirable from a commercial and user standpoint, including other buffers, diluents, filters, needles, syringes, and package inserts with instructions for use.

### Antibody conjugates

Anti-M-CSF antibodies may be administered in their "naked" or unconjugated form, or may be conjugated directly to other therapeutic or diagnostic agents, or may be conjugated indirectly to carrier polymers comprising such other therapeutic or diagnostic agents.

Antibodies can be detectably labeled through the use of radioisotopes, affinity labels (such as biotin, avidin, etc.), enzymatic labels (such as horseradish peroxidase, alkaline phosphatase, etc.) fluorescent or luminescent or bioluminescent labels (such as FITC or rhodamine, etc.), paramagnetic atoms, and the like. Procedures for accomplishing such labeling are well known in the art; for example, see (Sternberger, L.A. et al., J. Histochem. Cytochem. 18:315 (1970); Bayer, E.A. et al., Meth. Enzym. 62:308 (1979); Engval, E. et al., Immunol. 109:129 (1972); Goding, J.W. J. Immunol. Meth. 13:215 (1976)).

Conjugation of antibody moieties is described in U.S. Patent No. 6,306,393. General techniques are also described in Shih et al., Int. J. Cancer 41:832-839 (1988); Shih et al., Int. J. Cancer 46:1101-1106 (1990); and Shih et al., U.S. Pat. No. 5,057,313. This general method involves reacting an antibody component having an oxidized carbohydrate portion with a carrier polymer that has at least one free amine function and that is loaded with a plurality of drug, toxin, chelator, boron addends, or other therapeutic agent. This reaction results in an initial Schiff base (imine) linkage, which can be stabilized by reduction to a secondary amine to form the final conjugate.

The carrier polymer may be, for example, an aminodextran or polypeptide of at least 50 amino acid residues. Various techniques for conjugating a drug or other agent to the carrier polymer are known in the art. A polypeptide carrier can be used instead of aminodextran, but the polypeptide carrier should have at least 50 amino acid residues in the chain, preferably 100-5000 amino acid residues. At least some of the amino acids should be lysine residues or glutamate or aspartate residues. The pendant amines of lysine residues and pendant carboxylates of glutamine and aspartate are convenient for attaching a drug, toxin, immunomodulator, chelator, boron addend or other therapeutic agent. Examples of suitable polypeptide carriers include polylysine, polyglutamic acid, polyaspartic acid, co-polymers thereof, and mixed polymers of these amino acids and others, e.g., serines, to confer desirable solubility properties on the resultant loaded carrier and conjugate.

Alternatively, conjugated antibodies can be prepared by directly conjugating an antibody component with a therapeutic agent. The general procedure is analogous to the indirect method of conjugation except that a therapeutic agent is directly attached to an oxidized antibody component. For example, a carbohydrate moiety of an antibody can be attached to polyethyleneglycol to extend half-life.

Alternatively, a therapeutic agent can be attached at the hinge region of a reduced antibody component via disulfide bond formation, or using a heterobifunctional cross-linker, such as N-succinyl 3-(2-pyridyldithio)proprionate (SPDP). Yu et al., Int. J. Cancer56:244 (1994). General techniques for such conjugation are well-known in the art. See, for example, Wong, Chemistry Of Protein Conjugation and Cross-Linking (CRC Press 1991); Upeslacis et al., "Modification of Antibodies by Chemical Methods," in Monoclonal Antibodies: Principles and Applications, Birch et al. (eds.), pages 187-230 (Wiley-Liss, Inc. 1995); Price, "Production and Characterization of Synthetic Peptide-Derived Antibodies," in Monoclonal Antibodies: Production, Enineering and Clinical Application, Ritter et al. (eds.), pages 60-84 (Cambridge University Press 1995). A variety of bifunctional protein coupling agents are known in the art, such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

Finally, fusion proteins can be constructed that comprise one or more anti-M-CSF antibody moieties and another polypeptide. Methods of making antibody fusion proteins are well known in the art. See, *e.g.,* U.S. Patent No. 6,306,393. Antibody fusion proteins comprising an interleukin-2 moiety are described by Boleti et al., Ann. Oncol. 6:945 (1995), Nicolet et al., Cancer Gene Ther. 2:161 (1995), Becker et al., Proc. Nat'l Acad. Sci. USA 93:7826 (1996), Hank et al., Clin. Cancer Res. 2:1951 (1996), and Hu et al., Cancer Res. 56:4998 (1996).

The invention is illustrated by the following examples, which are not intended to be limiting in any way.

### EXAMPLES

### EXAMPLE 1

This example shows that M-CSF antibodies RX1 and 5A1 are species specific and that antibodies RX1, MC1, and MC3 neutralize human M-CSF activity. RX1 is a commercially sold antibody that was available more than a year prior to the filing date of this application. Exemplary commercial sources include, but are not limited to, mouse anti-human M-CSF monoclonal antibody clones 116, 692, and 21 (Anogen); anti-human M-CSF antibody clones 21113.131, 26730, and 26786 (R & D Systems, Inc.); and anti-human M-CSF antibodyclone M16 (Antigenix America, Inc.).

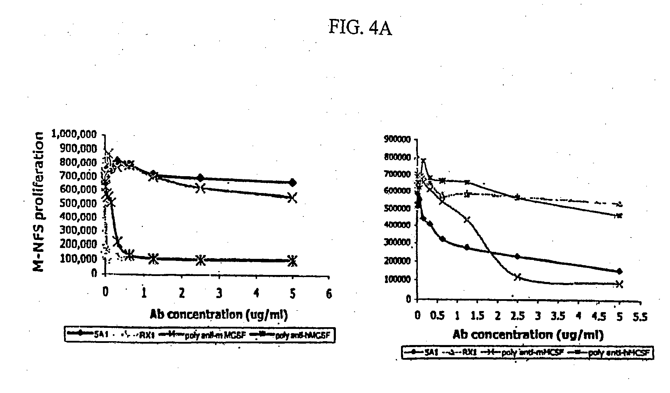

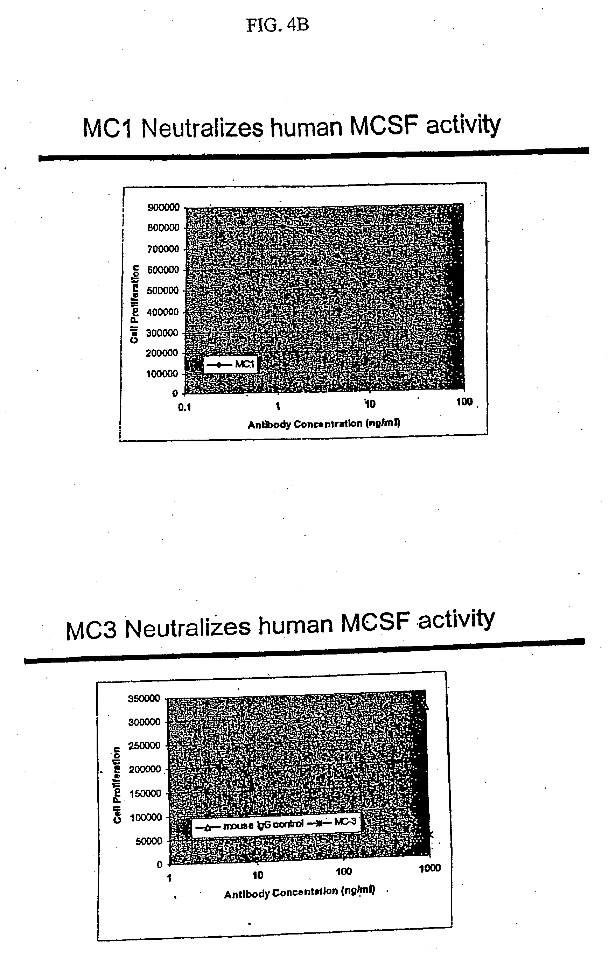

To test the neutralizing activity of RX1 and 5A1, a proliferation assay of M-NFS-60 cell line was used (American Type Culture Collection Accession No. CRL-1838, available from ATCC in Rockville, MD, USA, derived from a myelogenous leukemia induced with the Cas-Br-MuLV wild mouse ecotropic retrovirous, responsive to both interleukin 3 and M-CSF and which contain a truncated c-myb proto-oncogene caused by the integration of a retrovirus). Proliferation of M-NFS-60 requires active M-CSF in a dose-dependent fashion. In the assay, M-NFS-60 cells were washed and plated in RPMI 1640 medium with 10% FBS and 3000 U/ml of M-CSF and 1% Pen/Strep. Recombinant human M-CSF (at 10 ng/ml final concentration), human or murine-specific, was incubated with various concentrations of antibodies for 1 hour at 37°C in 5% CO₂ in an incubator. Following the incubation, the mixture was added to the M-NFS-60 culture in 96 well microtiter plates. The total assay volume per well was 100µl, with 10 ng/ml M-CSF, and the antibody concentration indicated in Figure 4. Cells were incubated at 37°C under 5% CO₂ for 72 hours before cell numbers were quantified by CellTiter Glo assay (Promega). The aforementioned assay was repeated for antibodies MC3 and MC1.

As shown in Figure 4, M-CSF antibodies RX1 and 5A1 are species specific. Cell proliferation is presented as the fluorescent reading from CellTiter Glo assay, which is linear with cell number. Species specific neutralizing activity of RX1 and 5A1 is shown by its ability to inhibit M-NFS-60 in the presence of either human or murine M-CSF. Finally, as shown in Figure 4B, antibodies MC3 and MC1 are also effective inhibitors of M-CSF activity.

### EXAMPLE 2

This example sets out a procedure for humanization of the RX1 antibody. 5H4, MC1 and MC3 are humanized using similar procedures.

### Design of genes for humanized RX1 light and heavy chains

The nucleotide and amino acid sequence for murine RX1 are set forth in Figure 3B. The sequence of a human antibody identified using the National Biomedical Foundation Protein Identification Resource or similar database is used to provide the framework of the humanized antibody. To select the sequence of the humanized heavy chain, the murine RX1 heavy chain sequence is aligned with the sequence of the human antibody heavy chain. At each position, the human antibody amino acid is selected for the humanized sequence, unless that position falls in any one of four categories defined below, in which case the murine RX1 amino acid is selected:
(1) The position falls within a complementarity determining region (CDR), as defined by Kabat, J. Immunol., 125, 961-969 (1980);
(2) The human antibody amino acid is rare for human heavy chains at that position, whereas the murine RX1 amino acid is common for human heavy chains at that position;
(3) The position is immediately adjacent to a CDR in the amino acid sequence of the murine RX1 heavy chain; or
(4) 3-dimensional modeling of the murine RX1 antibody suggests that the amino acid is physically close to the antigen binding region.

To select the sequence of the humanized light chain, the murine RX1 light chain sequence is aligned with the sequence of the human antibody light chain. The human antibody amino acid is selected at each position for the humanized sequence, unless the position again falls into one of the categories described above and repeated below:
(1) CDR's;
(2) murine RX1 amino acid more typical than human antibody;
(3) Adjacent to CDR's; or
(4) Possible 3-dimensional proximity to binding region.

The actual nucleotide sequence of the heavy and light chain genes is selected as follows:
(1) The nucleotide sequences code for the amino acid sequences chosen as described above;
(2) 5' of these coding sequences, the nucleotide sequences code for a leader (signal) sequence. These leader sequences were chosen as typical of antibodies;
(3) 3' of the coding sequences, the nucleotide sequences are the sequences that follow the mouse light chain J5 segment and the mouse heavy chain J2 segment, which are part of the murine RX1 sequence. These sequences are included because they contain splice donor signals; and
(4) At each end of the sequence is an Xba I site to allow cutting at the Xba I sites and cloning into the Xba I site of a vector.

### Construction of humanized light and heavy chain genes

To synthesize the heavy chain, four oligonucleotides are synthesized using an Applied Biosystems 380B DNA synthesizer. Two of the oligonucleotides are part of each strand of the heavy chain, and each oligonucleotide overlaps the next one by about 20 nucleotides to allow annealing. Together, the oligonucleotides cover the entire humanized heavy chain variable region with a few extra nucleotides at each end to allow cutting at the Xba I sites. The oligonucleotides are purified from polyacrylamide gels.

Each oligonucleotide is phosphorylated using ATP and T4 polynucleotide kinase by standard procedures (Maniatis et al., Molecular Cloning: A Laboratory Manual, 2nd ed., Cold Spring Harbor Laboratory, Cold Spring Harbor, N.Y. (1989)). To anneal the phosphorylated oligonucleotides, they are suspended together in 40 ul of TA (33 mM Tris acetate, pH 7.9, 66 mM potassium acetate, 10 mM magnesium acetate) at a concentration of about 3.75 uM each, heated to 95 °C. for 4 min. and cooled slowly to 4 °C. To synthesize the complete gene from the oligonucleotides by synthesizing the opposite strand of each oligonucleotide, the following components are added in a final volume of 100 ul:

| | |
|---|---|
| 10 ul | annealed oligonucleotides |
| 0.16 mM | each deoxyribonucleotide |
| 0.5 mM | ATP |
| 0.5 mM | DTT |
| 100 ug/ml | BSA |
| 3.5 ug/ml | T4 g43 protein (DNA polymerase) |
| 25 ug/ml | T4 g44/62 protein (polymerase accessory protein) |
| 25 ug/ml | 45 protein (polymerase accessory protein) |

The mixture is incubated at 37 °C for 30 min. Then 10 u of T4 DNA ligase is added and incubation at 37 °C is resumed for 30 min. The polymerase and ligase are inactivated by incubation of the reaction at 70 °C for 15 min. To digest the gene with Xba I, 50 ul of 2 X TA containing BSA at 200 ug/ml and DTT at 1 mM, 43 ul of water, and 50 u of Xba I in 5 ul is added to the reaction. The reaction is incubated for 3 hr at 37 °C, and then purified on a gel. The Xba I fragment is purified from a gel and cloned into the Xba I site of the plasmid pUC19 by standard methods. Plasmids are purified using standard techniques and sequenced using the dideoxy method.

Construction of plasmids to express humanized light and heavy chains is accomplished by isolating the light and heavy chain Xba I fragments from the pUC19 plasmid in which it had been inserted and then inserting it into the Xba I site of an appropriate expression vector which will express high levels of a complete heavy chain when transfected into an appropriate host cell.

### Synthesis and affinity of humanized antibody

The expression vectors are transfected into mouse Sp2/0 cells, and cells that integrate the plasmids are selected on the basis of the selectable marker(s) conferred by the expression vectors by standard methods. To verify that these cells secreted antibody that binds to M-CSF, supernatant from the cells are incubated with cells that are known to express M-CSF. After washing, the cells are incubated with fluorescein-conjugated goat anti-human antibody, washed, and analyzed for fluorescence on a FACSCAN cytofluorometer.

For the next experiments, cells producing the humanized antibody are injected into mice, and the resultant ascites is collected. Humanized antibody is purified to substantial homogeneity from the ascites by passage through an affinity column of goat anti-human immunoglobulin antibody, prepared on an Affigel-10 support (Bio-Rad Laboratories, Inc., Richmond, Calif.) according to standard techniques. To determine the affinity of the humanized antibody relative to the original murine RX1 antibody, a competitive binding experiment is performed according to techniques known in the art.

### EXAMPLE 3

This example describes cloning and expression of Human Engineered™ RX1 antibodies, as well as purification of such antibodies and testing for binding activity. Human Engineered™ 5H4, MC1, and MC3 antibodies are prepared using similar procedures.

### Design of Human Engineered™ sequences

Human Engineering™ of antibody variable domains has been described by Studnicka [See, e.g., Studnicka et al. U.S. Patent No. 5,766,886; Studnicka et al. Protein Engineering 7: 805-814 (1994)] as a method for reducing immunogenicity while maintaining binding activity of antibody molecules. According to the method, each variable region amino acid has been assigned a risk of substitution. Amino acid substitutions are distinguished by one of three risk categories : (1) low risk changes are those that have the greatest potential for reducing immunogenicity with the least chance of disrupting antigen binding; (2) moderate risk changes are those that would further reduce immunogenicity, but have a greater chance of affecting antigen binding or protein folding; (3) high risk residues are those that are important for binding or for maintaining antibody structure and carry the highest risk that antigen binding or protein folding will be affected. Due to the three-dimensional structural role of prolines, modifications at prolines are generally considered to be at least moderate risk changes, even if the position is typically a low risk position. Subtitutional changes are preferred but insertions and deletions are also possible. Figures 3B and 3C show the risk assignment for each amino acid residue of murine RX1 light and heavy chains, respectively, categorized as a high, moderate or low risk change.

Variable regions of the light and heavy chains of the murine RX1 antibody were Human Engineered™ using this method. Amino acid residues that are candidates for modification according to the method at low risk positions were identified by aligning the amino acid sequences of the murine variable regions with a human variable region sequence. Any human variable region can be used, including an individual VH or VL sequence or a human consensus VH or VL sequence. The amino acid residues at any number of the low risk positions, or at all of the low risk positions, can be changed. For the Human Engineered™ "low risk" heavy chain sequence in Figures 13A-B, human consensus Vh2 (based on Kabat) was used as the template, and for each position where the murine and human amino acid residues differed at low risk positions, an amino acid modification was introduced that replaced the murine residue with the human residue. For the Human Engineered™ "low risk" light chain sequence in Figures 14A-B, human consensus kappa 3 (based on Kabat) was used as the template, and for each position where the murine and human amino acid residues differed at low risk positions, an amino acid modification was introduced that replaced the murine residue with the human residue. A total of 16 amino acid low risk modifications were made to the light chain and 8 low risk modifications were made to the heavy chain.

Similarly, amino acid residues that are candidates for modification according to the method at all of the low and moderate risk positions were identified by aligning the amino acid sequences of the murine variable regions with a human variable region sequence. The amino acid residues at any number of the low or moderate risk positions, or at all of the low and moderate risk positions, can be changed. For the Human Engineered™ heavy chain sequence in Figures 13A-B, human consensus Vh2 (based on Kabat) was used as the template, and for each position where the murine and human amino acid residues differed at low or moderate risk positions, an amino acid modification was introduced that replaced the murine residue with the human residue. For the Human Engineered™ light chain sequence in Figures 14A-B, human consensus kappa 3 (based on Kabat) was used as the template, and for each position where the murine and human amino acid residues differed at low or moderate risk positions, an amino acid modification was introduced that replaced the murine residue with the human residue. A total of 19 low and moderate risk amino acid modifications were made to the light chain and 12 low and moderate modifications were made to the heavy chain.

An "alternative low risk" light chain sequence was also prepared as shown in Figures 15A-B, in which the modification at position 54 was reversed back to murine. An "alternative low+moderate risk" light chain sequence was also prepared as shown in Figures 15A-B, in which the modifications at positions 54-56 were reversed back to murine.

Finally, a Human Engineered™ "low+moderate risk" light chain V region sequence also was produced using human germline VK6 subgroup 2-1-(1) A14 as the template, as shown in Figures 16A-B.

Also contemplated by the present disclosure is retaining amino acids 41-43 (NGS) of Figure 3A which represent the glycosylation site. Alternatively, only one or two of amino acids 41-43 (e.g., NG) may be retained.

### Preparation of Expression Vectors for Permanent Cell Line Development

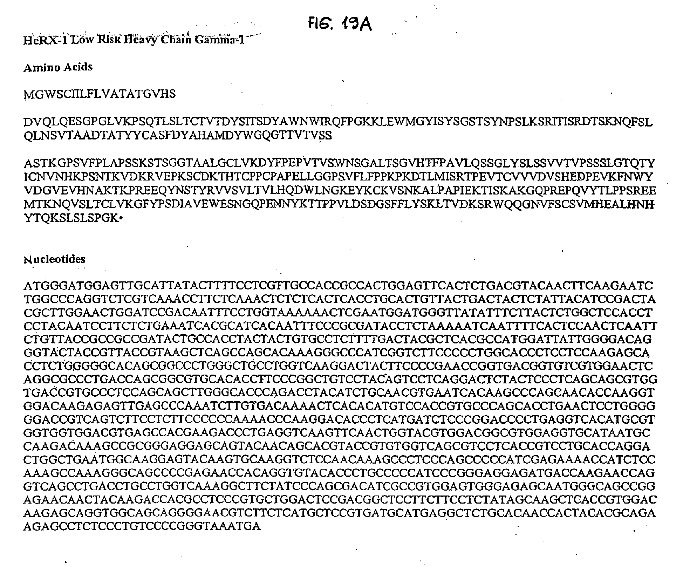

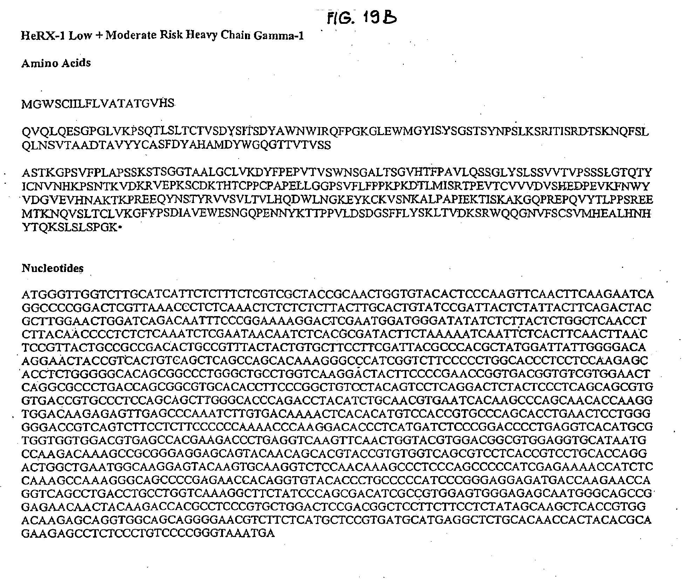

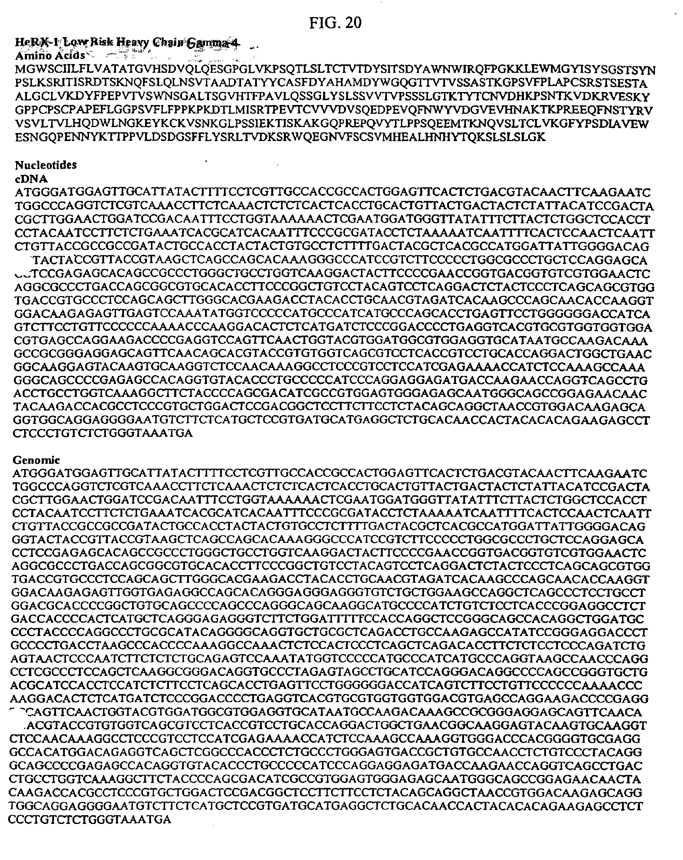

DNA fragments encoding each of the above-described heavy and light chain V region sequences along with antibody-derived signal sequences were constructed using synthetic nucleotide synthesis. DNA encoding each of the light chain V region amino acid sequences described above were inserted into vector pMXP10 containing the human Kappa light chain constant region. DNA encoding each of the heavy chain V region amino acid sequences described above were inserted into vector pMXP6 containing the human Gamma-2 heavy chain constant region. Additional vectors were constructed containing the the heavy chain V region amino acid sequences fused to the human Gamma-1 (cDNA)and Gamma-4 (genomic and cDNA) constant regions having sequences displayed in figures 19A, 19b, and 20. All of these vectors contain a hCMV promoter and a mouse kappa light chain 3' untranslated region as well as selectable marker genes such as *neo* or or *his* for selection of G418 - or histidinol - resistant transfectants, respectively. The light and heavy chain vectors are described in Tables 2 and 3, respectively.

**Table 2. Single gene permanent Kappa light chain vectors.**

| **Plasmid** | **V Region** | **Selective Marker** |
|---|---|---|
| pMXC2 | Low + Mod Risk (Kabat) | *neo* |
| pMXC6 | Low Risk (Kabat) | *neo* |
| pMXC13 | Low Risk (Kabat) - R54 to S | *neo* |
| pMXC14 | Low+Mod Risk (Kabat)-RAT54,55,56 to SIS | *neo* |
| pMXC22 | Low + Mod Risk (Germline) | *neo* |

**Table 3. Single gene permanent heavy chain vectors.**

| **Plasmid** | **V Region** | **C Region** | **Selective Marker** |
|---|---|---|---|
| pMXC7 | Low + Mod Risk (Kabat) | Gamma 2 | *neo* |
| pMXC8 | Low Risk (Kabat) | Gamma 2 | *neo* |
| pMXC40 | Low Risk (Kabat) | Gamma 1 | *neo* |
| pMXC41 | Low + Mod Risk (Kabat) | Gamma 1 | *neo* |
| pMXC45 | Low + Mod Risk (Kabat) | Gamma 4 (genomic) | *neo* |
| pMXC46 | Low + Mod Risk (Kabat) | Gamma 4 (cDNA) | *neo* |

Vectors comprising the desired Human Engineered™ light plus heavy chain genes (Gamma-1, Gamma-2 and Gamma-4) were then constructed. These "2-Gene" vectors contain genes encoding each antibody chain, heavy and light, under control of the hCMV promoter, CMV splice donor, SV40 16S splice acceptor and the mouse kappa light chain 3' untranslated DNA including the polyA site. They also contain a selectable marker gene such as *neo* or *his* and the ampicillin resistance gene. Vectors containing both heavy and light chain genes are described in Table 4. Vectors comprising two copies of each light and heavy chain genes (four gene vectors) also can be constructed.

**Table 4. Two-gene permanent expression vectors**

| | | **Heavy Chain** | | |
|---|---|---|---|---|
| **Plasmid** | **Kappa Light Chain** | **V region** | **C region** | **Selective Marker** |
| pMXC12 | Low Risk (Kabat) | Low Risk (Kabat) | Gamma 2 | *neo* |
| pMXC37 | Low Risk (Kabat) | Low Risk (Kabat) | Gamma 2 | *his* |
| pMXC9 | Low + Mod Risk (Kabat) | Low + Mod Risk (Kabat) | Gamma 2 | *neo* |
| pMXC16 | Low Risk (Kabat) | Low + Mod Risk (Kabat) | Gamma 2 | *neo* |
| pMXC17 | Low + Mod Risk (Kabat) | Low Risk (Kabat) | Gamma 2 | *neo* |
| pMXC18 | Low Risk (Kabat) R54 to S | Low + Mod Risk (Kabat) | Gamma 2 | *neo* |
| pMXC19 | Low+Mod Risk (Kabat)-RAT54,55,56 to SIS | Low + Mod Risk (Kabat) | Gamma 2 | *neo* |
| pMXC20 | Low Risk (Kabat) - R54 to S | Low Risk (Kabat) | Gamma 2 | *neo* |
| pMXC21 | Low+Mod Risk (Kabat)-RAT54,55,56 to SIS | Low Risk (Kabat) | Gamma 2 | *neo* |
| pMXC25 | Low + Mod Risk (Germline) | Low + Mod Risk (Kabat) | Gamma 2 | *neo* |
| pMXC47 | Low + Mod Risk (Germline) | Low + Mod Risk (Kabat) | Gamma 2 | *his* |
| pMXC26 | Low + Mod Risk (Germline) | Low Risk (Kabat) | Gamma 2 | *neo* |
| pMXC42 | Low + Mod Risk (Germline) | Low Risk (Kabat) | Gamma 1 | *neo* |
| pMXC43 | Low + Mod Risk (Germline) | Low + Mod Risk (Kabat) | Gamma 1 | *neo* |
| pMXC50 | Low + Mod Risk (Germline) | Low + Mod Risk (Kabat) | Gamma 1 | *his* |
| pMXC48 | Low + Mod Risk (Germline) | Low + Mod Risk (Kabat) | Gamma 4 (cDNA) | *Neo* |
| pMXC49 | Low + Mod Risk (Germline) | Low + Mod Risk (Kabat) | Gamma 4 (genomic) | *neo* |

### Preparation of Expression Vectors for Transient Expression

Vectors containing either the light or heavy chain genes described above also were constructed for transient transfection. These vectors are similar to those described above for permanent transfections except that instead of the *neo* or *his* genes, they contain the Epstein-Barr virus *ori*P for replication in HEK293 cells that express the Epstein-Barr virus nuclear antigen. The vectors for transient transfection are described in Tables 5 and 6.

**Table 5. Transient Kappa light chain vectors.**

| **Plasmid** | **V Region** |
|---|---|
| pMXC1 | Low + Mod Risk (Kabat) |
| pMXC2 | Low Risk (Kabat) |
| pMXC10 | Low+Mod Risk (Kabat)-RAT54,55,56 to SIS |
| pMXC11 | Low Risk (Kabat) - R54 to S |
| pMXC15 | Low + Mod Risk (Germline) |

**Table 6. Transient heavy chain vectors.**

| **Plasmid** | **V Region** | **C Region** |
|---|---|---|
| pMXC3 | Low + Mod Risk (Kabat) | Gamma 2 |
| pMXC4 | Low Risk (Kabat) | Gamma 2 |
| pMXC29 | Low Risk (Kabat) | Gamma 1 |
| pMXC38 | Low Risk (Kabat) | Gamma 4 (genomic) |
| pMXC39 | Low + Mod Risk (Kabat) | Gamma 1 |

### Transient Expression of Human-Engineered RX1 in HEK293E Cells

Separate vectors each containing *ori*P from the Epstein-Barr Virus and the light chain or heavy chain genes described above were transfected transiently into HEK293E cells. Transiently transfected cells were allowed to incubate for up to 10 days after which the supernatant was recovered and antibody purified using Protein A chromatography. The proteins produced by transient transfection of 293E cells are described in Table 7 below.

**Table 7. Human-engineered RX1 antibodies prepared.**

| | **Light Chain** | | **Heavy Chain** | |
|---|---|---|---|---|
| **Antibody** | **Plasmid** | **Protein** | **Plasmid** | **Protein** |
| **heRX1-1.G2** | pMXC2 | Low Risk (Kabat) | pMXC4 | Low Risk (Kabat) |
| **heRX1-2.G2** | pMXC2 | Low Risk (Kabat) | pMXC3 | Low + Mod Risk (Kabat) |
| **heRX1**-**3**.**G2** | pMXC1 | Low + Mod Risk (Kabat) | pMXC4 | Low Risk (Kabat) |
| **heRX1-4.G2** | pMXC1 | Low + Mod Risk (Kabat) | pMXC3 | Low + Mod Risk (Kabat) |
| **heRX1-5.G2** | pMXC11 | Low Risk (Kabat) - R54 to S | pMXC4 | Low Risk (Kabat) |
| **heRX1-6.G2** | pMXC11 | Low Risk (Kabat) - R54 to S | pMXC4 | Low Risk (Kabat) |
| **heRX1-7.G2** | pMXC10 | Low+Mod Risk (Kabat)-RAT54,55,56 to SIS | pMXC4 | Low Risk (Kabat) |
| **heRX1-8.G2** | pMXC10 | Low+Mod Risk (Kabat)-RAT 54,55,56 to SIS | pMXC3 | Low + Mod Risk (Kabat) |
| **heRX1-9.G2** | pMXC15 | Low + Mod Risk (Germline) | pMXC4 | Low Risk (Kabat) |
| **heRX1-10.G2** | pMXC15 | Low + Mod Risk (Germline) | pMXC3 | Low + Mod Risk (Kabat) |
| **heRX1-1.G1** | pMXC2 | Low Risk (Germline) | pMXC29 | Low Risk (Kabat) |
| **heRX1-10.G1** | pMXC15 | Low + Mod Risk (Germline) | pMXC39 | Low + Mod Risk (Kabat) |
| **heRX1-9.G4** | pMXC15 | Low + Mod Risk (Germline) | pMXC38 | Low Risk (Kabat) |

### Development of Permanently Transfected CHO-K1 Cells

The vectors described above (Table 4) containing one copy each of the light and heavy genes together are transfected into Ex-Cell 302-adapted CHO-K1 cells. CHO-K1 cells adapted to suspension growth in Ex-Cell 302 medium are typically electroporated with 40 ug of linearized vector. Alternatively, linearized DNA can be complexed with linear polyethyleneimine (PEI) and used for transfection. The cells are plated in 96 well plates containing Ex-Cell 302 medium supplemented with 1% FBS and G418. Clones are screened in 96 well plates and the top ∼10% of clones from each transfection are transferred to 24 well plates containing Ex-Cell 302 medium.

A productivity test is performed in 24 well plates in Ex-Cell 302 medium for cultures grown for 7 and 14 days at which time culture supernatants are tested for levels of secreted antibody by an immunoglobulin ELISA assay for IgG.

The top clones are transferred to shake flasks containing Ex-Cell 302 medium. As soon as the cells are adapted to suspension growth, a shake flask test is performed with these clones in Ex-Cell 302 medium. The cells are grown for up to 10 days in 125 ml Erlenmeyer flasks containing 25 ml media. The flasks are opened at least every other day of the incubation period to allow for gas exchange and the levels of immunoglobulin polypeptide in the culture medium are determined by IgG ELISA at the end of the incubation period. Multiple sequential transfections of the same cell line with two or three multi-unit transcription vectors results in clones and cell lines that exhibit further increases in levels of immunoglobulin production, preferably to 300 µg/ml or more.

### Purification

A process for the purification of immunoglobulin polypeptides from vectors and all lines may be designed. According to methods well known in the art, cells are removed by filtration after termination. The filtrate is loaded onto a Protein A column (in multiple passes, if needed). The column is washed and then the expressed and secreted immunoglobulin polypeptides are eluted from the column. For preparation of antibody product, the Protein A pool is held at a low pH (pH 3 for a minimum of 30 minutes and a maximum of one hour) as a viral inactivation step. An adsorptive cation exchange step is next used to further purify the product. The eluate from the adsorptive separation column is passed through a virus retaining filter to provide further clearance of potential viral particles. The filtrate is further purified by passing through an anion exchange column in which the product does not bind. Finally, the purification process is concluded by transferring the product into the formulation buffer through diafiltration. The retentate is adjusted to a protein concentration of at least 1 mg/mL and a stabilizer is added.

### Binding activity

The M-CSF binding activity of the recombinant Human Engineered™ antibodies is evaluated. Protein is purified from shake flask culture supernatants by passage over a protein A column followed by concentration determination by A₂₈₀. Binding assays are performed as described in Example 1 above or 7 below. Immulon II plates are precoated with the sM-CSF antigen pre-diluted in a PBS coating solution to immobilize it to the microplate. Various test concentrations of M-CSF ranging from 0.25 to 20 ug/ml are added at 50 ul/well and incubated at 4°C overnight. The plates are then washed 3 times with PBS-0.05% Tween. Blocking is performed by adding in PBS-0.05% Tween 1% BSA followed by a 30 minute incubation at 37°C. Dilutions of immunoglobulin polypeptides are prepared in PBS-0.05% Tween 1% BSA solution. 2- or 3-fold serial dilutions are prepared and added (100 ul/well) in duplicate or triplicate. After a 90 minute incubation at 37°C, the microplate is washed 3 times with PBS-0.05% Tween. For signal development, goat anti-human IgG (gamma- or Fc-specific) secondary antibody conjugated to peroxidase is added to each well and incubated for 60 minutes at 37°C followed by addition of OPD at 0.4 mg/ml in citrate buffer plus 0.012% H₂O₂. After 5 - 10 minutes at room temperature the assay is stopped by the addition of 100 ul 1M H₂SO₄ and the plates are read at 490nm. Both goat anti-human IgG (gamma-specific) and goat anti-human IgG (Fc-specific) antibodies have been employed.

### EXAMPLE 4

The following example sets out a procedure for the treatment of humans using M-CSF-specific antibody, such as an RX1-derived or RX1-competing antibody, including an RX1 Human Engineered™ antibody with a modified or unmodified IgG1 or IgG4 constant region. The procedure can also be followed for an MC1- or MC3-derived or MC1- or MC3-competing antibody.

The measured M-CSF level in human plasma is about 1 ng/ml. M-CSF neutralizing antibody RX1 has a measured EC₅₀ of 2 ng/ml against 1 ng/ml human M-CSF. Accordingly, the effective antibody concentration in human plasma is expected to be 10 to 50,000 fold over its EC₅₀, i.e. 20 ng/ml to 100 ug/ml antibody in human plasma.

Subjects suffering from a macrophage-associated disease are administered anti-M-CSF antibody at an initial dose of 10 mg/kg on a weekly basis and observed for signs of adverse effects or improvement in symptoms of clinical disease. Subjects that show no signs of adverse effects are administered gradually escalating doses of 15, 20, 25 or 30 mg/kg and observed for signs of improvement in symptoms of clinical disease.

### EXAMPLE 5

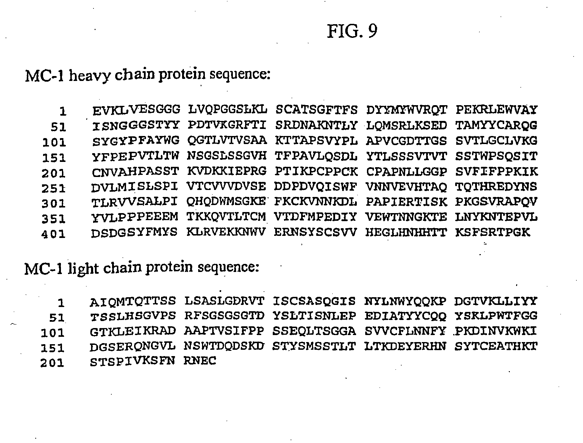

The following example shows the procedure for producing antibodies MC1 and MC3. MC1 and MC3 are two monoclonal murine antibodies that neutralize human M-CSF antibody and bind to human M-CSF. The amino acid sequences of these antibodies are shown in Figures 9 and 10, respectively. They were identified by a series of steps including a) immunization of Balb C mice with recombinant human M-CSF; b) screening for positive clones that produce antibodies which bind to human M-CSF in an ELISA format; c) subcloning of positive clones to generate stable hybridoma clones; d) scale-up of cell culture to produce large quantity of antibodies; e) purification and characterization of antibodies in affinity analysis, cell binding, and neutralizing activity assay as described in previous examples.

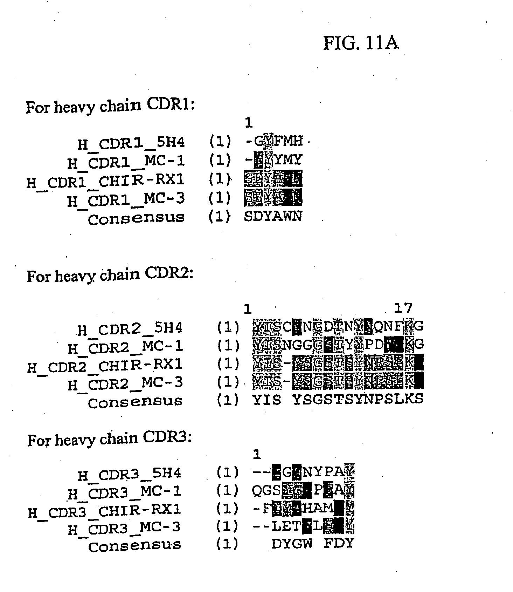

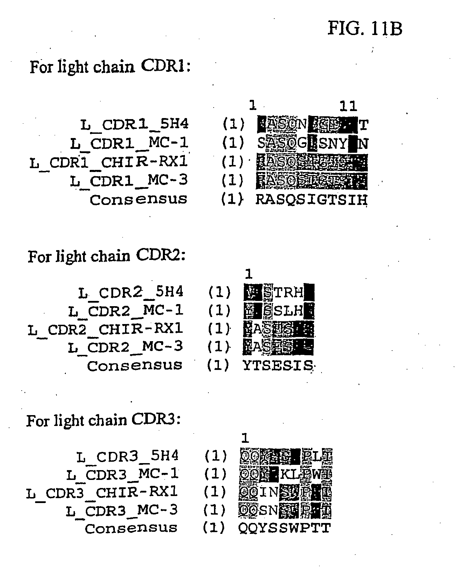

Figures 11A and 11B show the alignment of the CDRs of the heavy and light chains, respectively, of antibodies RX1, 5H4, MC1 and MC3.

Humanized and Human Engineered™ versions are generated as described in the examples above.

### EXAMPLE 6

This example shows that murine anti-M-CSF antibodies RX1 and 5H4, as well as Fab fragments thereof, have different neutralizing activities. The following example also shows that antibodies RX1, 5H4, and MC3 have varying affinities for M-CSF. This example further demonstrates that the affinities of the aforementioned intact antibodies are higher relative to Fab fragments of the aforementioned antibodies.

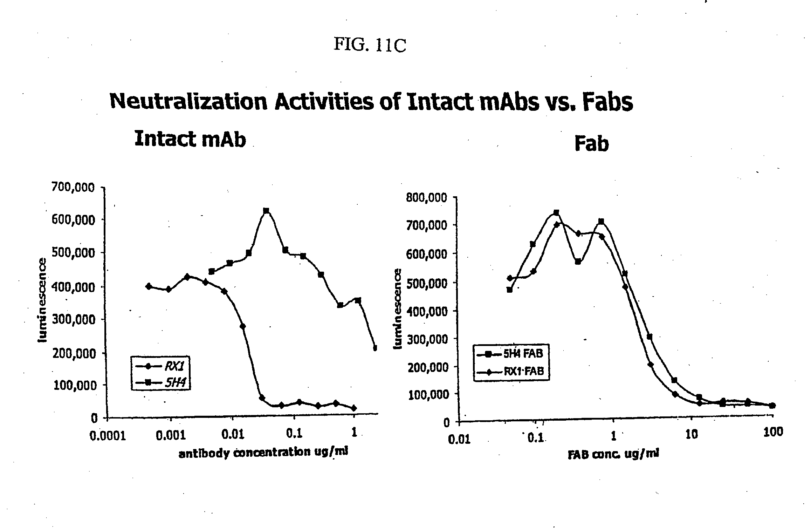

Neutralization activities of intact RX1 and 5H4 versus Fab fragments of RX1 and 5H4 were determined by measuring M-CSF-dependent cell proliferation in the presence of various concentrations of antibody. The cell proliferation was determined by chemiluminescent dye. As shown in Figure 11C, intact RX1 has the highest potency, while the Fab fragment of RX1 loses its potency and behaves like 5H4 and the 5H4 Fab fragment.

Binding properties of the aforementioned antibodies were analyzed using Biacore analyses. In order to determine the relative affinities of RX1, 5H4, and MC3 to M-CSF, rabbit anti-mouse Fc was immobilized onto a CM5 biosensor chip via amine coupling. The aforementioned antibodies were then captured on the anti-mouse Fc/CM5 biosensor chip at 1.5µg/ml for 3 min at 2µl/min. M-CSF was flowed over the modified biosensor surface at varying concentrations (Rmax ∼15). Test antibodies and antigen were diluted in 0.01 M HEPES pH 7.4, 0.15 M NaCL, 3 mM EDTA, 0.005% Surfactant P20 (HBS-EP). All experiments were performed at 25°C. Kinetic and affinity constants were determined using Biaevaluation software (Biacore) with a 1:1 interaction model/global fit. As shown below in Table 8, RX1 binds to M-CSF with the highest affinity relative to 5H4 and MC3.

**Table 8**

| | **Ka (M-1 Sec-1)** | **Kd (sec-1)** | **KD (nM)** |
|---|---|---|---|
| RX1 | 1.64e6 | 2.7e-4 | 0.16 |
| 5H4 | 5.94e5 | 1.77e-3 | 3.0 |
| MC3 | 7.04e5 | 1.93e-4 | 0.27 |

### EXAMPLE 7

The following example reveals the linear epitope (i.e., amino acid sequence) on M-CSF recognized by murine antibodies RX1, 5H4, and MC3.

Initially, the epitope mapping strategy was designed to determine whether antibodies RX1, 5H4, and MC3 recognized linear epitopes or conformational epitopes within M-CSF. Accordingly, the anti-M-CSF antibodies were tested against 0.1µg M-CSF under reducing as well as non-reducing conditions. Only the the non-reduced form of M-CSF was recognized by each of the antibodies, suggesting the epitopes recognized are discontinuous in nature.

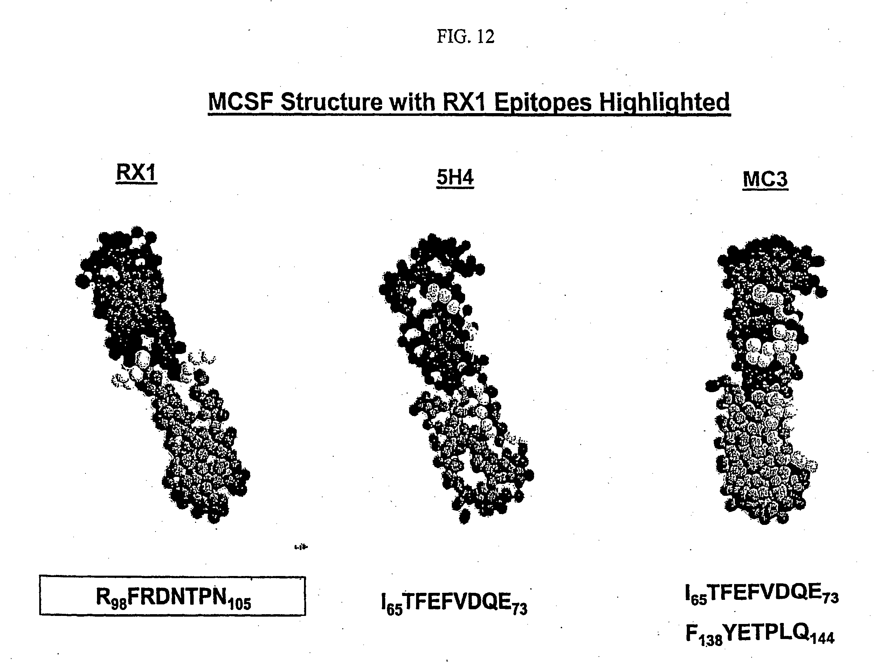

Next, the linear epitope of M-CSF was determined for each antibody. Specifically, SPOTs membranes (Sigma Genosys) were prepared where the M-CSF fragment sequence of interest, overlapping 10mer peptides synthesized with one amino acid offset, were loaded onto the cellulose membrane support. These membranes were then probed with the aforementioned antibodies and reactive SPOTs were identified. The peptide sequence was then identified by its corresponding location on the membrane, and overlapping amino acids within the positive reacting peptides were identified as the epitope. As shown in Figure 12, RX1 binds to a different linear epitope than 5H4 and MC3, which map to a different location on M-CSF. RX1 binds to a linear epitope represented by RFRDNTPN (SEQ ID NO: 120) or RFRDNTAN (SEQ ID NO: 121), amino acids 98-105 of M-CSF of Figure 7. 5H4 binds to a linear epitope represented by ITFEFVDQE (SEQ ID NO: 122), amino acids 65-73 of M-CSF of Figure 7. MC3 binds to two linear epitopes represented by (1) ITFEFVDQE (SEQ ID NO: 122), amino acids 65-73 of M-CSF of Figure 7 and (2) FYETPLQ (SEQ ID NO: 123), amino acids 138-144 of M-CSF of Figure 7.

### EXAMPLE 8

The following example sets out a procedure for an *in vivo* study on the therapeutic efficacy of anti-M-CSF neutralizing antibody of the invention in reducing atherosclerotic lesions.

Anti-M-CSF neutralizing antibody, for example an RX1-derived or RX1-competing antibody, is tested in rhesus monkeys (*Macaca mulatta*) with atherosclerotic lesions induced by feeding them a high-saturated fatty acid and high-cholesterol diet. Macaques with induced lesions are treated with anti-M-CSF antibody according to an escalating dosing regimen (0.2 mg/kg to 20 mg/kg) weekly for up to six months. A control group of macaques with induced lesions are injected with expedient solutions. The extent of lesions in three major coronary arteries and the right carotid artery is evaluated morphometrically by light microscopy in all groups of animals. Lesion regression is evaluated in the RX1 treated group versus the control group. It is expected that treatment with RX1 will significantly induce the regression of atherosclerosis lesions.

The experiments may also include dosing with a second therapeutic agent for treating atherosclerotic disease, such as a drug which beneficially alters the serum lipid profile (e.g., statins such as lovastatin, simvastatin and pravastatin, fluvastatin, atorvastatin, cerivastatin and rosuvastatin, drugs that lower intestinal absorption of cholesterol such as ezetimibe, fibrates, cholestyramine or colestipol resins, or nicotinic acid, or drugs containing highly polyunsaturated or omega-3 fatty acids, e.g. eicosapentaenoic acid and docosahexaenoic acid from fish oil), anti-anginal agents such as nitrates, beta-blockers, angiotensin converting enzyme inhibitors, angiotensin receptor blockers, calcium channel antagonists, anti-platelet agents, and anticoagulants.

### EXAMPLE 9

The following example sets forth a procedure for an in vivo study on the therapeutic efficacy of anti-M-CSF neutralizing antibody of the invention in treating AIDS.

Anti-M-CSF neutralizing antibody is tested in an AIDS model in rhesus macaques infected with a chimera (RT-SHIV) of simian immunodeficiency virus containing reverse transcriptase from human immunodeficiency virus type-1 (HIV-1). RT-SHIV-infected macaques are treated with RX1 with escalating dosing regimen (0.2 mg/kg to 20 mg/kg) weekly up to six months. A control group of RT-SHIV-infected macaques is injected with expedient solutions. Plasma viral RNA levels in all animals are tracked for reduction after 4 weeks and followed up to 10 weeks. Virus loads are followed throughout the treatment. Both plasma viral RNA levels and viral loads are followed after the stop of treatment. Post-treatment RT-SHIV isolates are examined for mutations associated with resistance to the treatment. It is expected that the treatment with anti-M-CSF antibody will block HIV infection through reduction of its plasma viral RNA level and virus loads.

The experiments may include dosing with a second therapeutic agent for HIV, including, for example, a second anti-M-CSF antibody, or agents used in highly active antiretroviral therapy (HAART) as described in Barbaro G, et al., Curr Pharm Des.;11(4):1805-43 (2005).

### EXAMPLE 10

The following example sets forth the procedure for measuring the ability of anti-M-CSF antibody of the invention to inhibit the spread of HIV-1.

### (1) Monocyte isolation and culture

PBMC are isolated from blood following leukapheresis of HIV-1-seronegative donors and subsequent density-gradient centrifugation; monocytes are purified by countercurrent centrifugal cell elutriation (Gruber, M. F., et al., J. Immunol. 154:5528 (1995); Gerrard, T. L., et al., Cell. Immunol. 82:394 (1983)). Elutriated monocyte viability is determined by trypan blue exclusion, and presence of CD 14 is determined by flow cytometry (FACS) analysis of representative samples. Monocytes are differentiated in culture for 8 days at 37°C in 5% CO2 at a concentration of 4 x 10⁶/2 ml in six-well tissue culture plates (Costar, Cambridge, MA) using DMEM (Life Technologies, Gaithersburg, MD) complete medium containing 10% pooled human serum, 2 mM L-glutamine (Life Technologies), 1 mM sodium pyruvate (Life Technologies), and penicillin (50 U/ml)/streptomycin (50 µg/ml) (Life Technologies) to generate MDM. All reagents used in the isolation and culture of MDM are tested for endotoxin.

### (2) Virus infection of monocyte-derived macrophages

MDM are harvested by scraping and plated into 24-well tissue culture plates (Nunc, Naperville, IL), at a concentration of 500,000 cells/ml, 1.5 ml/well. After 24-48 h, MDM are infected with HIV-1, (Gruber, M. F., et al., J. Immunol. 154:5528 (1995)). Every 3 days thereafter, 80% of the culture medium is collected, stored at -80°, then replaced. In some experiments, AZT (Sigma, St. Louis, MO) is added at a concentration of 1 µM following virus adsorption and replenished every 3 days. In other experiments, anti-M-CSF antibody, is added after virus adsorption and replenished every 3 days. The concentration of anti-M-CSF added should be sufficient to neutralize 100 ng/ml of M-CSF bioactivity. MDM cultures infected with HIV-1 are generally maintained in DMEM complete medium, as described above. Optionally the experiments may include addition of a second therapeutic anti-HIV agent, such as a reverse transcriptase inhibitor or protease inhibitor.

A reverse-transcriptase (RT) assay is used to measure the progression of infection in MDM infected with HIV-1. The RT assay used is a ³H-based modification of the methods described by Hoffman (Hoffman, A. D., Virology 147:326 (1985)). Briefly, 60 µl of harvested culture supernatants are diluted with 60 µlpI of Tris buffer (pH 7.8)/0.05% Triton X-100. Replicate 50- µl samples are then added to 100 µl of a solution containing poly (rA) (Pharmacia LKB, Piscataway, NJ), oligo (dT) (Pharmacia LKB), MgCl, and ³H-labeled dTTP (NEN, Boston, MA) in a 96-well U-bottom microtiter plate (Falcon 3910) and incubated for 2 h at 37°C. After incubation, 100 µl of a solution containing 10% TCA is added to each well. The individual wells are transferred to glass-fiber filters (Wallac) by using a cell harvester (Skatron) connected to two fluid reservoirs containing 5% TCA/5% sodium pyrophosphate and 70% ethanol, which are run in sequence. Finally, the filters are counted on a beta scintillation counter (beta platereader, Pharmacia LKB). In this way, inclusion of M-CSF antagonists such as antibody RX1, which bind to M-CSF and prevent it from interacting with its receptor, is analyzed for its ability to inhibit HIV-1 replication.

From the foregoing it will be appreciated that, although specific embodiments of the invention have been described herein for purposes of illustration, various modifications may be made without deviating from the scope of the invention.

## Claims

1. A humanized anti-M-CSF antibody, for use in treating a human subject having an atherosclerotic disease, or for use in treating HIV infection in a human subject, or for use in treating a condition associated with HIV infection in a human subject having an HIV infection, wherein:
(i) the heavy chain comprises the heavy chain variable region sequence set forth in SEQ ID NO: 43 and an IgG1 constant region; and
(ii) the light chain comprises the light chain variable region sequence set forth in SEQ ID NO: 53.

2. The antibody for use according to claim 1 wherein the antibody comprises a mutation in the IgG1 constant region that reduces antibody-dependent cellular cytotoxicity or complement dependent cytotoxicity activity.

3. The antibody for use according to claim 1 or claim 2wherein the antibody comprises the heavy chain sequence set forth in SEQ ID NO: 116.

4. The antibody for use according to any preceding claim, wherein the light chain comprises a human kappa constant region.

5. A humanized anti-M-CSF antibody, for use in treating a human subject having an atherosclerotic disease, or for use in treating HIV infection in a human subject, or for use in treating a condition associated with HIV infection in a human subject having an HIV infection, wherein:
(i) the heavy chain comprises the heavy chain variable region sequence set forth in SEQ ID NO: 43 and an IgG1 constant region; and
(ii) the light chain comprises the light chain variable region sequence set forth in SEQ ID NO: 53,
wherein the antibody is for use in coordination with treatment using a second therapeutic agent.

6. Use of a humanized anti-M-CSF antibody, in the manufacture of a medicament for treating a human subject having an atherosclerotic disease, or treating HIV infection in a human subject, or treating a condition associated with HIV infection in a human subject having an HIV infection, wherein:
(i) the heavy chain comprises the heavy chain variable region sequence set forth in SEQ ID NO: 43 and an IgG1 constant region; and
(ii) the light chain comprises the light chain variable region sequence set forth in SEQ ID NO: 53,
wherein the medicament further comprises a second therapeutic agent.

## Patentansprüche

1. Humanisierter anti-M-CSF-Antikörper zur Verwendung bei der Behandlung eines menschlichen Individuums, das an einer arteriosklerotischen Krankheit leidet, oder zur Verwendung in der Behandlung von HIV-Infektion bei einem menschlichen Individuum, oder zur Verwendung in der Behandlung eines mit HIV-Infektion assoziierten Leidens bei einem menschlichen Individuum mit einer HIV-Infektion, wobei:
(i) die schwere Kette die Sequenz der variablen Region der schweren Kette gemäß SEQ ID NO: 43 und eine konstante Region eines IgG1 umfasst; und
(ii) die leichte Kette die Sequenz der variablen Region der leichten Kette gemäß SEQ ID NO: 53 umfasst.

2. Antikörper zur Verwendung nach Anspruch 1, wobei der Antikörper eine Mutation in der konstanten Region von IgG1, die die antikörperabhängige Zytotoxizitätsaktivität der Zelle oder die komplementabhängige Zytotoxizitätsaktivität reduziert, umfasst.

3. Antikörper zur Verwendung nach Anspruch 1 oder Anspruch 2, wobei der Antikörper die Sequenz der schweren Kette gemäß SEQ ID NO: 116 umfasst.

4. Antikörper zur Verwendung nach einem vorhergehenden Anspruch, wobei die leichte Kette eine humane konstante Kappa-Region umfasst.

5. Humanisierter anti-M-CSF-Antikörper zur Verwendung bei der Behandlung eines menschlichen Individuums, das an einer arteriosklerotischen Krankheit leidet, oder zur Verwendung in der Behandlung von HIV-Infektion bei einem menschlichen Individuum, oder zur Verwendung in der Behandlung eines mit HIV-Infektion assoziierten Leidens bei einem menschlichen Individuum mit einer HIV-Infektion, wobei:
(i) die schwere Kette die Sequenz der variablen Region der schweren Kette gemäß SEQ ID NO: 43 und eine konstante Region eines IgG1 umfasst; und
(ii) die leichte Kette die Sequenz der variablen Region der leichten Kette gemäß SEQ ID NO: 53 umfasst,
wobei der Antikörper für die Verwendung in Koordination mit einer Behandlung mit einem zweiten Therapeutikum bestimmt ist.

6. Verwendung eines humanisierten anti-M-CSF-Antikörpers in der Herstellung eines Arzneimittels zur Behandlung eines menschlichen Individuums, das an einer arteriosklerotischen Krankheit leidet, oder zur Behandlung von HIV-Infektion bei einem menschlichen Individuum, oder zur Behandlung eines mit HIV-Infektion assoziierten Leidens bei einem menschlichen Individuum mit einer HIV-Infektion, wobei:
(i) die schwere Kette die Sequenz der variablen Region der schweren Kette gemäß SEQ ID NO: 43 und eine konstante Region eines IgG1 umfasst; und
(ii) die leichte Kette die Sequenz der variablen Region der leichten Kette gemäß SEQ ID NO: 53 umfasst,
wobei das Arzneimittel weiterhin ein zweites Therapeutikum umfasst.

## Revendications

1. Anticorps anti-M-CSF humanisé pour utilisation dans le traitement d'un sujet humain atteint d'une maladie athérosclérotique, ou pour utilisation dans le traitement d'une infection par le VIH chez un sujet humain, ou pour utilisation dans le traitement d'un état associé à l'infection par le VIH chez un sujet humain atteint d'une infection par le VIH, dans lequel
(i) la chaîne lourde comprend la séquence de la région variable de la chaîne lourde présentée dans SEQ ID NO:43 et une région constante de l'IgG1 ; et
(ii) la chaîne légère comprend la séquence de la région variable de la chaîne légère présentée dans SEQ ID NO:53.

2. Anticorps pour utilisation selon la revendication 1, l'anticorps comprenant une mutation dans la région constante de l'IgG1, qui réduit l'activité de cytotoxicité cellulaire anticorps-dépendante ou de cytotoxicité complément-dépendante.

3. Anticorps pour utilisation selon la revendication 1 ou 2, dans lequel l'anticorps comprend la séquence de la chaîne lourde présentée dans SEQ ID NO:116.

4. Anticorps pour utilisation selon l'une quelconque des revendications précédentes, dans lequel la chaîne légère comprend la région constante kappa humaine.

5. Anticorps anti-M-CSF humanisé pour utilisation dans le traitement d'un sujet humain atteint d'une maladie athérosclérotique, ou pour utilisation dans le traitement d'une infection par le VIH chez un sujet humain, ou pour utilisation dans le traitement d'un état associé à l'infection par le VIH chez un sujet humain atteint d'une infection par le VIH, dans lequel
(i) la chaîne lourde comprend la séquence de la région variable de la chaîne lourde présentée dans SEQ ID NO:43 et une région constante de l'IgG1 ; et
(ii) la chaîne légère comprend la séquence de la région variable de la chaîne légère présentée dans SEQ ID NO:53,
l'anticorps étant destiné à être utilisé en coordination avec un traitement utilisant un deuxième agent thérapeutique.

6. Utilisation d'un anticorps anti-M-CSF humanisé pour la fabrication d'un médicament destiné au traitement d'un sujet humain atteint d'une maladie athérosclérotique, ou au traitement d'une infection par le VIH chez un sujet humain, ou au traitement d'un état associé à l'infection par le VIH chez un sujet humain atteint d'une infection par le VIH, pour laquelle
(i) la chaîne lourde comprend la séquence de la région variable de la chaîne lourde présentée dans SEQ ID NO:43 et une région constante de l'IgG1 ; et
(ii) la chaîne légère comprend la séquence de la région variable de la chaîne légère présentée dans SEQ ID NO:53,
le médicament comprenant en outre un deuxième agent thérapeutique.
